# EUROPEAN PATENT APPLICATION

(11) **EP 4 818 161 A1**
(43) Date of publication of application: **07.10.2026**
(21) Application number: 24898247.2
(22) Date of filing: 29.11.2024
(51) Int. Cl.: C07D 251/24, C07D 405/10, C07D 409/10, C07D 403/10, C07D 239/26, H10K 85/60

(54) **ORGANIC COMPOUND AND ORGANIC ELECTROLUMINESCENT DEVICE USING SAME**

(30) Priority: 01.12.2023 KR 20230172741
(71) Applicant: Solus Advanced Materials Co., Ltd., Iksan-si, Jeollabuk-do 54584 (KR)
(72) Inventor: PARK, Woojae, Seongnam-si, Gyeonggi-do 13636 (KR); SIM, Jaeyi, Seongnam-si, Gyeonggi-do 13636 (KR); LEE, Changyu, Seongnam-si, Gyeonggi-do 13636 (KR)
(74) Representative: Office Freylinger
(86) International application number: PCT/KR2024/019403
(87) International publication number: WO 2025/116655

(57) **Abstract**

The present invention relates to a novel organic compound and an organic electroluminescent device using same and, more specifically, to an organic compound that exhibits excellent electron transport ability, heat resistance, carrier transport capability, and luminescence, and an organic electroluminescent device in which the organic compound is included in one or more organic layers, thereby improving properties such as emission efficiency, driving voltage, and lifespan.

## Description

### Technical Field

The present invention relates to a novel organic compound and an organic electroluminescent device using the same and, more specifically, to a novel compound with excellent electron transporting ability, and an organic electroluminescent device exhibiting improved characteristics, such as luminous efficiency, driving voltages, and lifespan, by containing the novel compound in one or more organic layers.

### Background Art

In an organic electroluminescent device, upon the application of voltage between two electrodes, holes from an anode and electrons from a cathode are injected into organic layers. The injected holes and electrons combine with each other to form excitons, and the excitons fall down to the ground state to emit light. Particularly, materials used for the organic layers may be classified into light emission materials, hole injection materials, hole transport materials, electron transport materials, electron injection materials, and the like according to the function thereof.

Materials for forming an emissive layer of the organic electroluminescent device may be classified into blue, green and red light emission materials according to the color of light emission. Additionally, yellow and orange light emission materials may be used as light emission materials for displaying better natural colors. Additionally, host/dopant-based light emission materials may be used as light emission materials to increase color purity and improve luminous efficiency through energy transfer.

Dopant materials may be classified into fluorescent dopants using organic materials and phosphorescent dopants using metal complex compounds containing heavy atoms, such as Ir and Pt. These phosphorescent materials can theoretically improve the luminous efficiency up to four times compared to fluorescent materials, so research has been conducted on phosphorescent host materials as well as phosphorescent dopants.

Until today, NPB, BCP, Alq3, and the like have been widely known as materials for use in a hole injection layer, a hole transport layer, and an electron transport layer, and anthracene derivatives have been reported as the light emitting layer materials. In particular, among the light emitting layer materials, metal complex compounds containing Ir, such as Firpic, Ir(ppy)3, (acac)Ir(btp)2, and the like, which have advantages in terms of improving efficiency, are used as blue, green, and red phosphorescent dopant material, and 4,4-dicarbazollybiphenyl (CBP) is used as the phosphorescent host material.

However, conventional light emission materials have advantages in terms of light emission characteristics, but are not satisfactory in terms of lifespan of organic electroluminescent devices due to low glass transition temperatures and very poor thermal stability. Accordingly, there is a need to develop light emission materials with excellent performance.

### Disclosure of Invention

### Technical Problem

An aspect of the present invention is to provide a novel compound having excellent electron injection and transport properties, thermal stability, carrier transport ability, and luminescent properties, which can be used as a material for an organic layer of an organic electroluminescent device, specifically as a material for a light emitting layer (an emission layer), a lifespan improvement layer, a light emitting auxiliary layer, an electron transport layer, or an electron transport auxiliary layer, and more specifically as a material for an electron transport layer or an electron transport auxiliary layer.

Another aspect of the present invention is to provide an organic electroluminescent device exhibiting a low driving voltage, high luminous efficiency, and improved lifespan by containing the above-described novel compound.

### Solution to Problem

To achieve the above objectives, the present invention provides an organic compound represented by the following Chemical Formula 1: 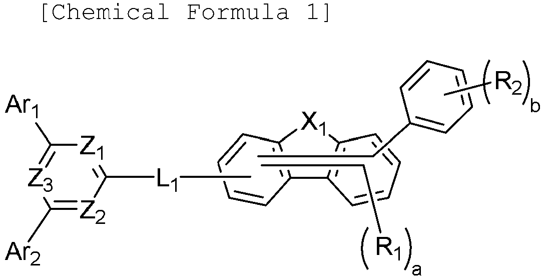 (wherein in Chemical Formula 1,
Z₁ to Z₃ are the same as or different from each other and are each independently N or C(Ar₃), provided that at least two of Z₁ to Z₃ are N;
Ar₁ to Ar₂ are the same as or different from each other and are each independently selected from the group consisting of a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group and a C₆-C₆₀ arylamine group,
L₁ is a linker represented by the following Chemical Formula L1-1 or L1-2, 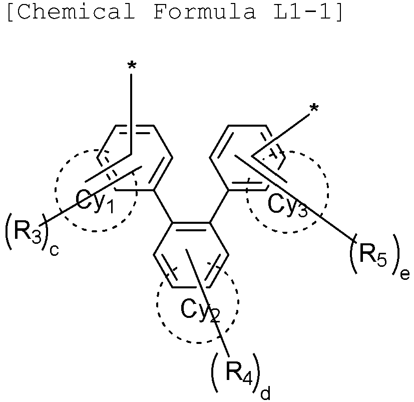 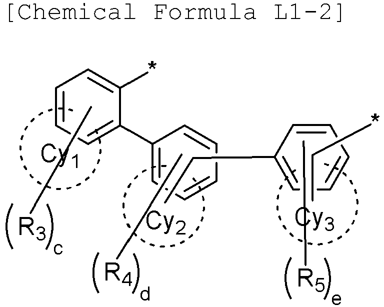
wherein in Chemical Formula L1-1 or L1-2,
a dotted line indicates that each ring is present or absent,
Cy₁ to Cy₃ are the same as or different from each other and are each independently a 6-membered aromatic ring,
a, c, d and e are each independently an integer of 0 to 6,
b is an integer of 0 to 5,
R₁ to R₅ are the same as or different from each other and are each independently selected from the group consisting of a hydrogen, a deuterium (D), a halogen, a cyano group, a nitro group, an amino group, a hydroxy group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
X₁ is selected from the group consisting of O, S, C(Ar₄)(Ar₅), N(Ar₆) and 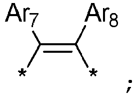
Ar₃ to Ar₈ are the same as or different from each other and are each independently selected from the group consisting of a hydrogen, a deuterium (D), a halogen, a cyano group, a nitro group, an amino group, a hydroxy group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, arylamine group and the fused ring of Ar₁ to Ar₈, and R₁ to R₅ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other).

In addition, the present invention also provides an organic electroluminescent device including: an anode, a cathode, and one or more organic layers disposed between the anode and the cathode, wherein at least one of the one or more organic layers includes the compound represented by Chemical Formula 1.

In one embodiment, the organic layer including the compound represented by Chemical Formula 1 may be an electron transport layer or an electron transport auxiliary layer.

### EFFECTS OF THE INVENTION

According to an embodiment of the present invention, the compound of the present invention has excellent characteristics such as electron transport ability, electrochemical stability, thermal stability, and the like, and thus may be used as an organic layer material of an organic electroluminescent device. In particular, when the compound of the present invention is used as an electron transport layer material, or an electron transport auxiliary layer material, it is possible to manufacture an organic electroluminescent device having excellent light emitting performance, low driving voltage, high current efficiency and long lifespan characteristics as compared with conventional materials. Accordingly, the organic electroluminescent device including the compound of the present invention may be effectively applied to manufacture full color display panels having improved performance and extended lifespan.

Effects according to the present invention are not limited by the description exemplified above, and more diverse effects are included in the present specification.

### BRIEF DESCRIPTION OF DRAWINGS

FIG. 1 is a cross-sectional view schematically illustrating an organic electroluminescent device according to a first embodiment of the present invention.
FIG. 2 is a cross-sectional view schematically illustrating an organic electroluminescent device according to a second embodiment of the present invention.
FIG. 3 is a cross-sectional view schematically illustrating an organic electroluminescent device according to a third embodiment of the present invention.

### <DESCRIPTION OF REFERENCE NUMERALS>

100: anode, 200: cathode,
300: organic layer, 310: hole injection layer,
320: hole transport layer, 330: light emitting layer,
340: electron transport layer, 350: electron injection layer,
360: electron transport auxiliary layer

### DETAILED DESCRIPTION OF THE INVENTION

Hereinafter, the present invention will be described in detail.

### <Novel organic compound>

The present invention provides a compound represented by the Chemical Formula 1, which has excellent electron injection and transport capabilities, electrochemical stability, thermal stability, carrier transport capability, and light-emitting capability, and thus can be used as a material for a highly efficient organic material layer, in particular, an electron transport layer material or an electron transport auxiliary layer material.

Specifically, the organic compound according to the present invention has a structure in which a nitrogen-containing heteroaromatic ring (Z₁ to Z₃-containing ring) moiety and a dibenzo-based moiety (X₁-containing ring moiety) are connected to each other through a terphenylene group serving as a linker group L₁.

However, in the present invention, a terphenylene group itself, which is a linker group L₁, may contain an ortho-bond, or the nitrogen-containing heteroaromatic ring moiety may be connected to a first benzene ring of the terphenylene group at an ortho-bonding position, or the dibenzo-based moiety may be connected to a third benzene ring of the terphenylene group at an ortho-bonding position. Specifically, the terphenylene group serving as the linker group L₁ may be an ortho-terphenylene group (for example, a linker group of Chemical Formula L-1). Alternatively, the nitrogen-containing heteroaromatic ring moiety may be bonded to a carbon 2-position of the first benzene ring of the terphenylene group, which is an ortho-position with respect to a central (second) benzene ring of the terphenylene group, or the dibenzo-based moiety may be bonded to a 2-position of the third benzene ring of the terphenylene group, which is an ortho-position with respect to the central (second) benzene ring of the terphenylene group. That is, a site connected to another moiety may be a terphenylene group (for example, a linker group of Chemical Formula L-2) at an ortho-bonding position with respect to the central (second) benzene ring. As described above, in the compound of the present invention, the nitrogen-containing heteroaromatic ring moiety and the dibenzo-based moiety may be connected by at least one ortho-bond around the terphenylene group serving as the linker group L₁. Accordingly, the compound of the present invention can induce delocalization of the lowest unoccupied molecular orbital (LUMO), thereby improving electron transport properties. Furthermore, in the compound of the present invention, steric hindrance is maximized to induce an increase in triplet energy, thereby preventing excitons generated in a light-emitting layer from diffusing (moving) to an adjacent electron transport layer or hole transport layer. Consequently, the number of excitons contributing to light emission is increased to improve light-emitting efficiency of the device, and durability and electrochemical stability of the device are improved to efficiently increase lifespan of the device. In addition, an organic electroluminescent device to which the compound of the present invention is applied can implement low-voltage driving.

Additionally, the compound of the present invention introduces an electron donating group (EDG) having a weak electron donating property, such as a phenyl group or the like, to a terminal of the dibenzo-based moiety, such that an electron donating property of the dibenzo-based moiety can be improved through an electron density increasing effect, thereby improving stability of a molecule.

Furthermore, in the compound of the present invention, various substituents (for example, an alkyl group, an aryl group, a heteroaryl group, and the like) are introduced into the nitrogen-containing heteroaromatic ring moiety to significantly increase a molecular weight, which leads to a high glass transition temperature, thereby improving thermal stability and electrochemical stability.

As described above, the compound represented by Chemical Formula 1 of the present invention has excellent electron transport ability, thermal stability, electrochemical stability, and the like, and thus may be applied as an organic layer material of an organic electroluminescent device, preferably as a light emitting layer material (a blue, green and/or red phosphorescent host material), an electron transport/injection layer material, a hole transport/injection layer material, a light emitting auxiliary layer material, a lifetime improvement layer material, or an electron transport auxiliary layer material, and more preferably as an electron transport layer material or an electron transport auxiliary layer material. In this case, the performance and lifespan characteristics of the organic electroluminescent device may be significantly improved, and the performance of a full-color organic electroluminescent panel including such an organic electroluminescent device may also be maximized.

In Chemical Formula 1 according to the present invention, Z₁ to Z₃ may be the same as or different from each other, and may each independently be N or C(Ar₃), provided that at least two of Z₁ to Z₃ are N. The ring moiety containing Z₁ to Z₃ is a kind of nitrogen-containing heteroaromatic ring and may be a monocyclic nitrogen-containing heteroaryl group containing at least two nitrogen atoms (for example, an azine group). As such, since the heteroaromatic ring moiety containing two to three nitrogen atoms (N) is included, more excellent electron absorption characteristics may be exhibited, which is advantageous for electron injection and transport.

In an embodiment, the ring moiety containing Z₁ to Z3 ( 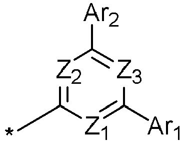 ) may be any one selected from the group consisting of moieties Az1-1 to Az1-3 below. However, it is not limited thereto. 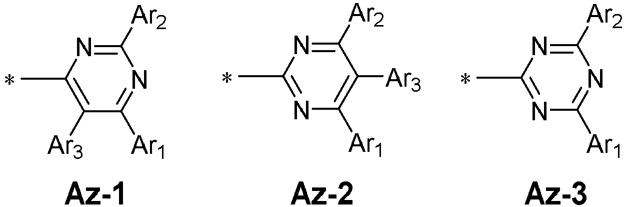 wherein in the moieties Az1-1 to Az1-3,
* indicates a site where a bond with Chemical Formula 1 is made, and
Ar₁, Ar₂ and Ar₃ are each as defined in Chemical Formula 1.

In another embodiment, each of Z1 to Z3 may be N. That is, the ring moiety containing Z₁ to Z₃ may be represented by moiety Az1-3.

In Chemical Formula 1, Ar₁ to Ar₂ may be the same as or different from each other and may each independently be selected from the group consisting of a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group and a C₆-C₆₀ arylamine group. Specifically, Ar₁ and Ar₂ may be each independently selected from the group consisting of a C₁-C₂₀ alkyl group, a C₆ to C₃₀ aryl group, and a heteroaryl group having 5 to 30 nuclear atoms.
the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂ may each independently be unsubstituted or substituted with at least one substituent selected from the group consisting of deuterium (D), a halogen (e. g., -F, -Cl, -Br, -I), a cyano group (-CN), a nitro group (-NO₂), an amino group (-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group. Specifically, the alkyl group, aryl group, heteroaryl group, arylphosphine oxide group, and arylamine group may each independently be unsubstituted or substituted with at least one substituent selected from the group consisting of deuterium (D), a halogen (e.g., -F, -Cl, -Br, and -I), a cyano group (-CN), a nitro group (-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group. When two or more substituents are present, they may be the same as or different from each other.

In an embodiment, Ar₁ to Ar₂ may be the same as or different from each other and may each independently be selected from the group consisting of deuterium (D), a methyl group, an ethyl group, a propyl group, a butyl group, a pentyl group, a hexyl group, a heptyl group, an octyl group, a phenyl group, a biphenyl group, a terphenyl group, a naphthyl group, a triphenylenyl group, a phenanthryl group, a fluorenyl group, an anthracenyl group, an anthryl group, a pyrenyl group, a pyridyl group, a pyrazinyl group, a pyrimidinyl group, a pyridazinyl group, a triazinyl group, a phenoxathiinyl group, an indolizinyl group, an indolyl group, a purinyl group, a quinolyl group, a benzothiazolyl group, a dibenzofuranyl group, a dibenzothiophenyl group, a phenanthrolinyl group, and a carbazolyl group.

In another embodiment, Ar₁ to Ar₂ may be the same as or different from each other and may each independently be selected from the group consisting of the following substituents S1-1 to S1-10, but are not limited thereto. 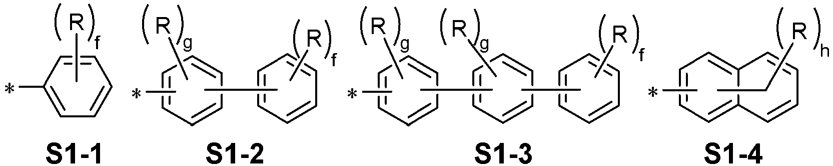 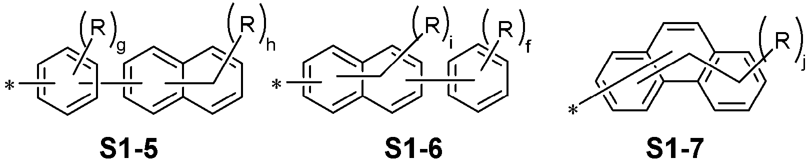 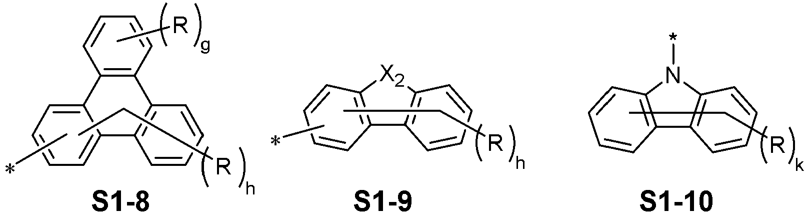 wherein in the moieties S1-1 to S1-10,
X₂ is selected from the group consisting of O, S, C(Ar₉)(Ar₁₀), Si(Ar₁₁)(Ar₁₂) and N(Ar₁₃),
Ar₉ to Ar₁₃ may be the same as or different from each other and may each independently be selected from the group consisting of deuterium (D), a halogen (e. g., -F, -Cl, - Br, -I), a cyano group(-CN), a nitro group(-NO₂), an amino group (-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may be bonded to any adjacent group (e.g., R₈-R₉, R₁₀-R₁₁, R₈-R, R₁₀-R, R₁₂-R) to form a fused ring. Specifically, Ar₉ to Ar₁₃ may each independently be selected from the group consisting of deuterium (D), a halogen (e. g., -F, -Cl, - Br, -I and the like), a cyano group(-CN), a C₁ to C₂₀ alkyl group, a C₆ to C₃₀ aryl group, a heteroaryl group having 5 to 60 ring atoms, a C₆ to C₃₀ arylphosphine oxide group, and a C₆ to C₃₀ arylamine group, or may be bonded to any adjacent group (e.g., R₈-R₉, R₁₀-R₁₁, R₈-R, R₁₀-R, R₁₂-R) to form a fused ring. More specifically, Ar₉ to Ar₁₃ may each independently be selected from the group consisting of deuterium (D), a cyano group(-CN), a methyl group, an ethyl group, a propyl group, a butyl group, a pentyl group, a hexyl group, a heptyl group, an octyl group, a phenyl group, a biphenyl group, a terphenyl group, and a naphthyl group, or may be fused with an adjacent group to form a fused ring.
f may be an integer of 0 to 5,
g may be an integer of 0 to 4,
h may be an integer of 0 to 7,
i may be an integer of 0 to 6,
j may be an integer of 0 to 9,
k may be an integer of 0 to 8,
when a plurality of Rs are present, the Rs may be the same as or different from each other,
R may be selected from the group consisting of deuterium (D), a halogen (e. g., -F, -Cl, -Br, -I), a cyano group (-CN), a nitro group(-NO₂), an amino group (-NH₂), a hydroxy group (-OH), a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group. Specifically, R may be selected from the group consisting of deuterium (D), a halogen (e. g., -F, -Cl, -Br, -I), a cyano group(-CN), a nitro group(-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group. Here, the fused ring may be at least one selected from the group consisting of a C₃ to C₆₀ fused aliphatic ring (specifically, a C₃ to C₃₀ fused aliphatic ring), a C₆ to C₆₀ fused aromatic ring (specifically, a C₆ to C₃₀ fused aromatic ring), a 5- to 60-membered fused heteroaromatic ring (specifically, a 5- to 30-membered fused heteroaromatic ring), a C₃ to C₆₀ spiro ring, and combinations thereof.

In another embodiment, Ar₁ to Ar₂ may be the same as or different from each other and may each independently be selected from the group consisting of the following substituents S1 to S26, but are not limited thereto.

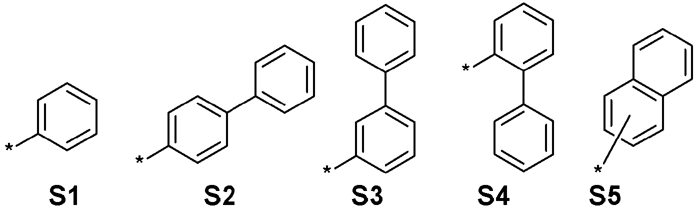

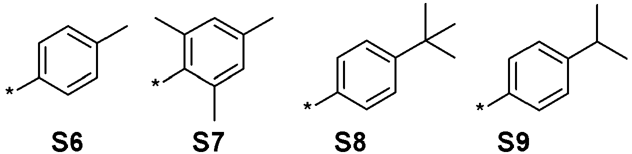

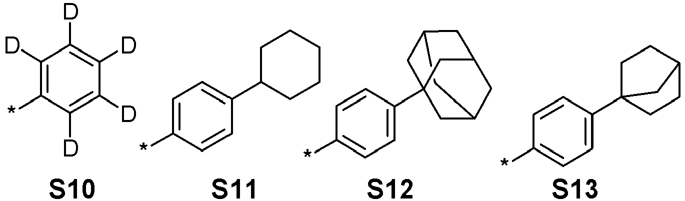

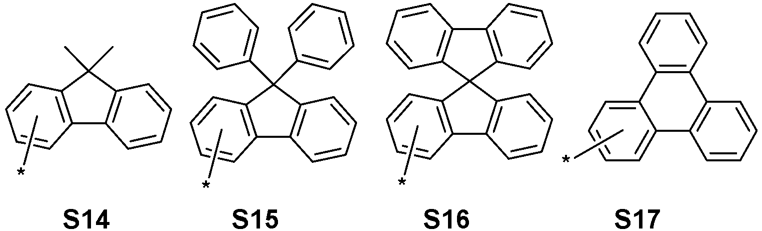

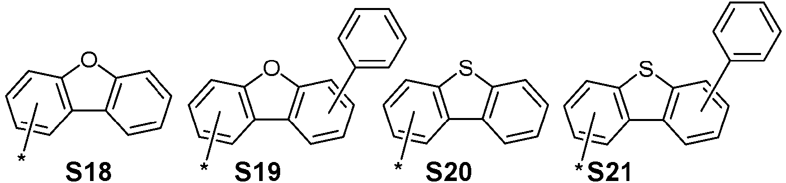

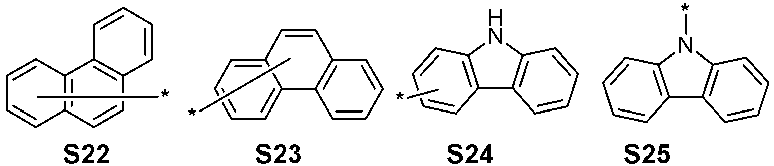

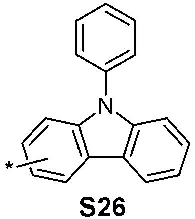

In Chemical Formula 1, Ar₃ may each independently be selected from the group consisting hydrogen, deuterium (D), a halogen (e. g., -F, -Cl, -Br, -I), a cyano group(-CN), a nitro group(-NO₂), an amino group(-NH₂), a hydroxy group(-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may be bonded to any adjacent group (e.g., Ar₃-Ar₁, Ar₃-Ar₂ and the like) to form a fused ring. Specifically, Ar₃ may each independently be selected from the group consisting of deuterium (D), a halogen (e. g., -F, -Cl, -Br, -I), a cyano group (-CN), a nitro group (-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group, or may be bonded to any adjacent group to form a fused ring. Here, the fused ring may be at least one selected from the group consisting of a C₃ to C₆₀ fused aliphatic ring (specifically, a C₃ to C₃₀ fused aliphatic ring), a C₆ to C₆₀ fused aromatic ring (specifically, a C₆ to C₃₀ fused aromatic ring), a 5- to 60-membered fused heteroaromatic ring (specifically, a 5- to 30-membered fused heteroaromatic ring), a C₃ to C₆₀ spiro ring, and combinations thereof.

In one embodiment, Ar₃ may be selected from the group consisting of hydrogen, deuterium (D), a cyano group (-CN), a methyl group, an ethyl group, a propyl group, a butyl group, a pentyl group, a hexyl group, a heptyl group, an octyl group, a phenyl group, a biphenyl group, a terphenyl group, a naphthyl group, a triphenylenyl group, a phenanthryl group, a fluorenyl group, an anthracenyl group, an anthryl group, a pyrenyl group, a pyridyl group, a pyrazinyl group, a pyrimidinyl group, a pyridazinyl group, a triazinyl group, a phenoxathiinyl group, an indolizinyl group, an indolyl group, a purinyl group, a quinolyl group, a benzothiazolyl group, a dibenzofuranyl group, a dibenzothiophenyl group, a phenanthrolinyl group, and a carbazolyl group.

In another embodiment, Ar₃ may be selected from the group consisting of hydrogen, deuterium (D), a cyano group (-CN), and the substituents S1-1 to S1-10.

In still another embodiment, Ar₃ may be hydrogen or may be selected from the group consisting of substituents S1 to S26.

In Chemical Formula 1 according to the present invention, the above-described nitrogen-containing heteroaromatic ring moiety is connected to a dibenzo-based moiety via a linker group (L₁). In this case, the linker group (L₁) connects the nitrogen-containing heteroaromatic ring moiety and the dibenzo-based moiety via at least one ortho-bond based on the linker group (L₁), and may be a linker group represented by Chemical Formula L1-1 or L1-2. For example, the linker group (L₁) may be an ortho-terphenylene group. Alternatively, the linker group (L₁) may be a terphenylene group in which a bonding site with another moiety corresponds to an ortho-position based on a central (second) benzene ring of the terphenylene group. Specifically, the nitrogen-containing heteroaromatic ring moiety may be bonded to the 2-position of a first benzene ring of the terphenylene group, which corresponds to an ortho-position based on a central (second) benzene ring of the terphenylene group, or the dibenzo-based moiety may be bonded to the 2-position of a third benzene ring of the terphenylene group, which corresponds to an ortho-position based on the central (second) benzene ring of the terphenylene group. For example, the nitrogen-containing heteroaromatic ring moiety and the dibenzo-based moiety may be bonded via the terphenylene group in (i) an ortho-(meta or para)-(meta or para) linkage, (ii) a (meta or para)-(meta or para)-ortho linkage, (iii) an ortho-(meta or para)-ortho linkage, or (iv) an (ortho-meta or para)-ortho-(ortho-meta or para) linkage.
wherein in Chemical Formula L1-1 or L1-2, a dotted line indicates that each ring is present or absent, and Cy₁ to Cy₃ may be aromatic rings fused to a benzene ring, may be the same as or different from each other, and are each independently a 6-membered aromatic ring. When each of Cy₁ to Cy₃ is present, the linker group may include naphthalene
wherein in the linker represented by Chemical Formula L1-1 or L1-2, c, d, and e may each independently be an integer of 0 to 6, specifically may each independently be an integer of 0 to 3, and more specifically may each independently be 0 or 1.

Here, when c is 0, it means that hydrogen is not substituted with substituent R₃; when d is 0, it means that hydrogen is not substituted with substituent R₄; and when e is 0, it means that hydrogen is not substituted with substituent R₃. Meanwhile, when c is an integer of 1 to 6, it means that hydrogen is substituted with substituent R₃; when d is an integer of 1 to 6, it means that hydrogen is substituted with substituent R₄; and when e is an integer of 1 to 6, it means that hydrogen is unsubstituted with substituent R₅.

Here, when plural R₃'s are present, the R₃'s may be the same as or different from each other; when plural R₄'s are present, the R₄'s may be the same as or different from each other; and when plural R₅'s are present, the R₅'s may be the same as or different from each other.

R₃ to R₅ are the same as or different from each other and are each independently selected from the group consisting of hydrogen, deuterium (D), a halogen (e. g., -F, -Cl, -Br, -I), a cyano group(-CN), a nitro group(-NO₂), an amino group (-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may be bonded to any adjacent group (e.g., R₃-R₃, R₄-R₄, R₅-R₅, R₃-R₄, R₄-R₅ and the like) to form a fused ring. Specifically, R₃ to R₅ are each independently selected from the group consisting of hydrogen, deuterium (D), a cyano group (-CN), a C₁ to C₂₀ alkyl group, a C₃ to C₂₀ cycloalkyl group, a heterocycloalkyl group having 3 to 20 ring atoms, a C₆ to C₃₀ aryl group, and a heteroaryl group having 5 to 30 ring atoms.

The alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, arylamine group and the fused ring of R₃ to R₅ may each independently be unsubstituted or substituted with at least one substituent selected from the group consisting of deuterium (D), a halogen (e. g., -F, -Cl, -Br, -I and the like), a cyano group (-CN), a nitro group(-NO₂), an amino group (-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group. Specifically, each of the groups may be substituted or unsubstituted with at least one substituent selected from the group consisting of deuterium (D), a halogen (e.g., -F, -Cl, -Br, and -I), a cyano group (-CN), a nitro group (-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group. When two or more substituents are present, they may be the same as or different from each other.

In one embodiment, L₁ may be a linker represented by any one of the following Chemical Formulas L2-1 to L2-5, but are not limited thereto. 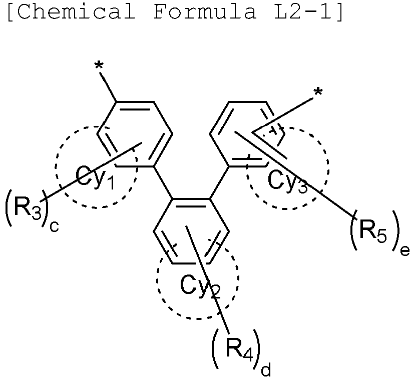 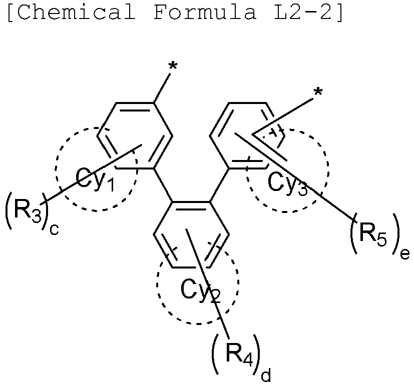 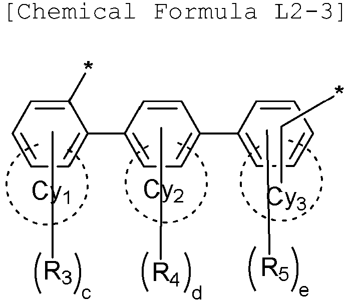 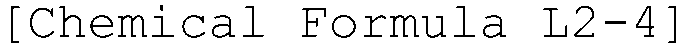 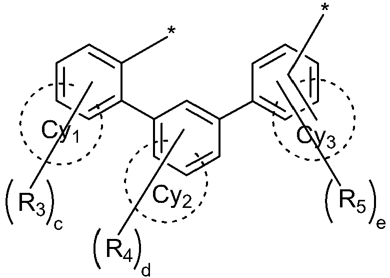 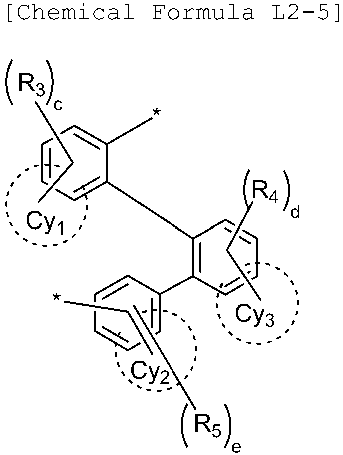
wherein in Chemical Formulae L2-1 or L2-5,
* indicates a site where a bond with Chemical Formula 1 is made, and
Cy₁ to Cy₃, c, d, e, R₃ to R₄ are each as defined in Chemical Formula 1.

Depending on the bonding configuration within the linker group (L₁), the compound represented by Chemical Formula 1 may be a compound represented by any one of the following Chemical Formulas 2 to 20, but is not limited thereto. 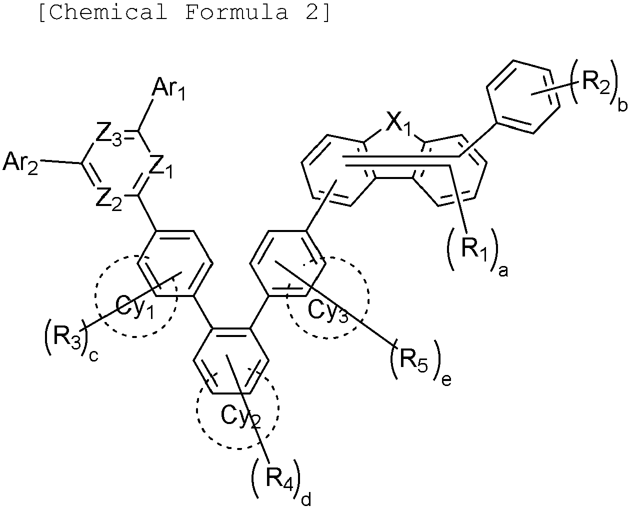 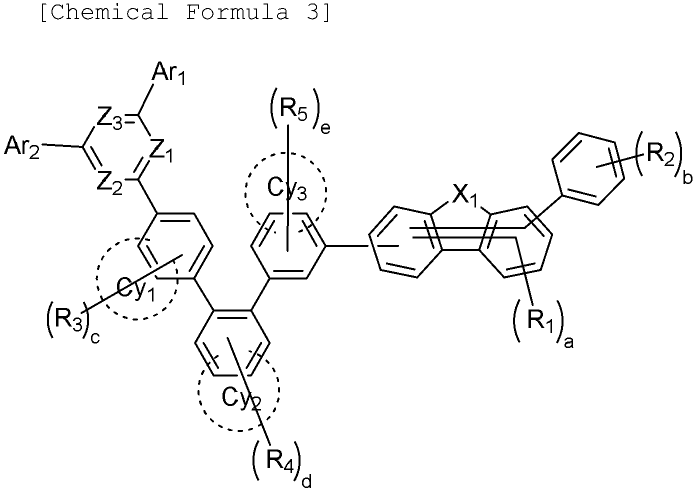 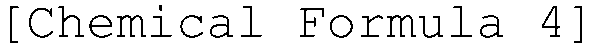 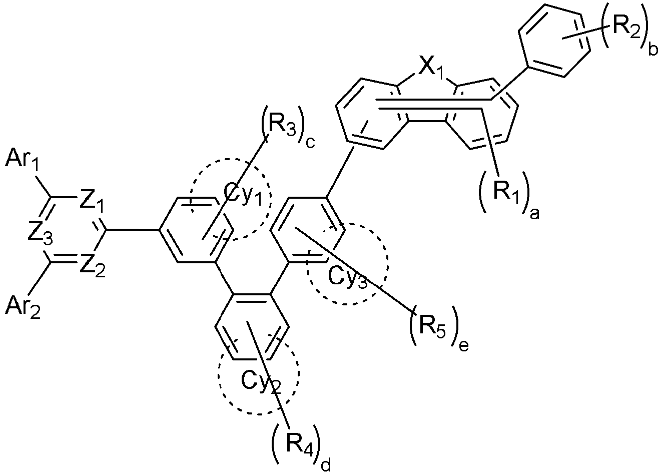 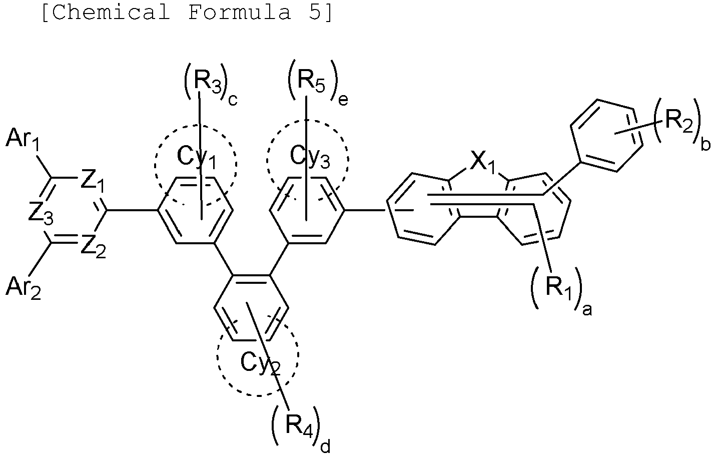 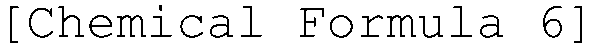 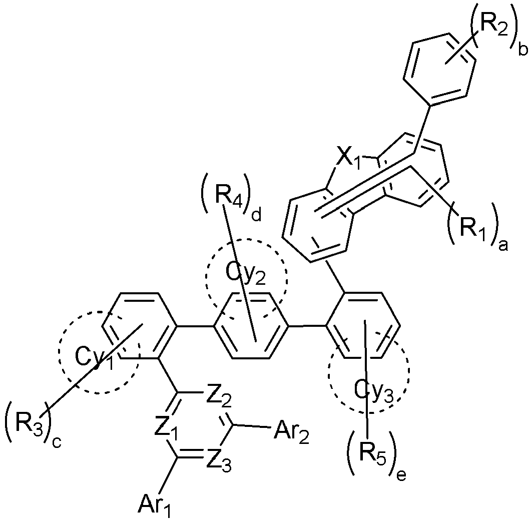 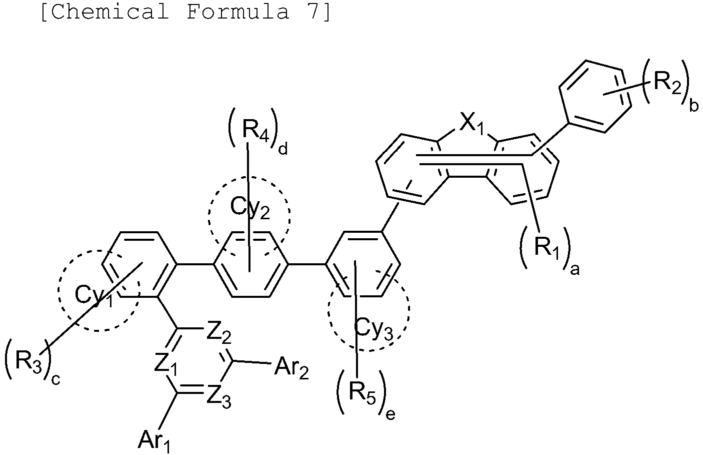 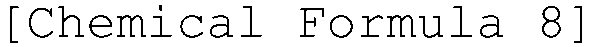 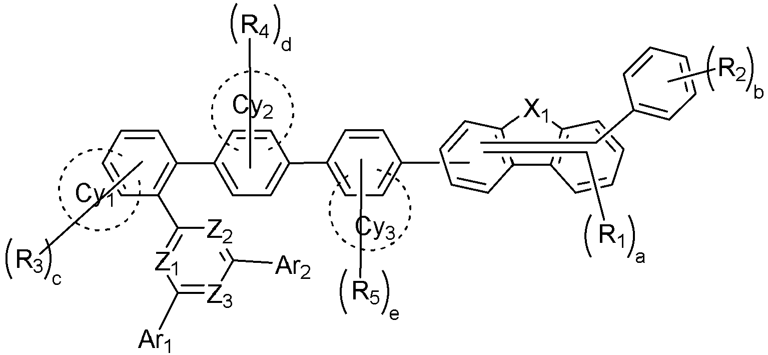 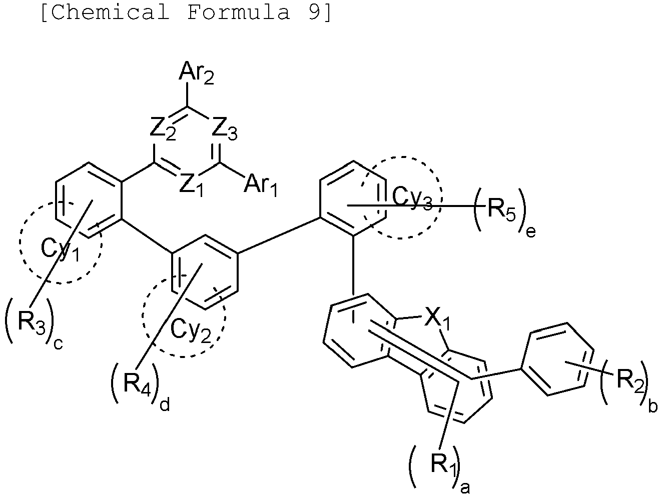 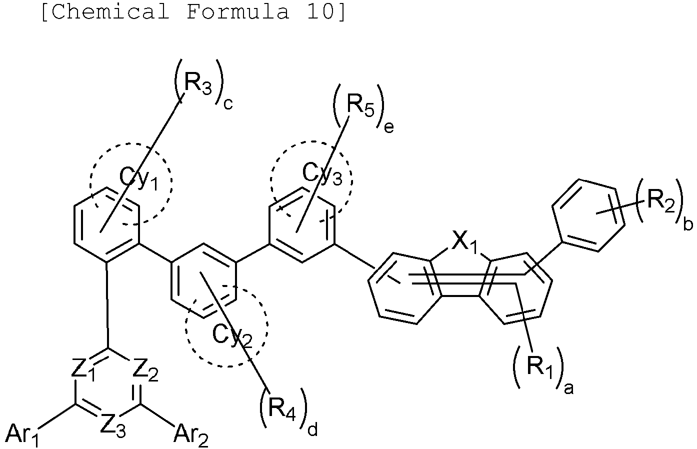 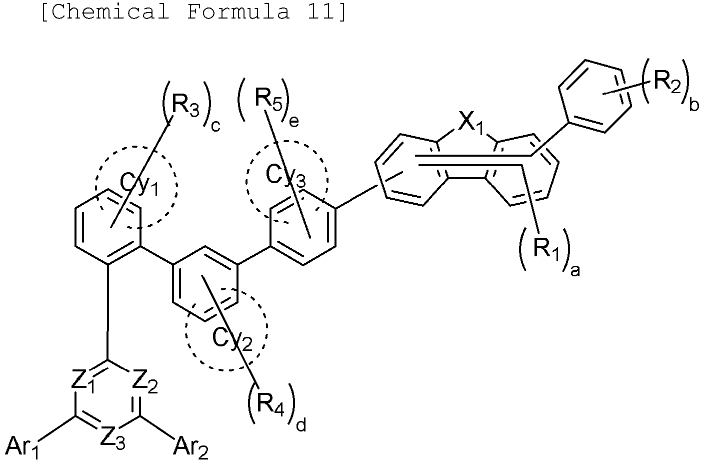 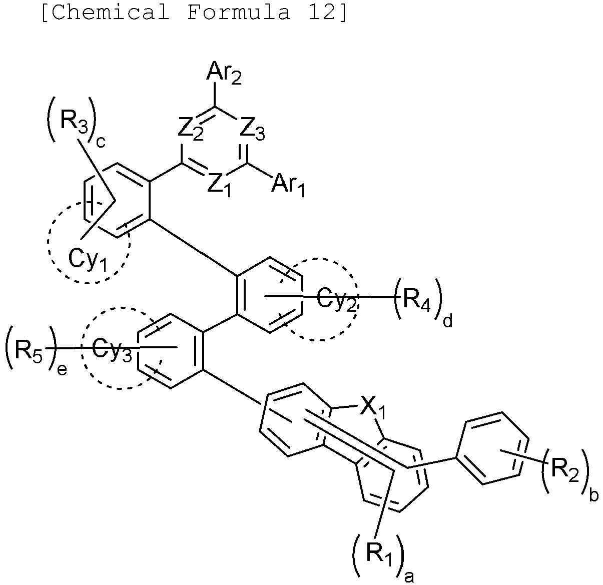 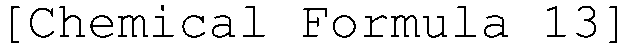 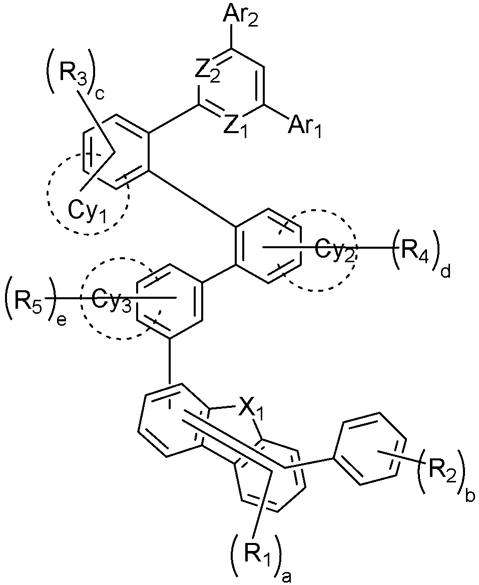 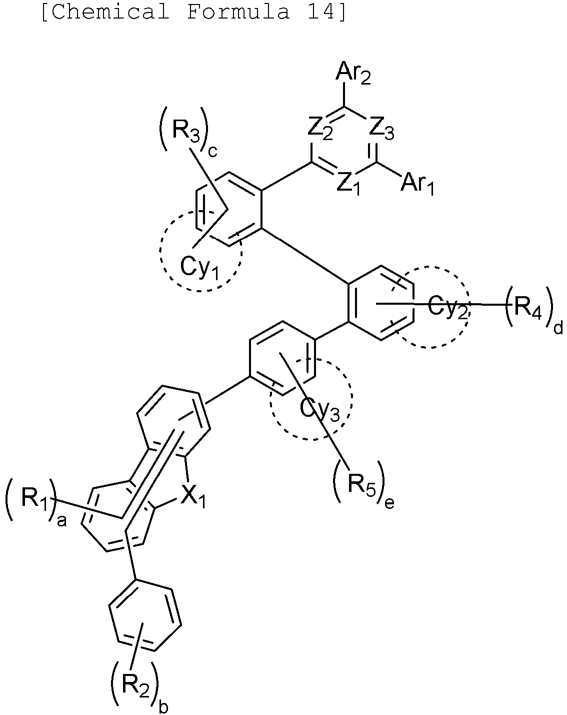 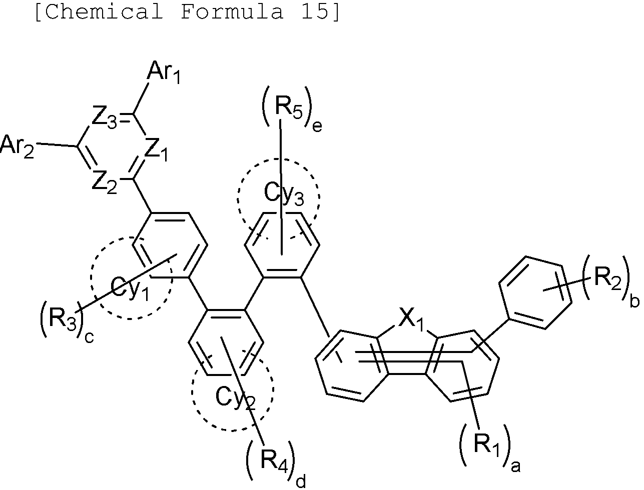 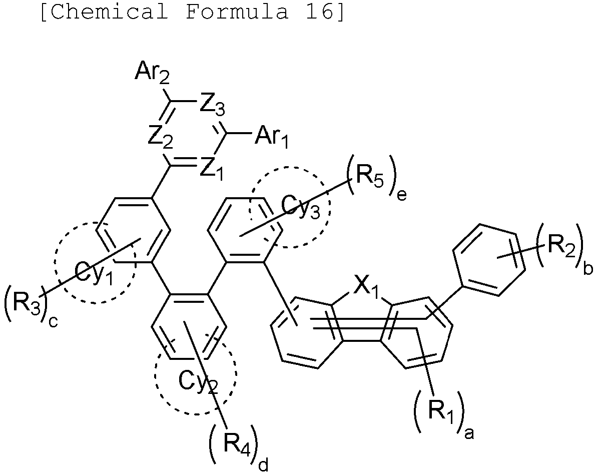 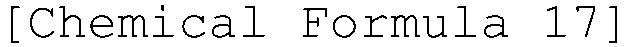 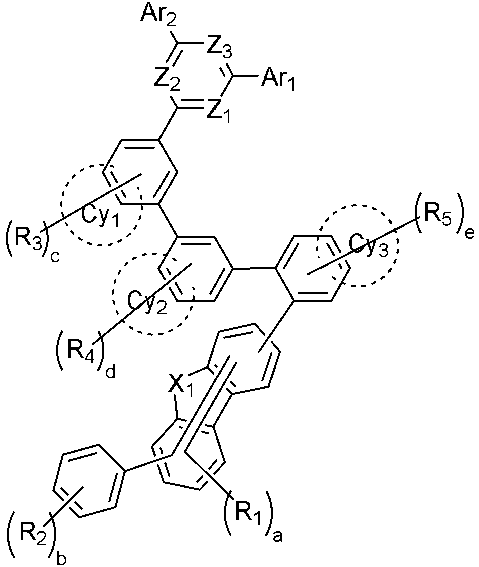 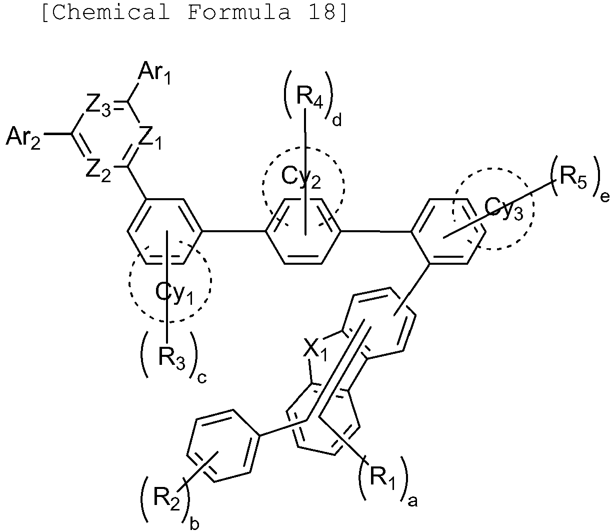 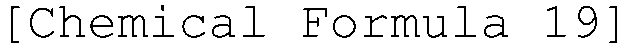 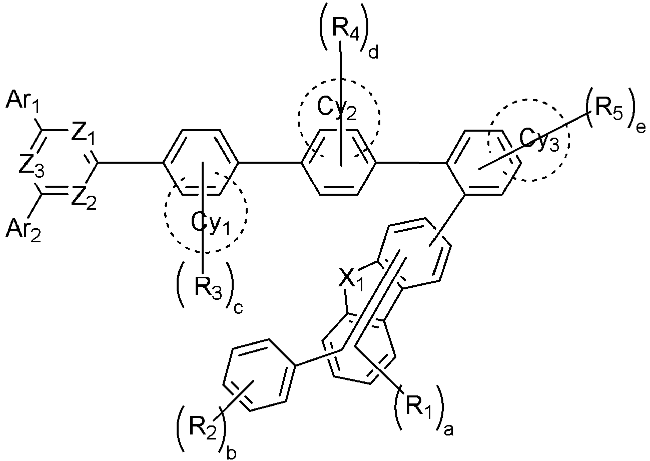 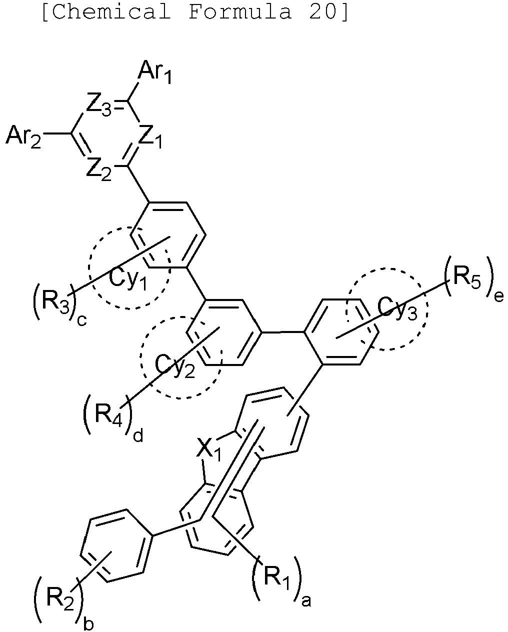
wherein in Chemical Formulas 2 to 20,
Z₁ to Z₃, X₁, Ar₁, Ar₂, the dotted line, Cy₁ to Cy₃, a, b, c, d, e, R₁ to R₅ are each as defined in Chemical Formula 1.

In Chemical Formula 1 according to the present invention, X₁ is selected from the group consisting of O, S, C(Ar₄)(Ar₅), N(Ar₆) and 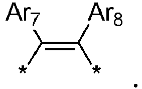 The X₁-containing ring moiety is a kind of dibenzo-based moiety, and depending on X₁, may be a dibenzofuran-based moiety (X₁ = O), a dibenzothiophene-based moiety (X₁ = S), a fluorene-based moiety [X₁ = C(Ar₄)(Ar₅)], a carbazole-based moiety [X₁ = N(Ar₆)], or a phenanthrene-based moiety (X₁ = 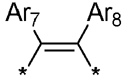 ).

In Chemical Formula 1, Ar₄ to Ar₈ are the same as or different from each other and are each independently selected from the group consisting of hydrogen, deuterium (D), a halogen (e. g., -F, -Cl, -Br, -I), a cyano group (-CN), a nitro group (-NO₂), an amino group (-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may be bonded to any adjacent group (e.g., Ar₄-Ar₅, Ar₄-R₁, Ar₆-R₁, Ar₇-Ar₈, Ar₇-R₁ and the like) to form a fused ring. Specifically, Ar₄ to Ar₈ are each independently selected from the group consisting of hydrogen, deuterium (D), a cyano group (-CN), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group, or may be bonded to any adjacent group (e.g., Ar₄-Ar₅, Ar₄-R₁, Ar₆-R₁, Ar₇-Ar₈, Ar₇-R₁ and the like) to form a fused ring. Here, the fused ring may be at least one selected from the group consisting of a C₃ to C₆₀ fused aliphatic ring (specifically, a C₃ to C₃₀ fused aliphatic ring), a C₆ to C₆₀ fused aromatic ring (specifically, a C₆ to C₃₀ fused aromatic ring), a 5- to 60-membered fused heteroaromatic ring (specifically, a 5- to 30-membered fused heteroaromatic ring), a C₃ to C₆₀ spiro ring, and combinations thereof.
the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, arylamine group and the fused ring of Ar₄ to Ar₈ may each independently be unsubstituted or substituted with at least one substituent selected from the group consisting of deuterium (D), a halogen (e. g., -F, -Cl, -Br, -I and the like), a cyano group(-CN), a nitro group(-NO₂), an amino group(-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group. Specifically, each of the groups may be substituted or unsubstituted with at least one substituent selected from the group consisting of deuterium (D), a halogen (e.g., -F, -Cl, -Br, and -I), a cyano group (-CN), a nitro group (-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group. When two or more substituents are present, they may be the same as or different from each other.

In one embodiment, The X₁-containing ring moiety ( 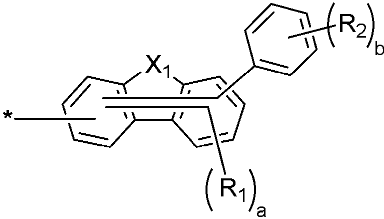 moiety) may be a moiety selected from the group consisting of Mo1-1 to Mo1-10, but is not limited thereto. 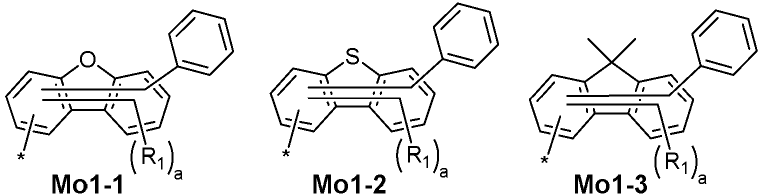 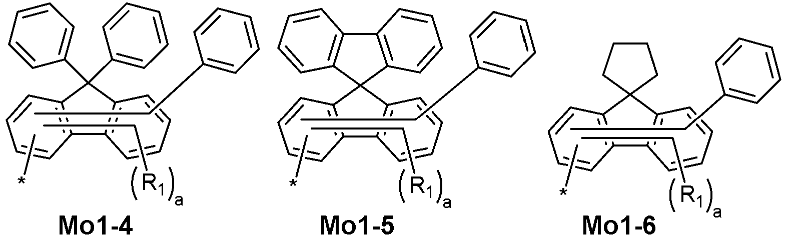 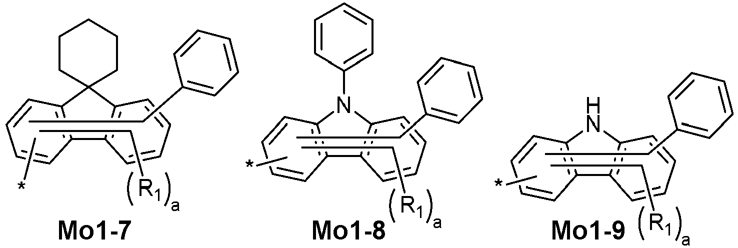 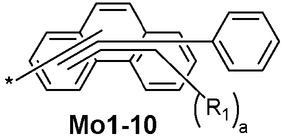
wherein in moieties Mo1-1 to Mo1-10,
* indicates a site where a bond with Chemical Formula 1 is made,
a may be an integer of 0 to 6, specifically may be an integer of 0 to 2, and more specifically may be 0 or 1, and
when plural R₁'s are present, the R₁'s may be the same as or different from each other, and
R₁ is as defined in Chemical Formula 1.

In another embodiment, The X₁-containing ring moiety may be a moiety selected from the group consisting of Mo2-1 to Mo2-10, but is not limited thereto. 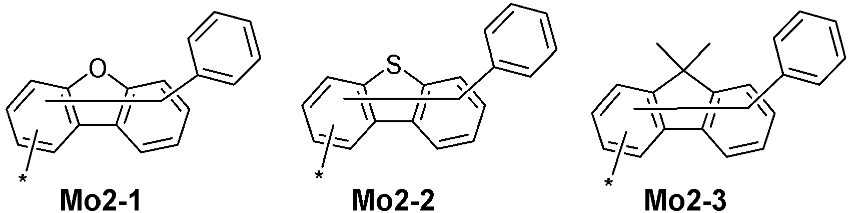 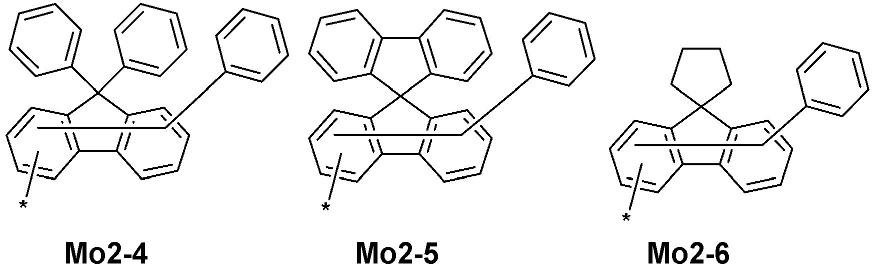 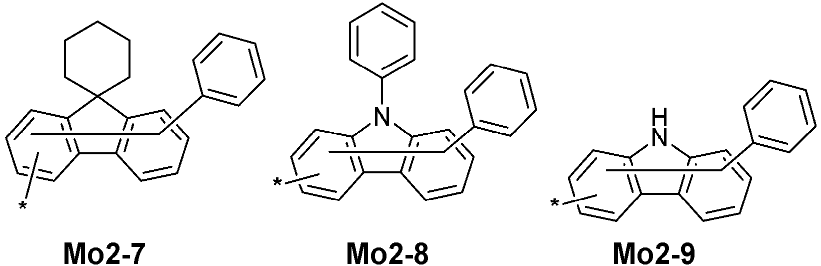 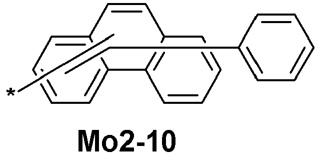
wherein in moieties Mo2-1 to Mo2-10,
* indicates a site where a bond with Chemical Formula 1 is made.

In Chemical Formula 1 according to present invention, a may be an integer of 0 to 6, specifically may be an integer of 0 to 3, and more specifically be 0 or 1. b may be an integer of 0 to 5, specifically may be an integer of 0 to 3, and more specifically be 0 or 1.

Here, when a is 0, it means that hydrogen is not substituted with substituent R₁; and when b is 0, it means that hydrogen is not substituted with substituent R₂. Meanwhile, when a is an integer of 1 to 6, it means that hydrogen is substituted with substituent R₁; and when b is an integer of 1 to 5, it means that hydrogen is substituted with substituent R₂.

Here, when plural R₁'s are present, the R₁'s may be the same as or different from each other; and when plural R₂'s are present, the R₂'s may be the same as or different from each other.

R₁ to R₂ are the same as or different from each other and are each independently selected from the group consisting of hydrogen, deuterium (D), a halogen (e. g., -F, -Cl, -Br, -I), a cyano group(-CN), a nitro group(-NO₂), an amino group(-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or may be bonded to any adjacent group (e.g., R₁-R₁, R₂-R₂, R₁-R₂ and the like) to form a fused ring. Specifically, R₁ to R₂ are each independently selected from the group consisting of hydrogen, deuterium (D), a cyano group(-CN), a C₁ to C₂₀ alkyl group, a C₃ to C₂₀ cycloalkyl group, a heterocycloalkyl group having 3 to 20 ring atoms, a C₆ to C₃₀ aryl group, and a heteroaryl group having 5 to 30 ring atoms.
the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, arylamine group and the fused ring of R₁ to R₂ may each independently be unsubstituted or substituted with at least one substituent selected from the group consisting of deuterium (D), a halogen (e. g., -F, -Cl, -Br, -I and the like), a cyano group(-CN), a nitro group(-NO₂), an amino group(-NH₂), a hydroxy group (-OH), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group. Specifically, each of the groups may be substituted or unsubstituted with at least one substituent selected from the group consisting of deuterium (D), a halogen (e.g., -F, -Cl, -Br, and -I), a cyano group (-CN), a nitro group (-NO₂), an amino group (-NH₂), a hydroxy group (-OH), a C₁ to C₂₀ alkyl group, a C₆ to C₃₀ aryl group, a heteroaryl group having 5 to 30 ring atoms, a C₆ to C₃₀ arylphosphine oxide group, and a C₆ to C₃₀ arylamine group. When two or more substituents are present, they may be the same as or different from each other.

Depending on the bonding configuration between the two moieties centered on L₁ and the presence or absence of Cy₁ to Cy₃ in L₁, the compound represented by Chemical Formula 1 may be a compound represented by any one of Chemical Formulas 21 to 115 below, but is not limited thereto. 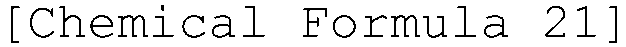 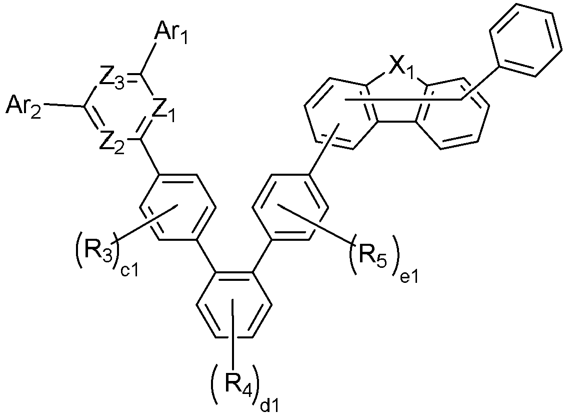 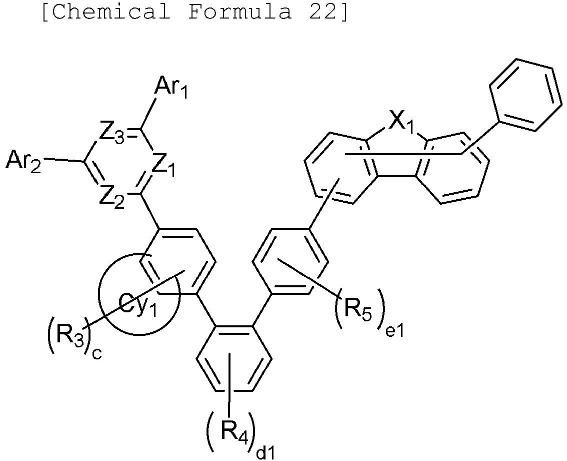 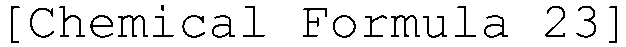 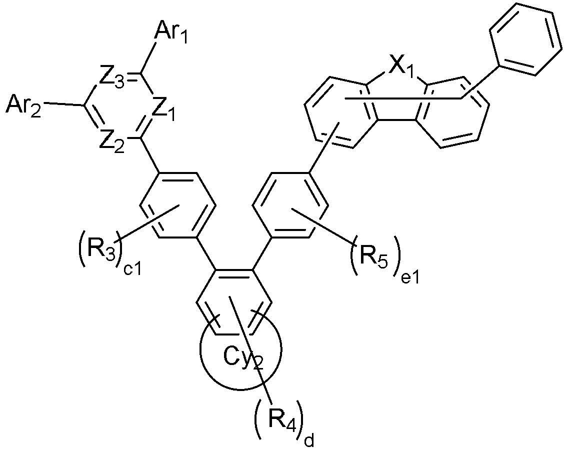 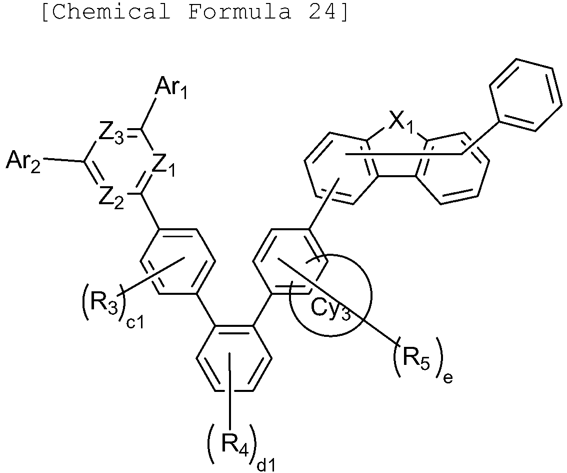 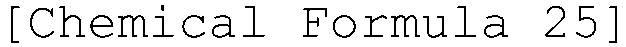 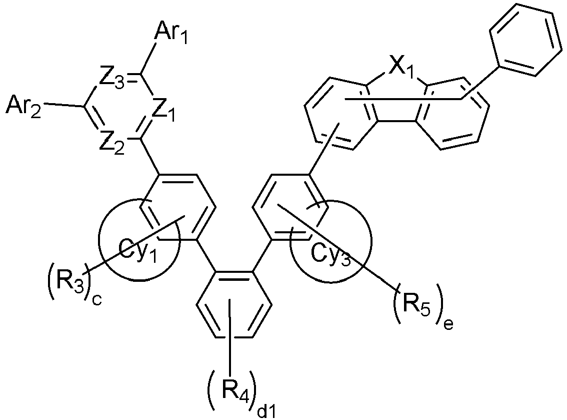 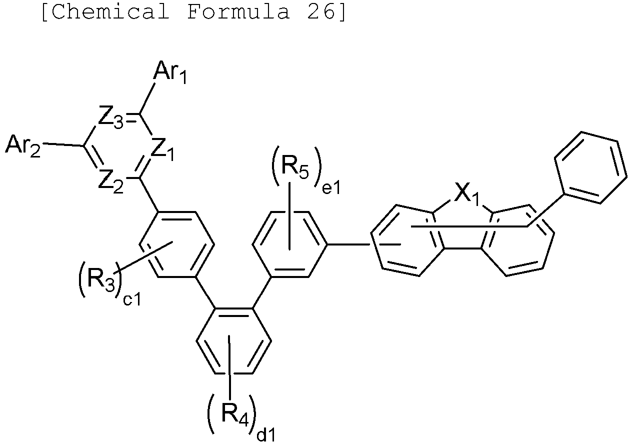 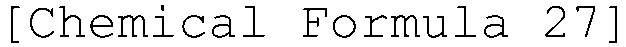 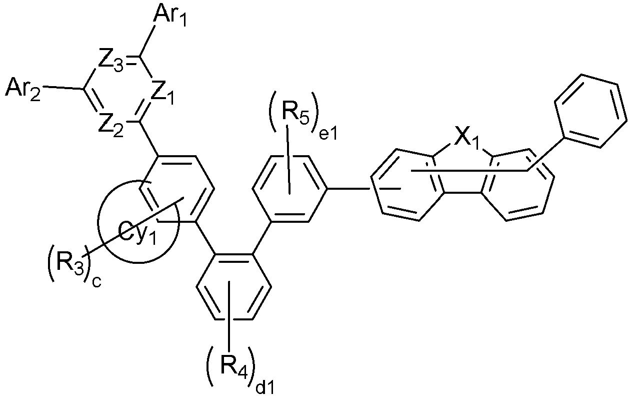 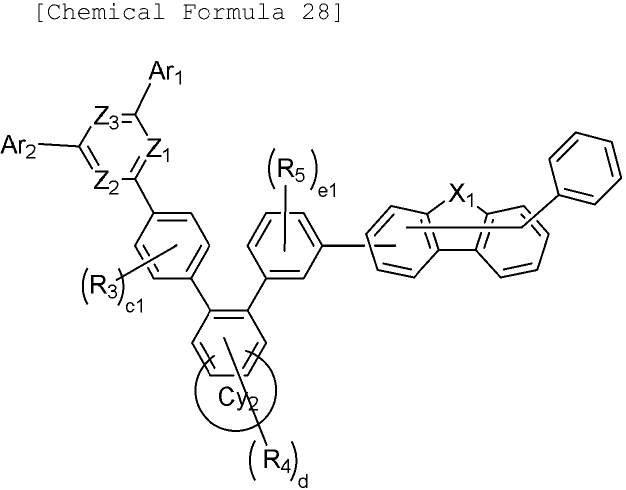 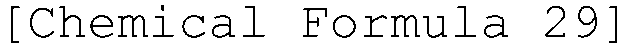 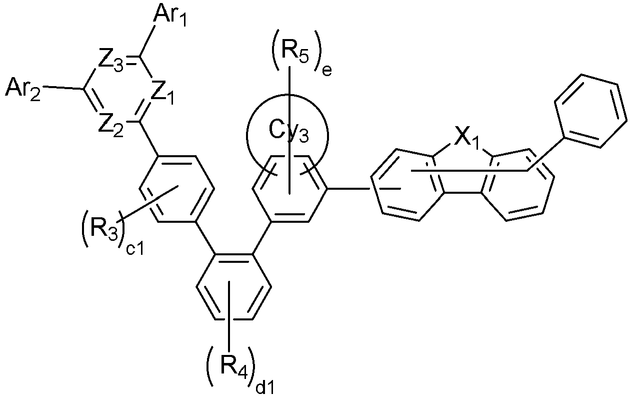 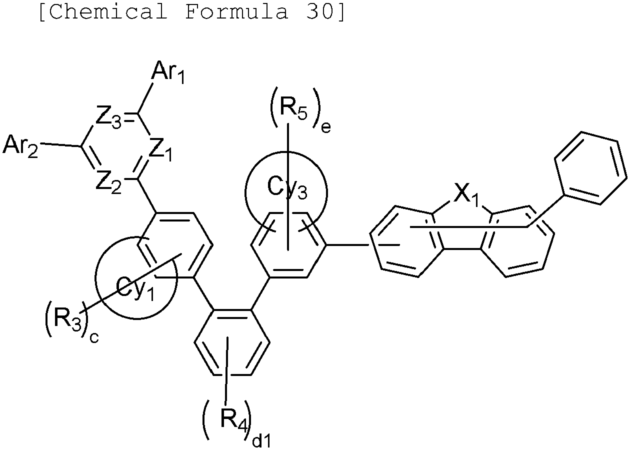 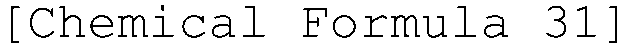 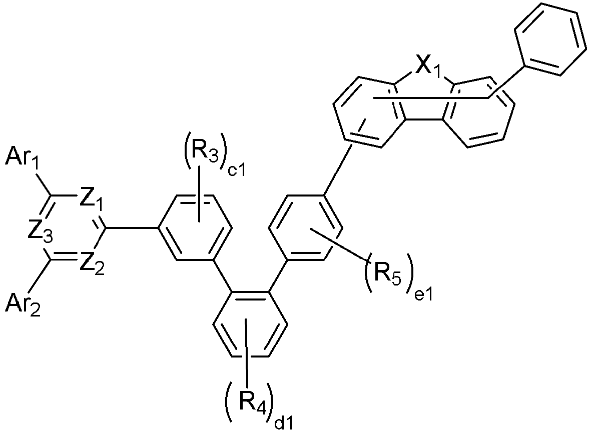 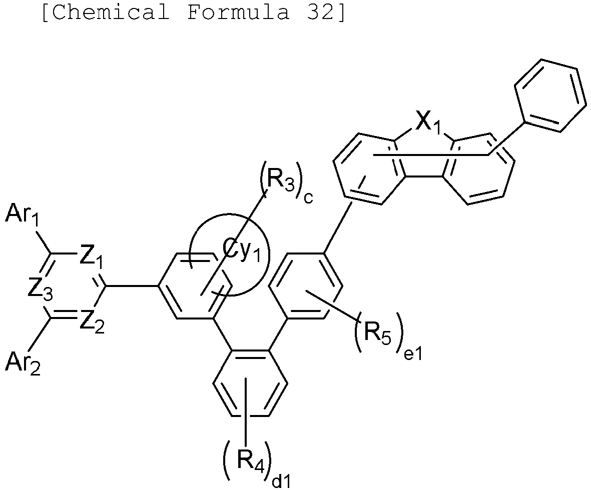 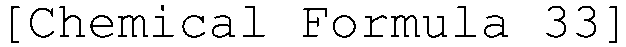 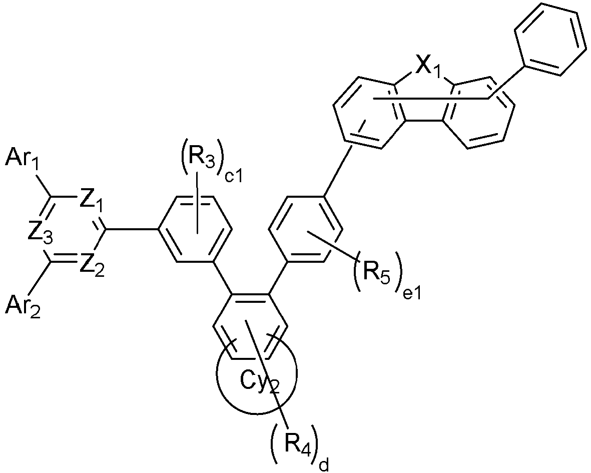 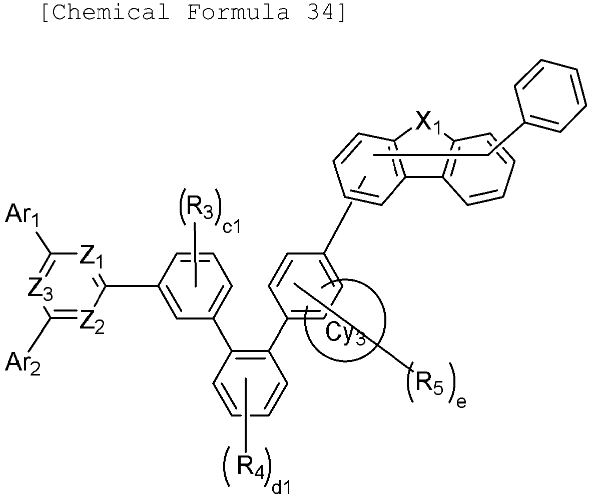 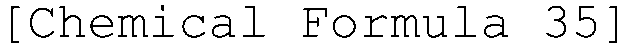 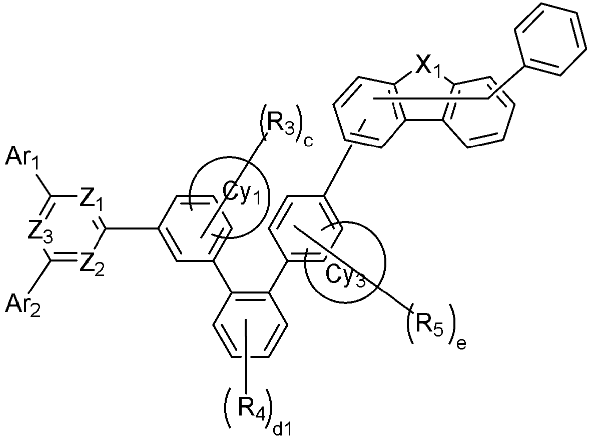 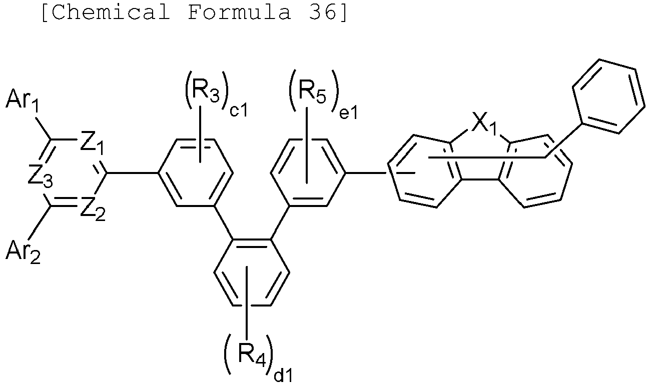 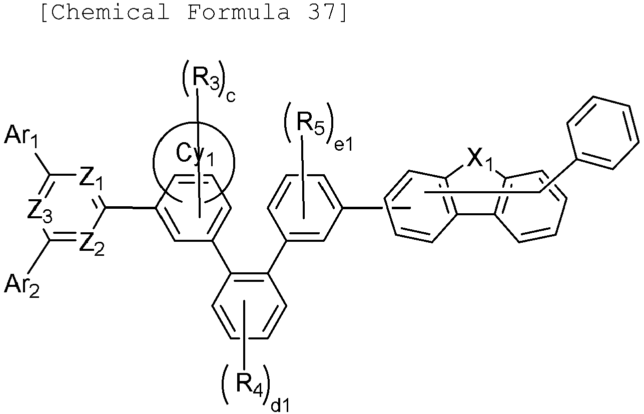 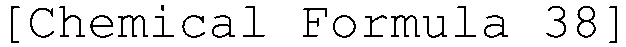 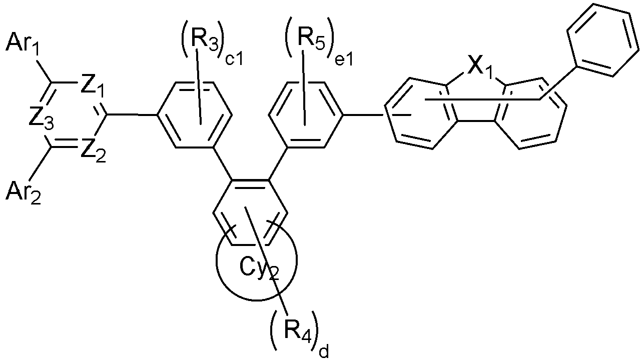 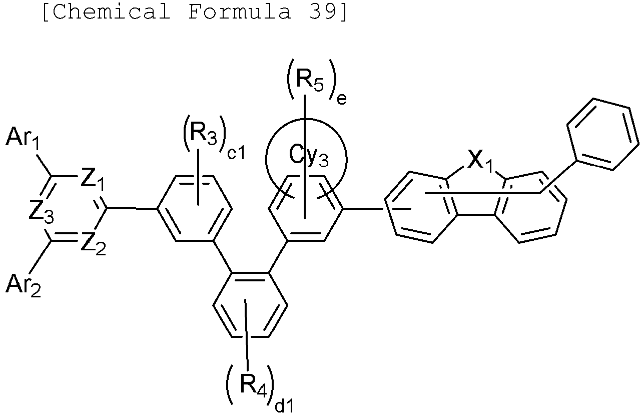 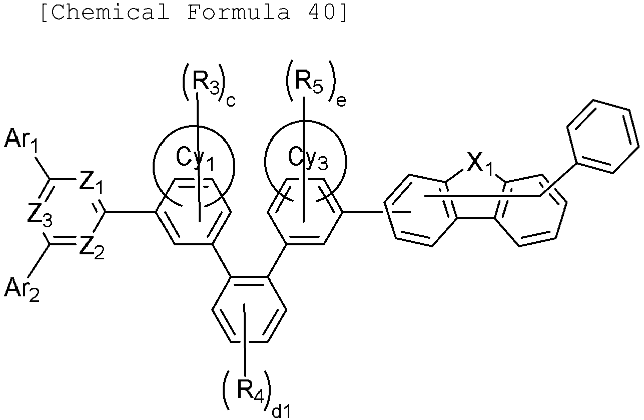 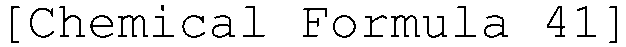 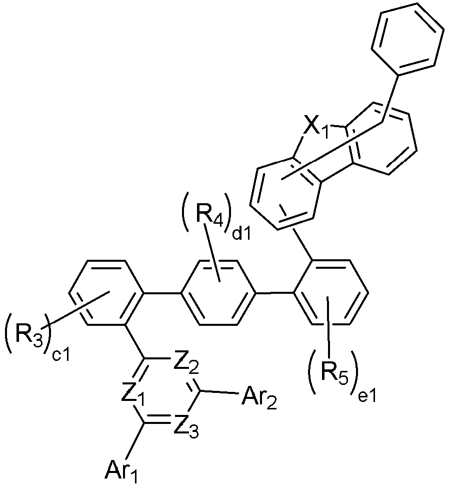 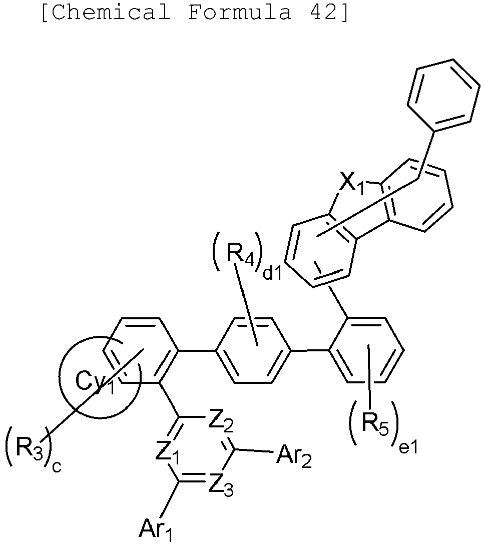 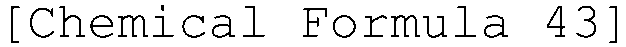 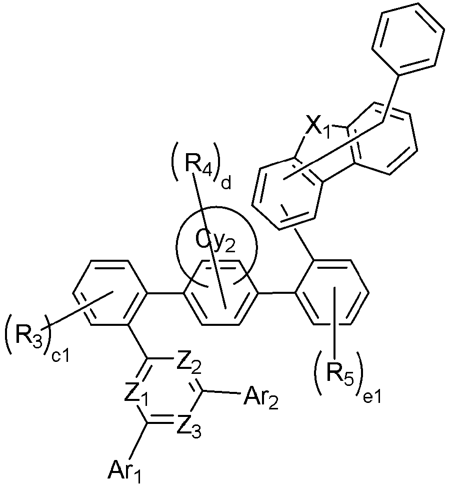 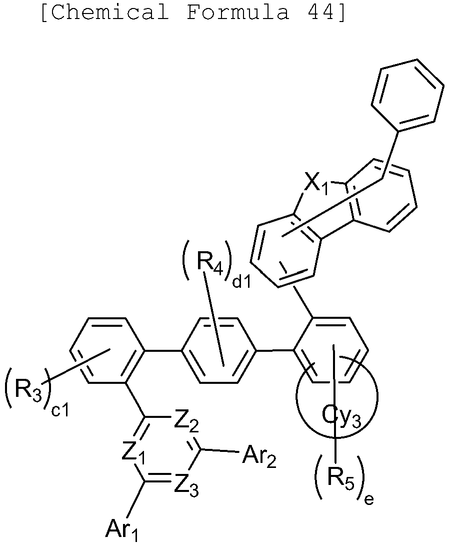 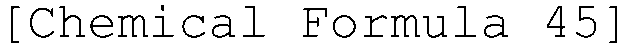 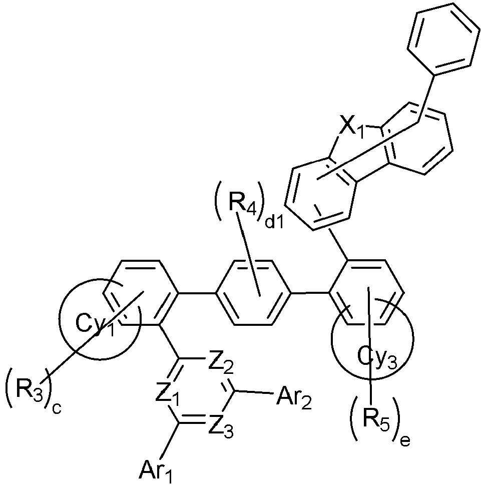 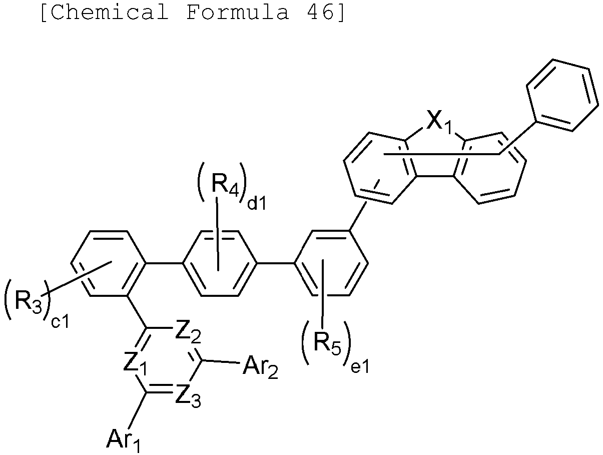 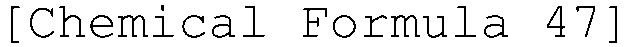 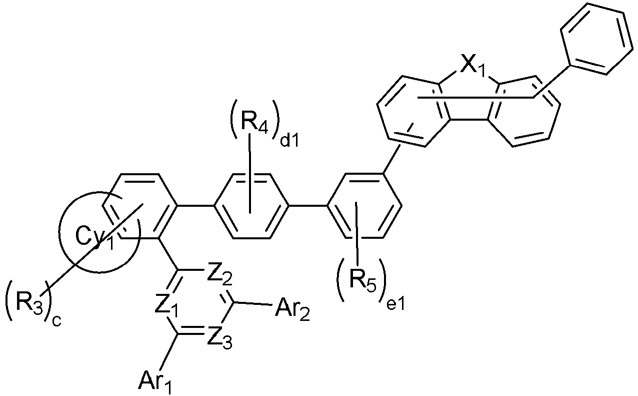 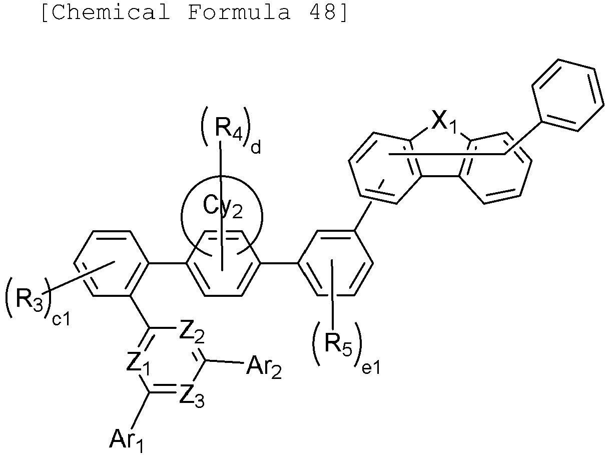 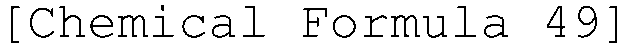 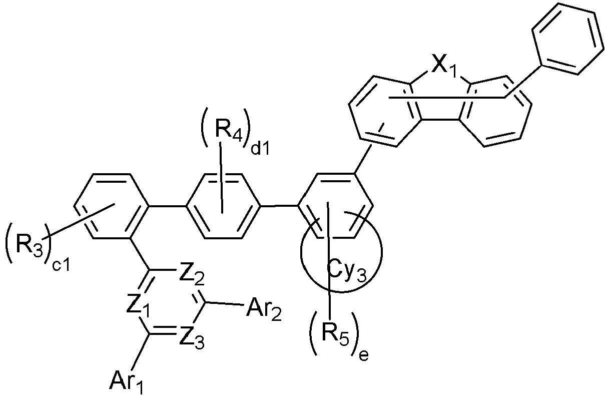 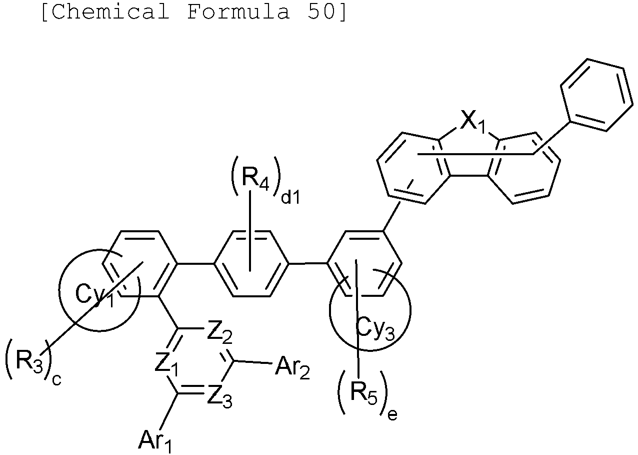 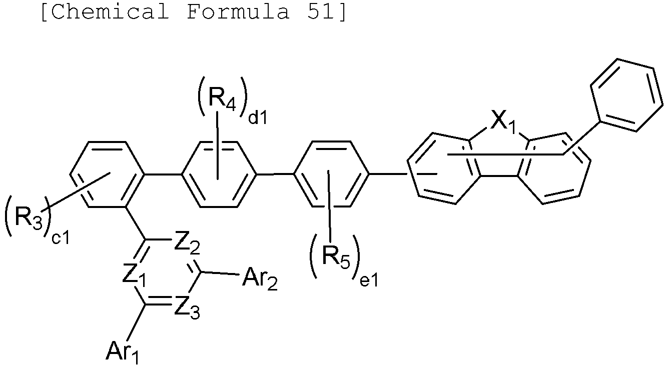 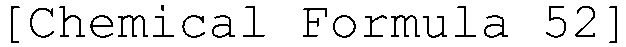 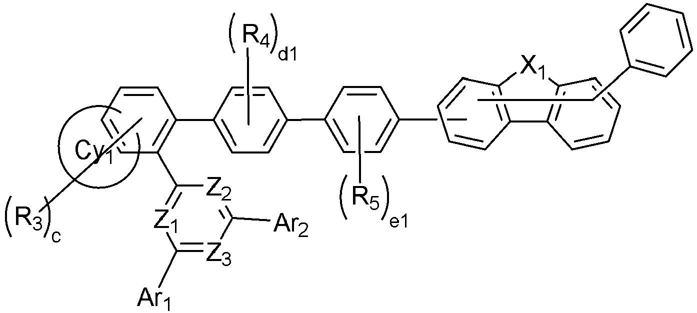 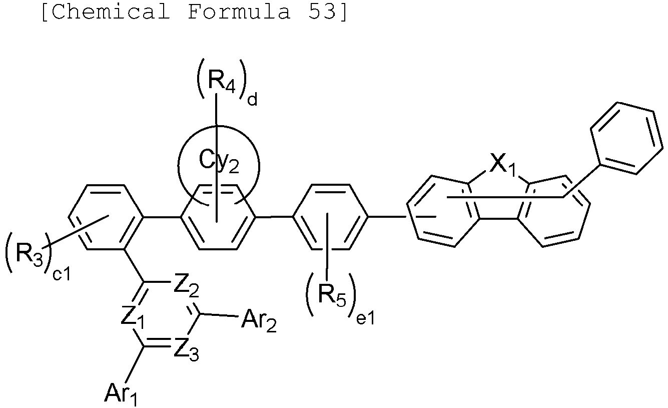 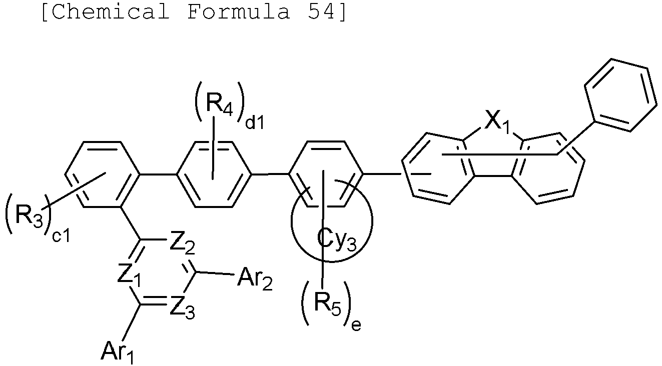 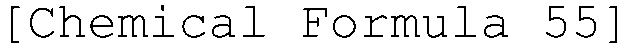 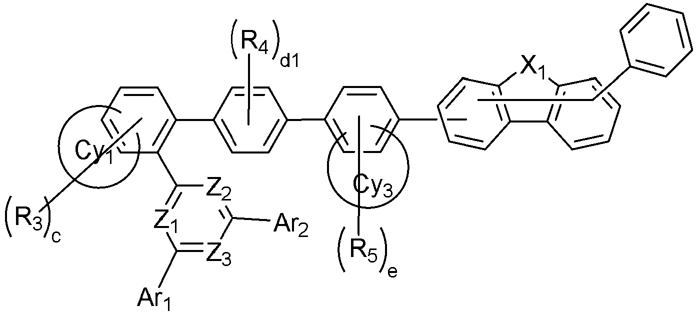 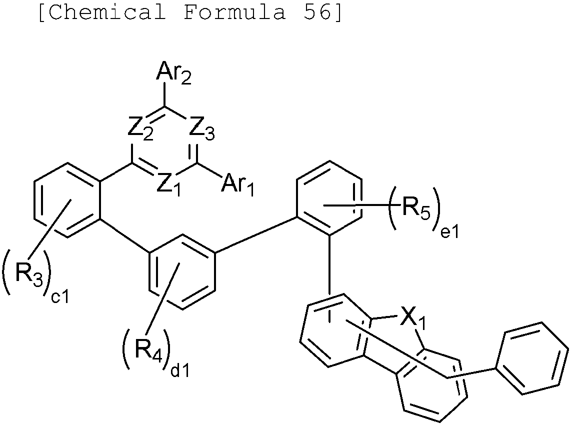 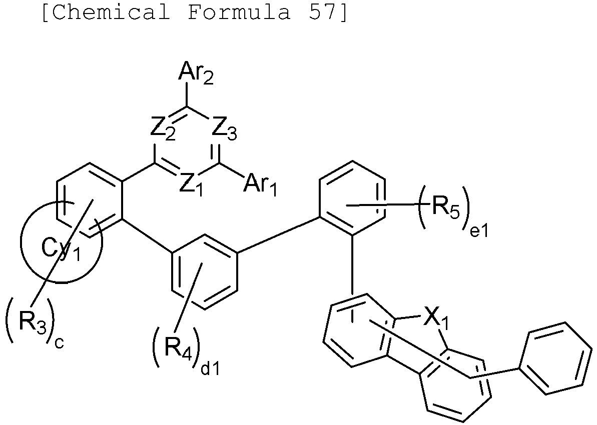 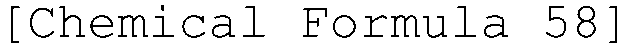 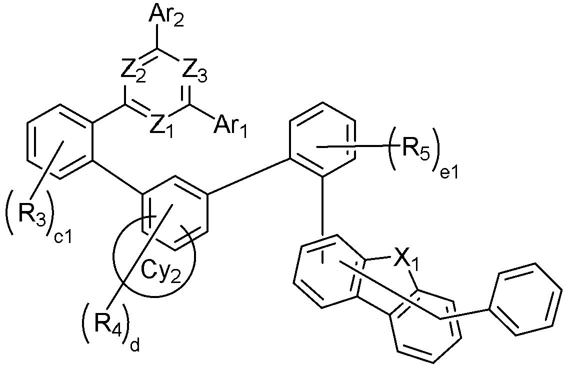 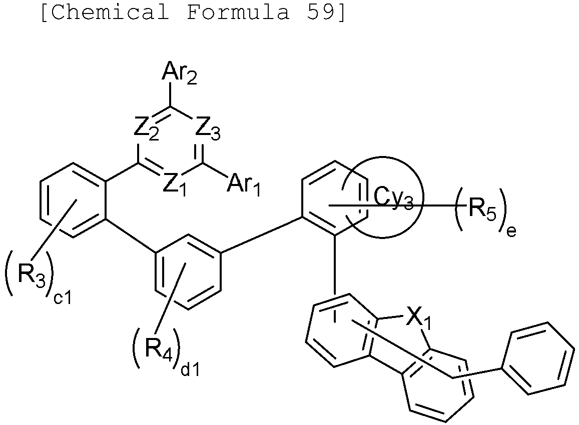 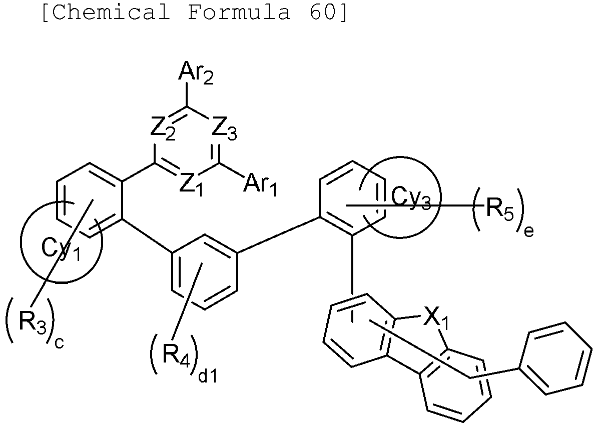 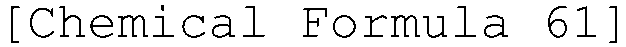 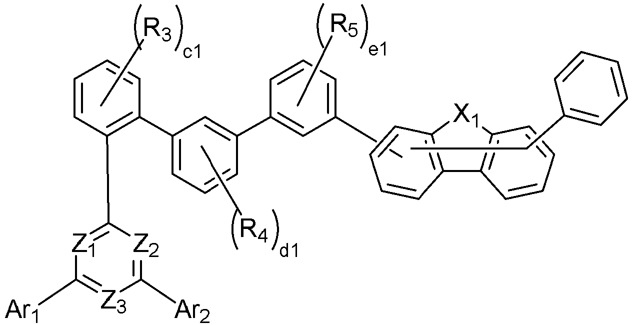 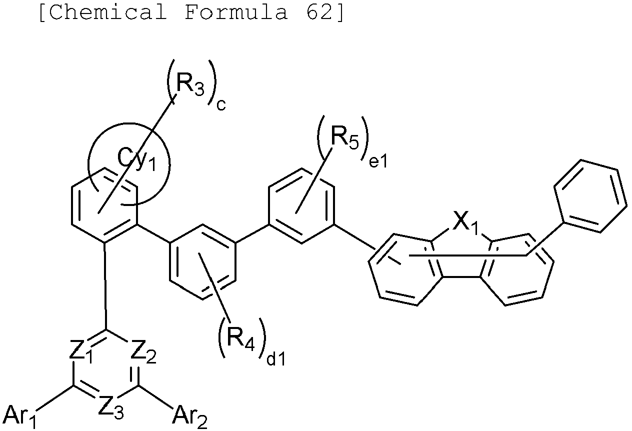 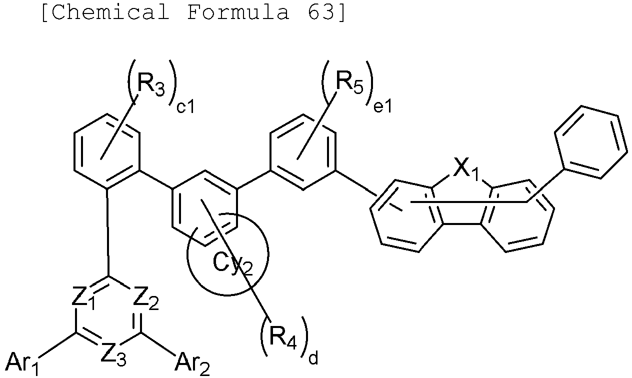 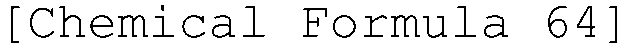 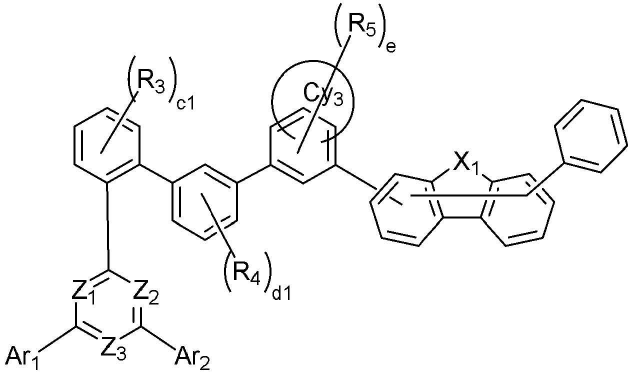 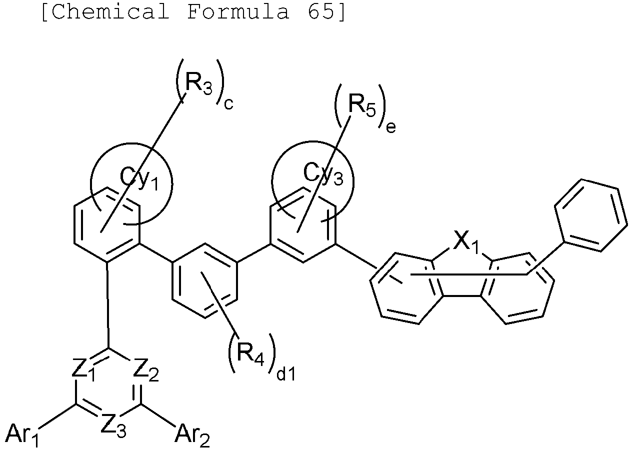 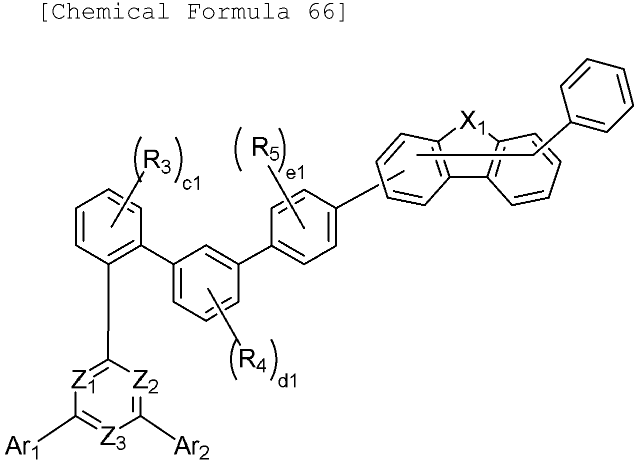 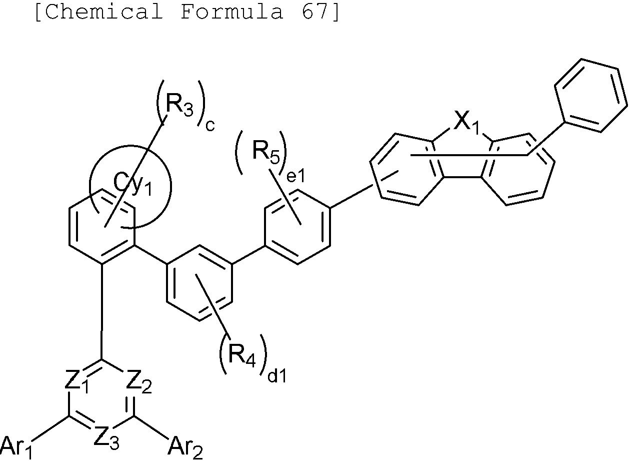 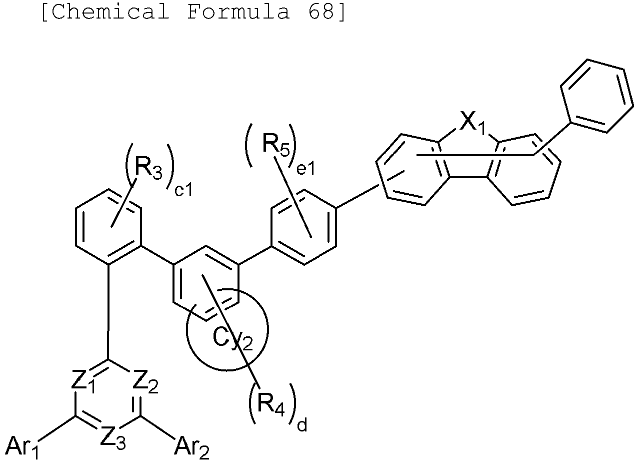 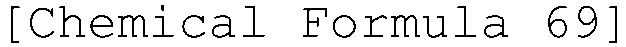 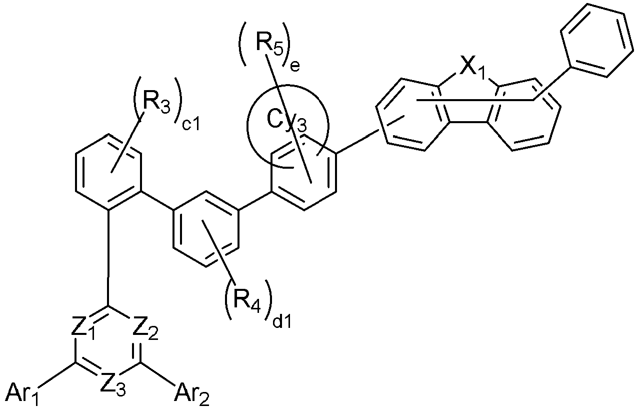 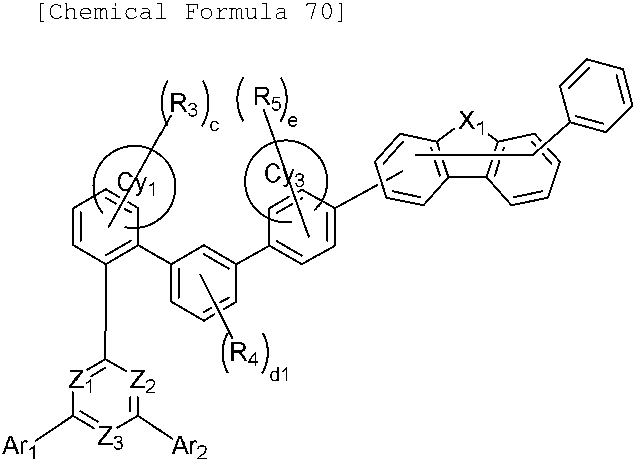 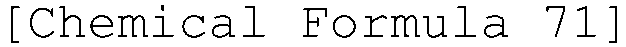 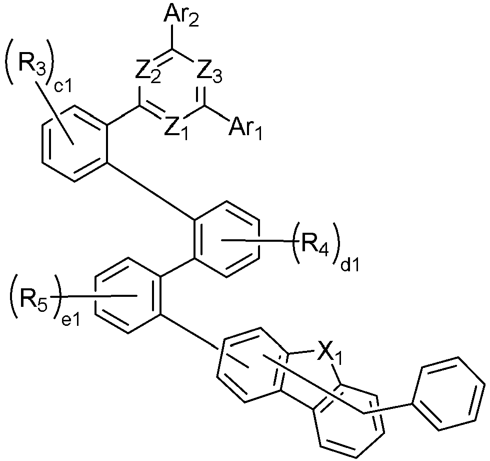 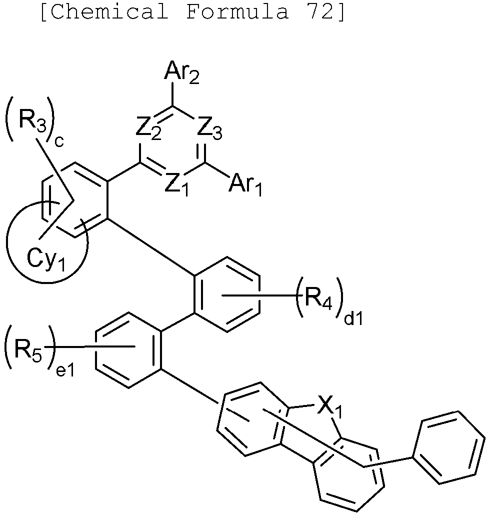 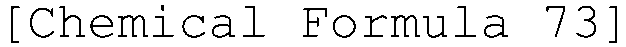 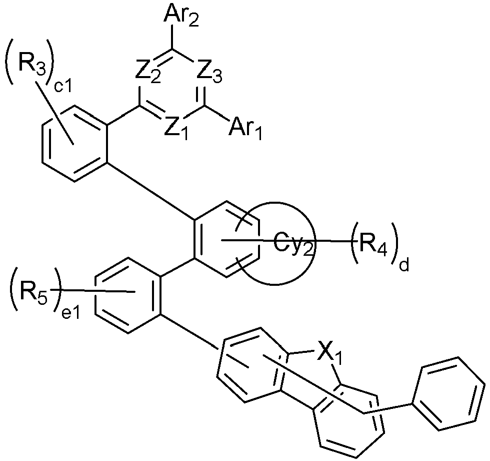 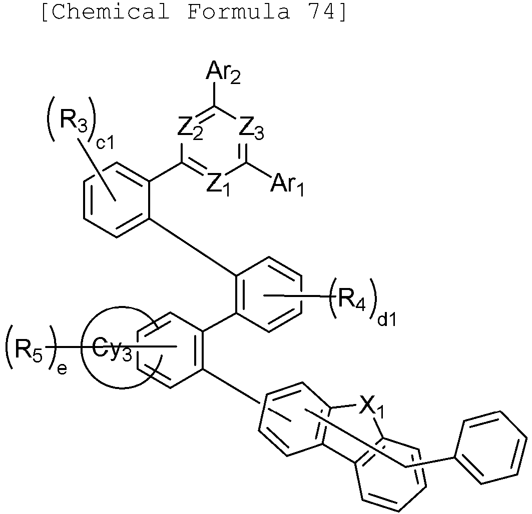 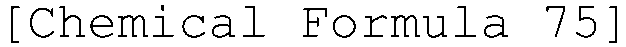 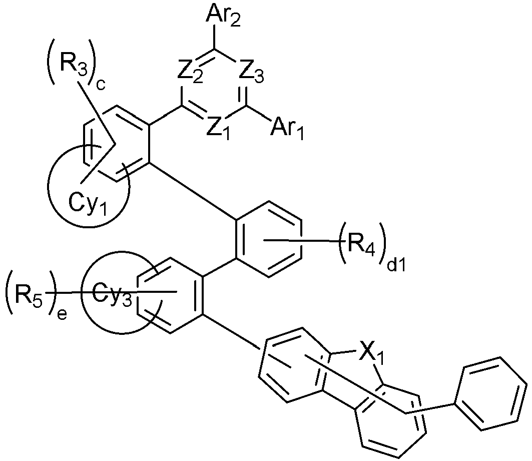 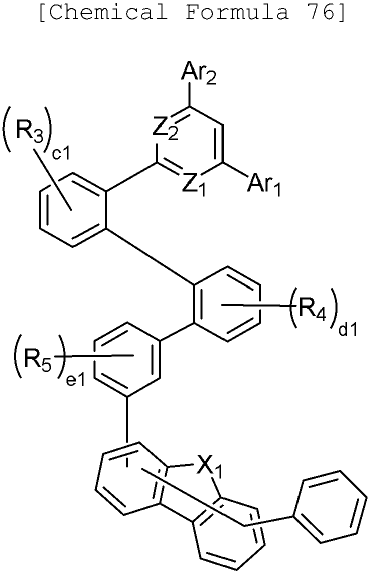 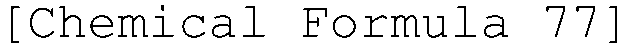 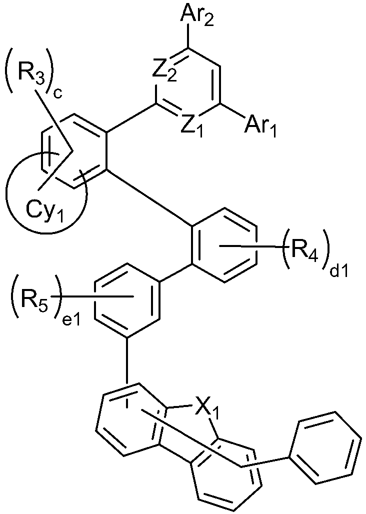 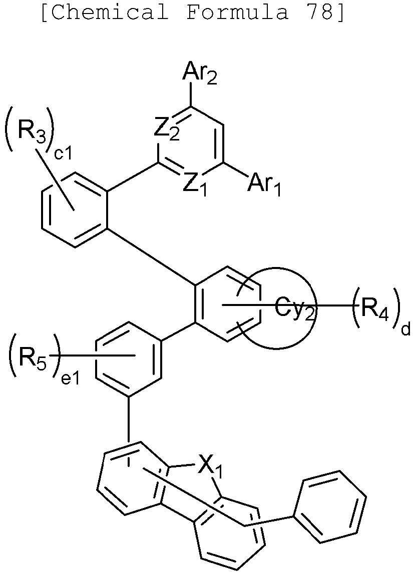 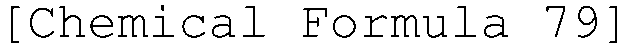 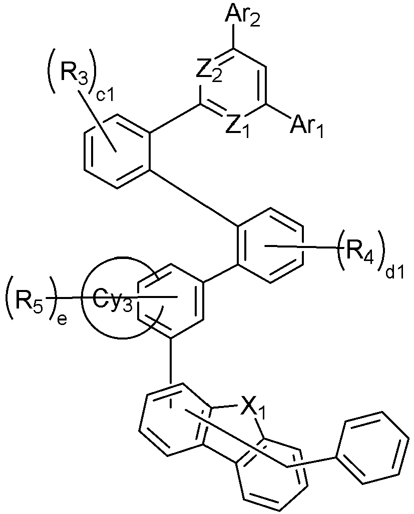 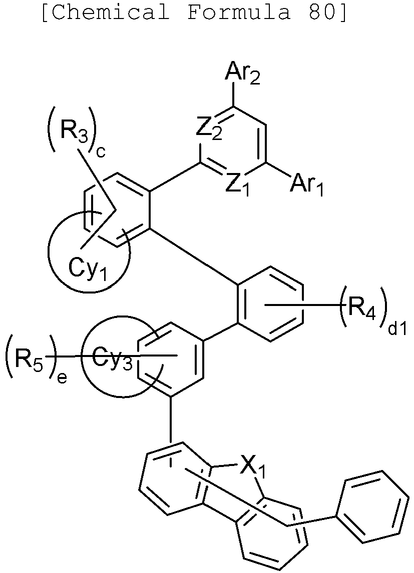 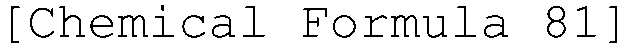 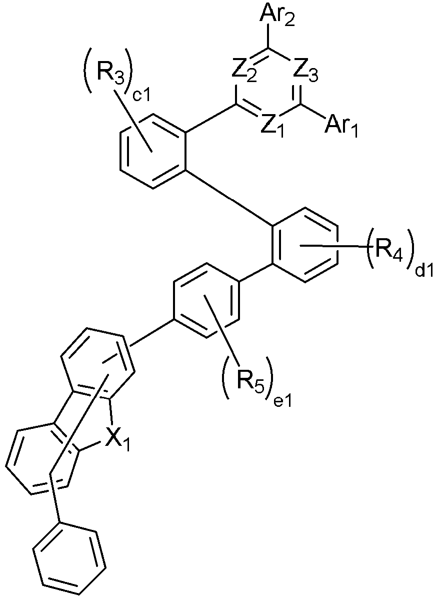 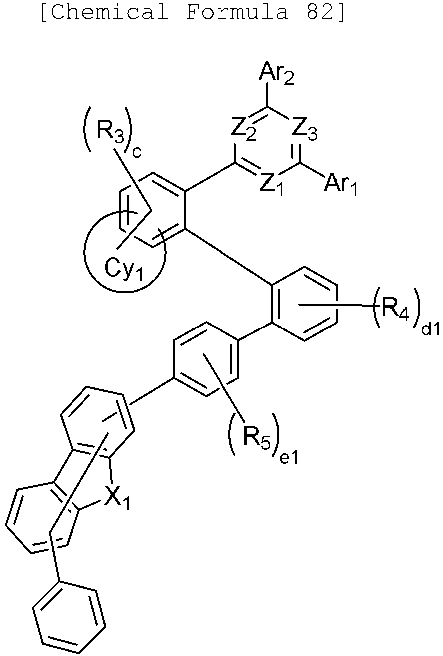 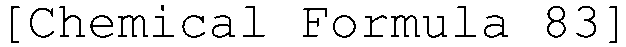 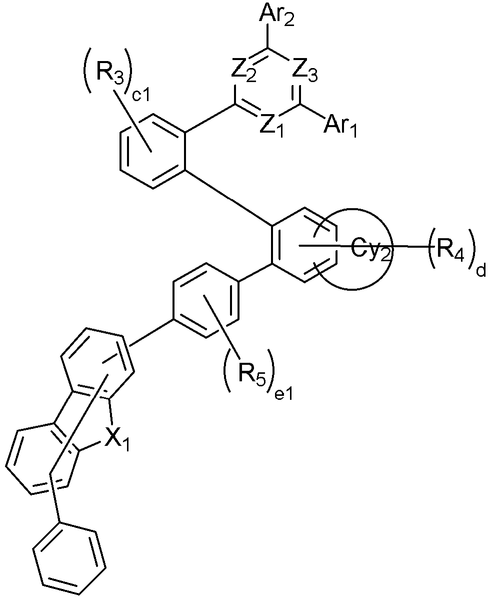 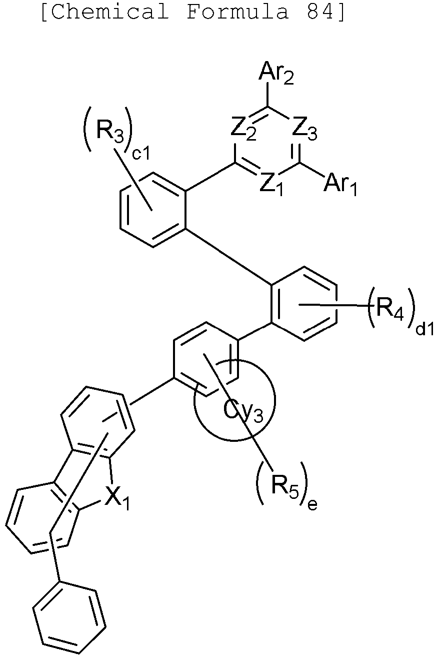 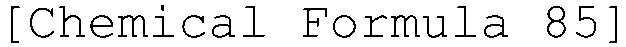 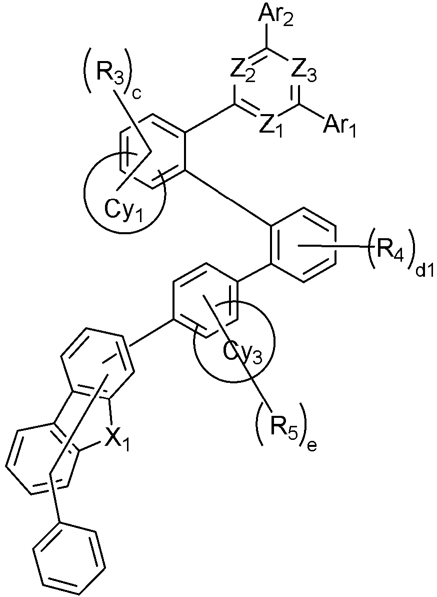 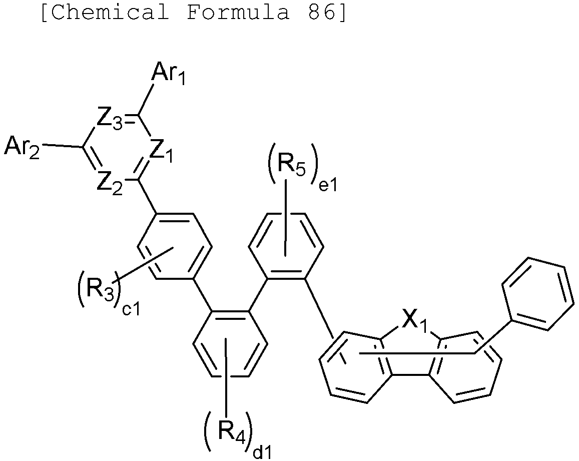 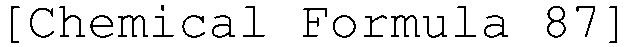 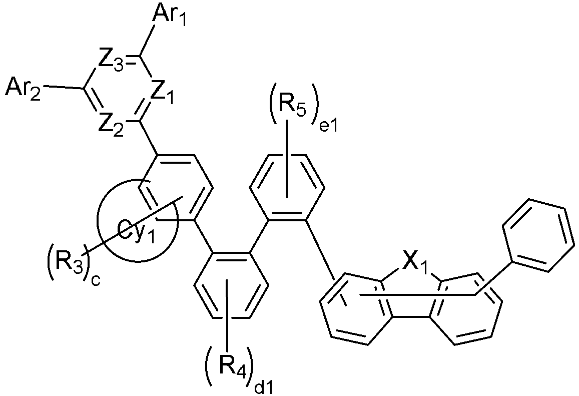 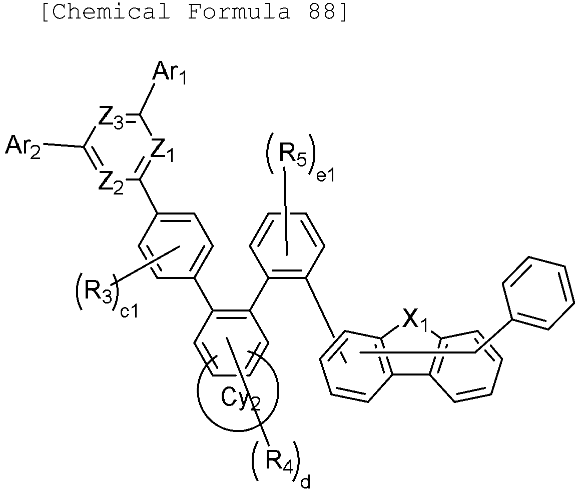 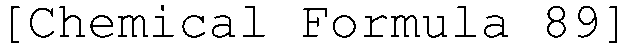 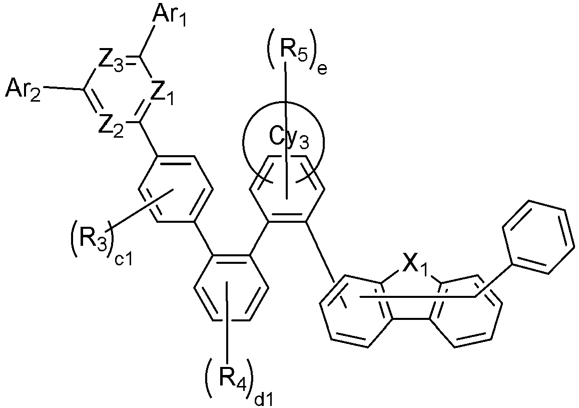 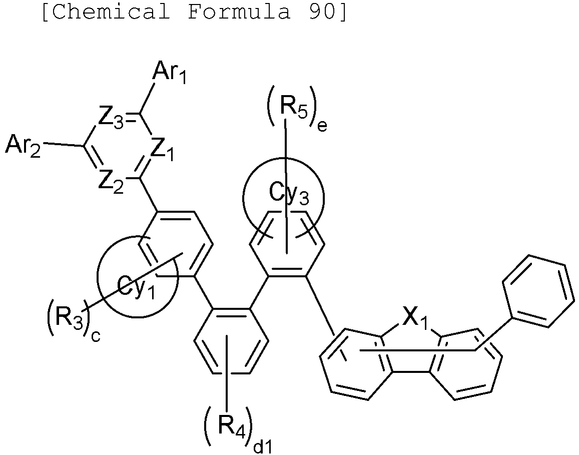 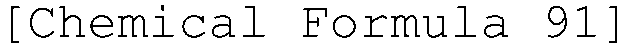 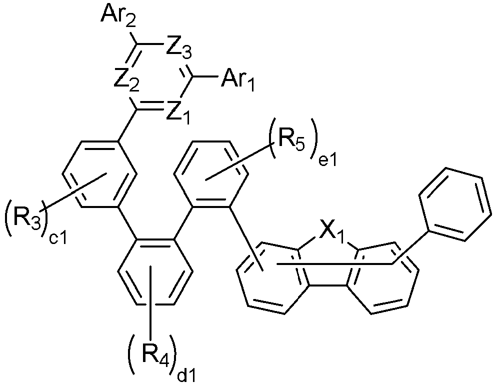 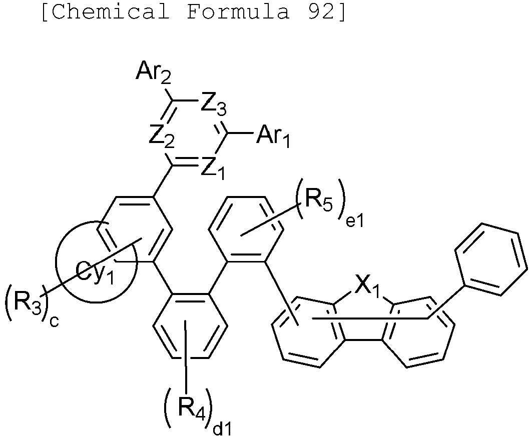 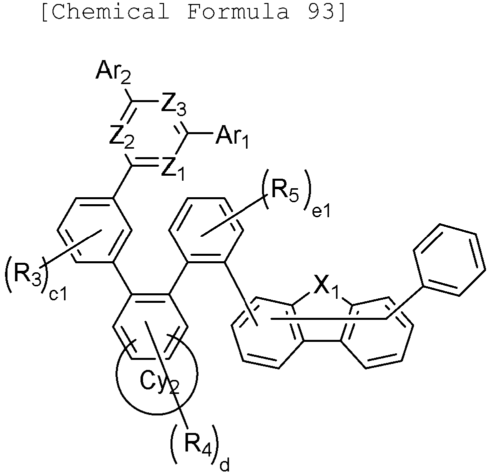 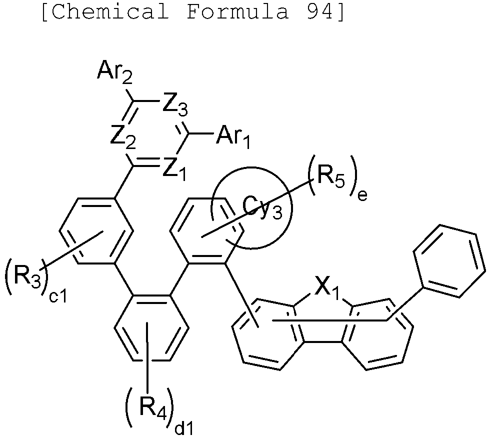 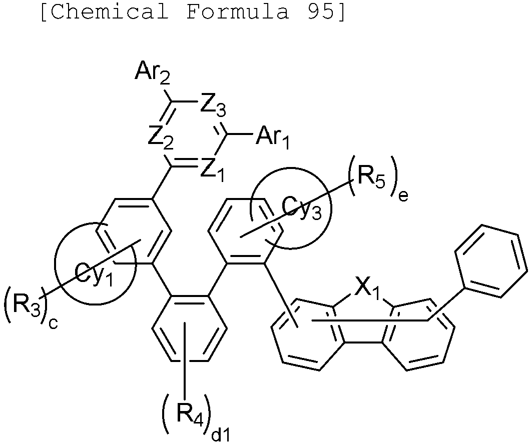 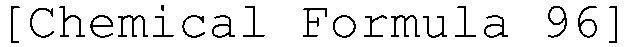 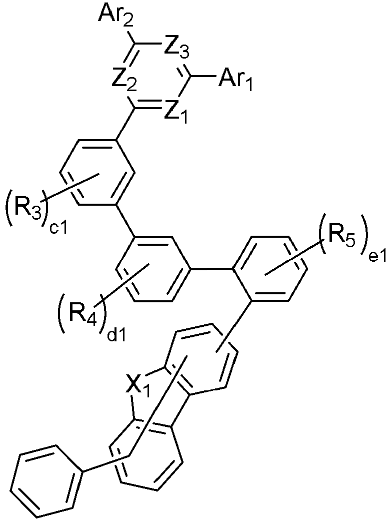 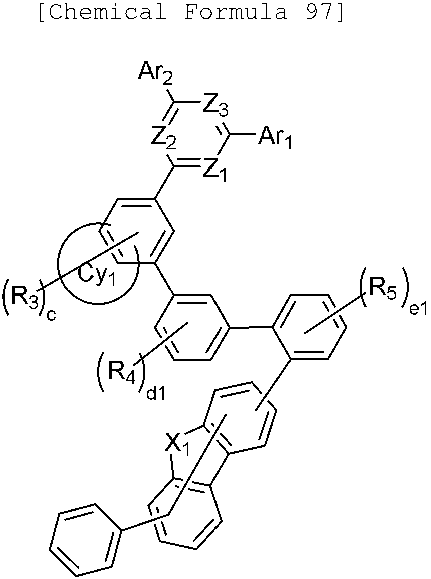 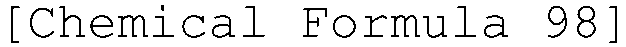 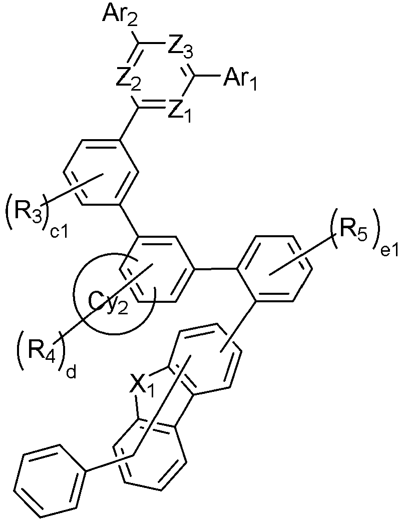 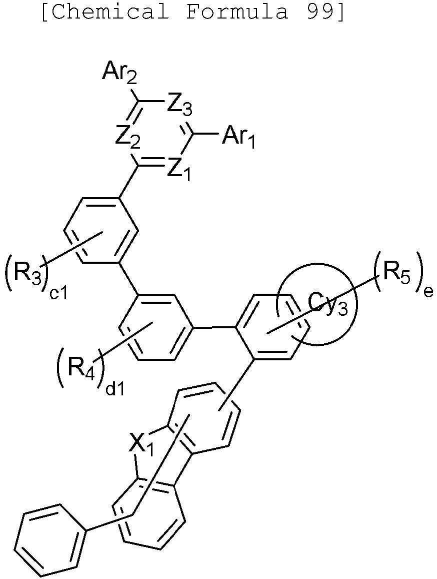 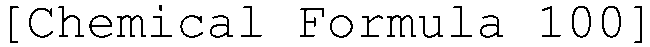 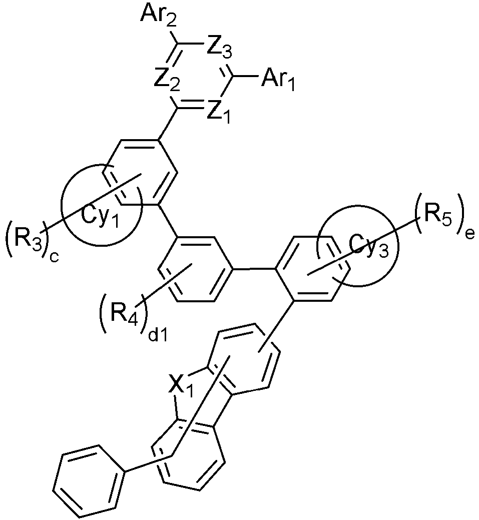 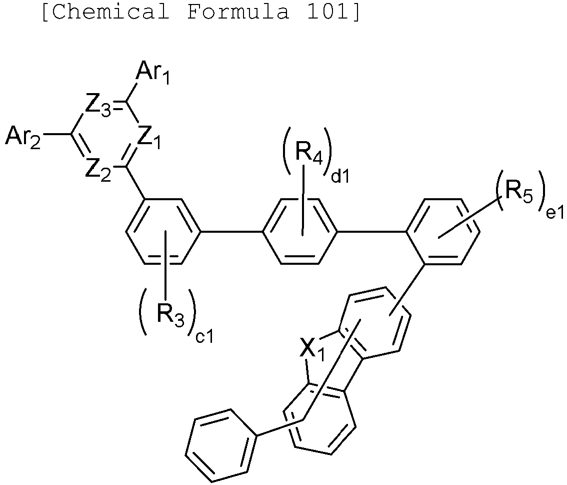 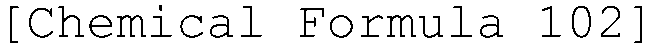 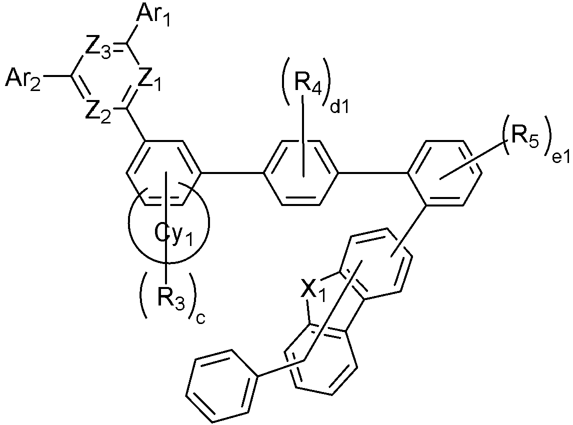 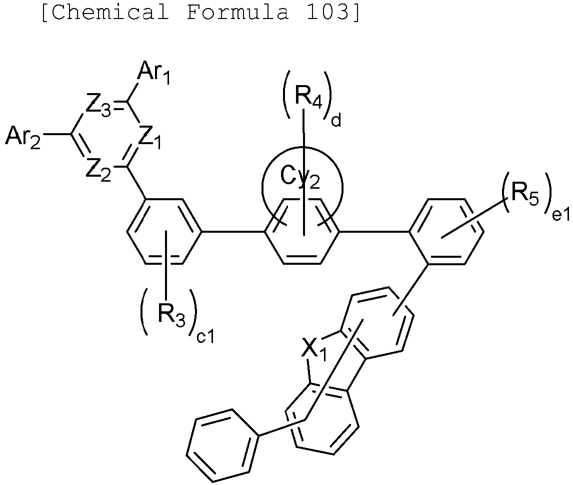 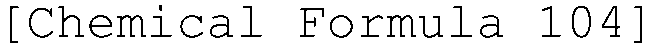 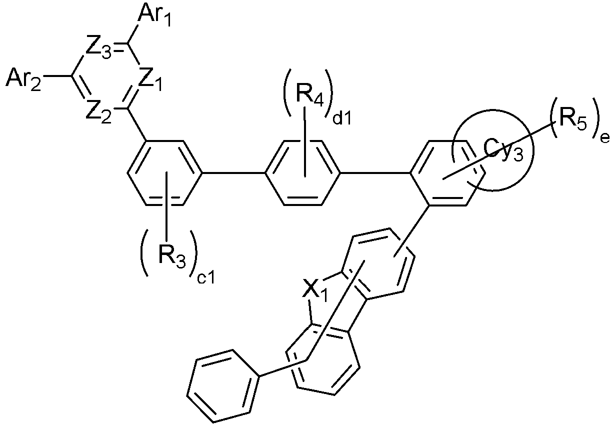 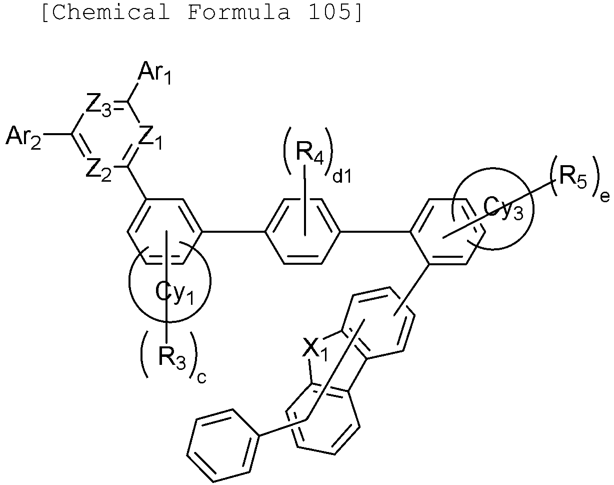 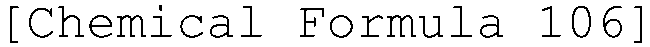 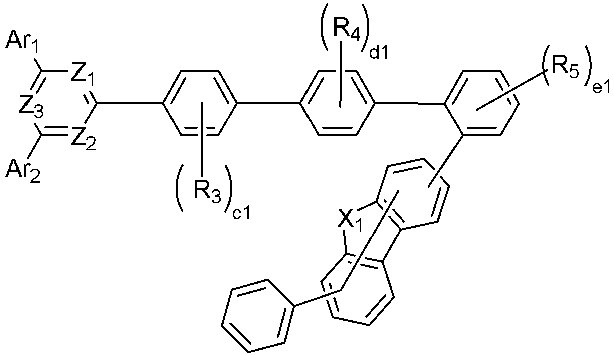 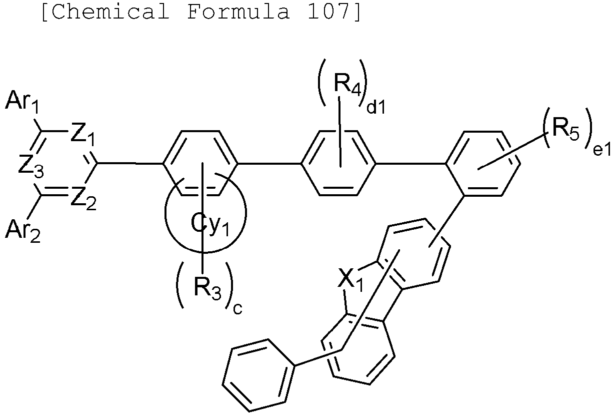 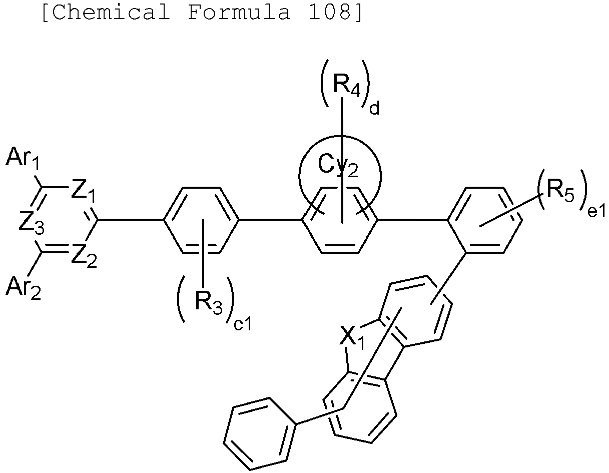 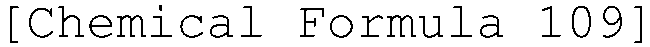 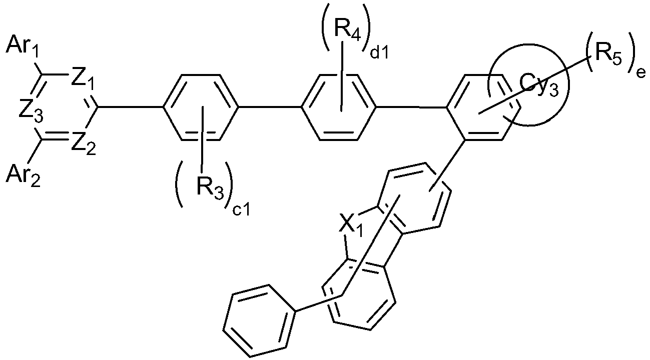 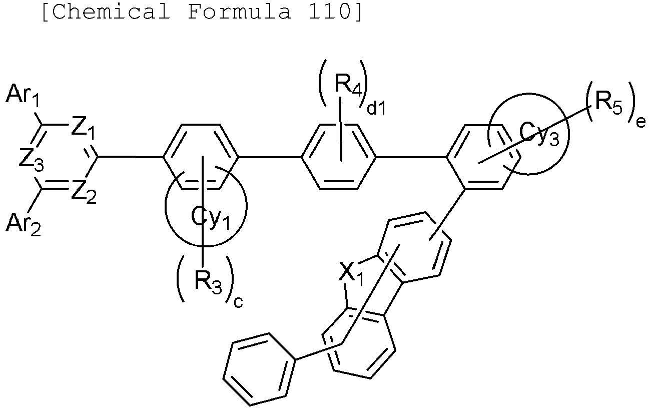 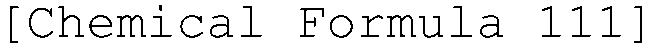 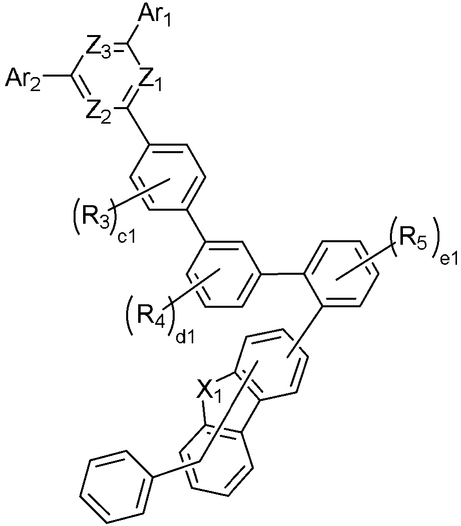 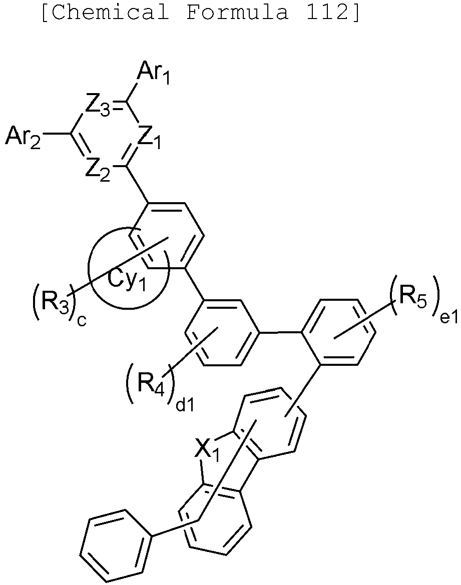 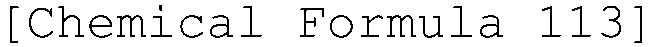 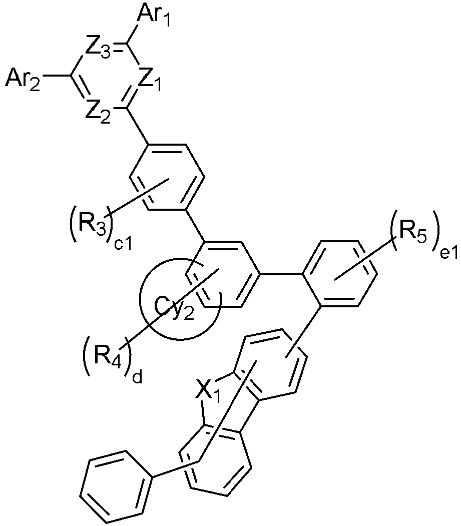 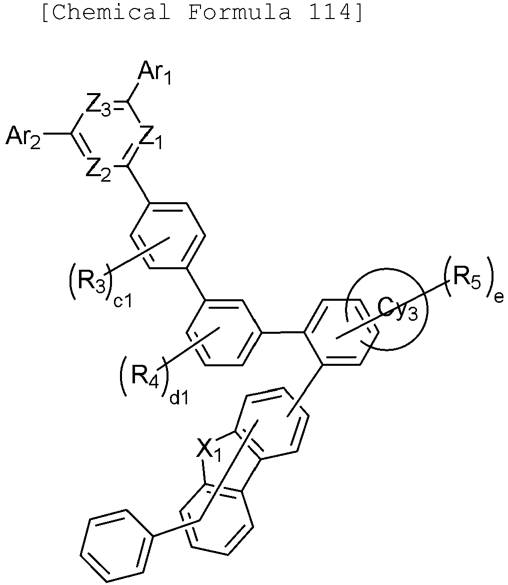 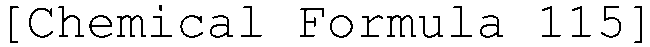 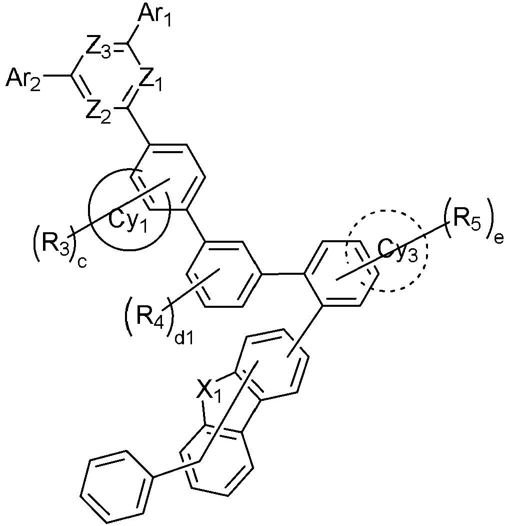
wherein in Chemical Formulas 2 to 115,
Z₁ to Z₃, X₁, Ar₁, Ar₂, Cy₁ to Cy₃, c, d, e, R₃ to R₃ are each as defined in Chemical Formula 1, and
c1, d1 and e1 may each independently be an integer of 0 to 4, specifically 0 to 2, and more specifically 0 or 1.

The compound represented by Chemical Formula 1 of the present invention described above may be further specified as compounds 1 to 252 below. However, the compound represented by Chemical Formula 1 of the present invention is not limited to those exemplified below.

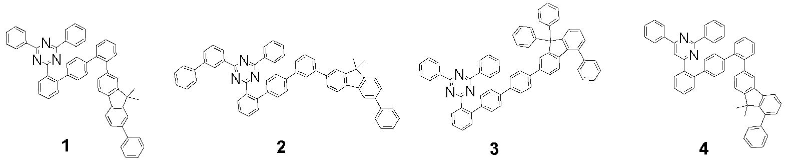

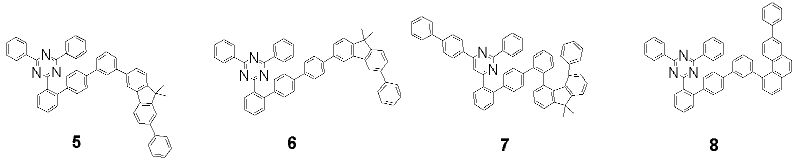

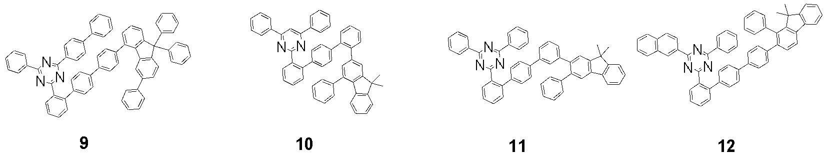

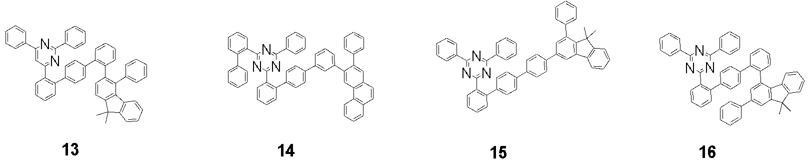

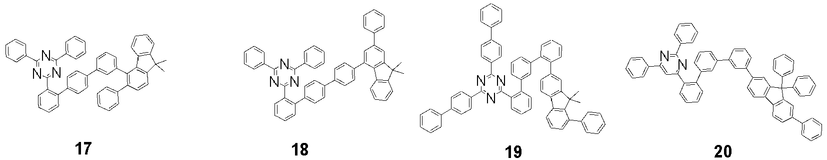

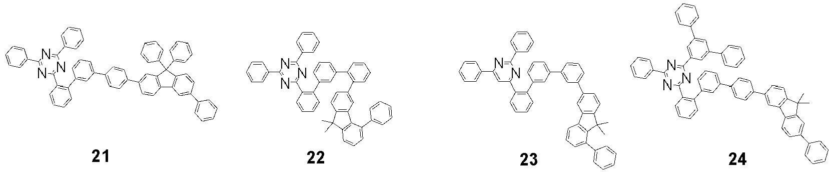

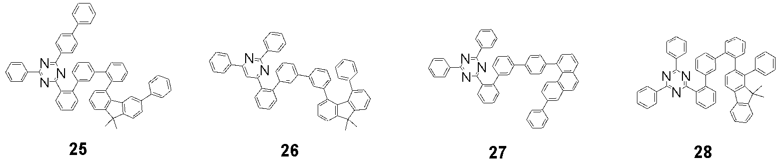

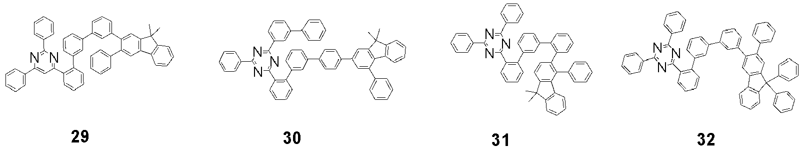

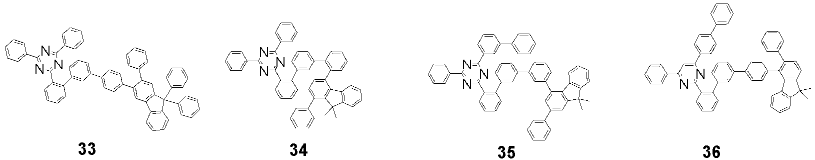

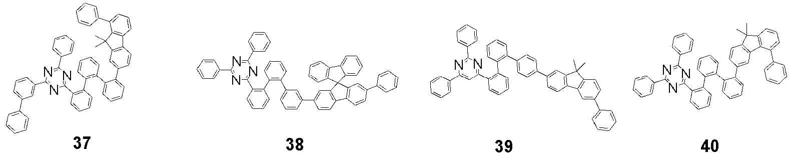

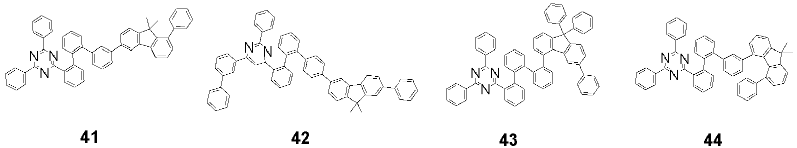

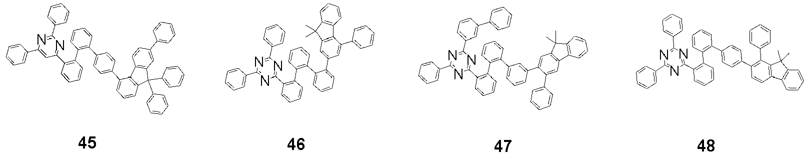

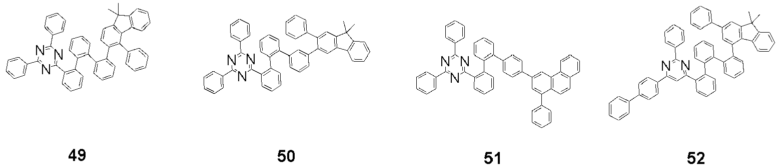

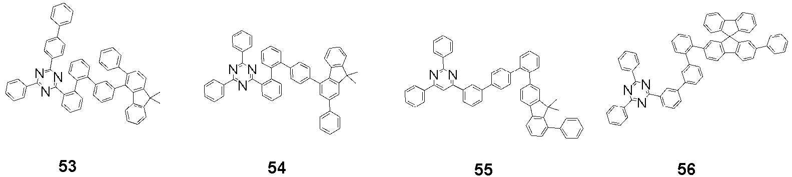

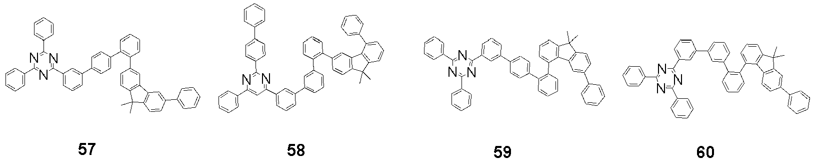

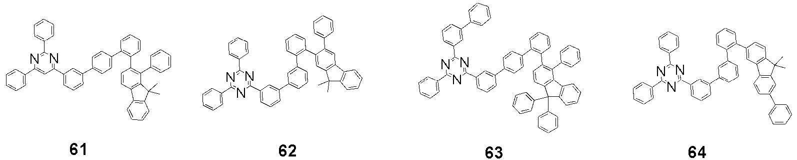

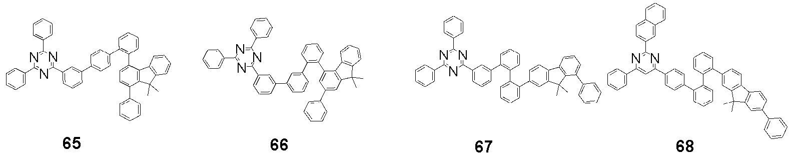

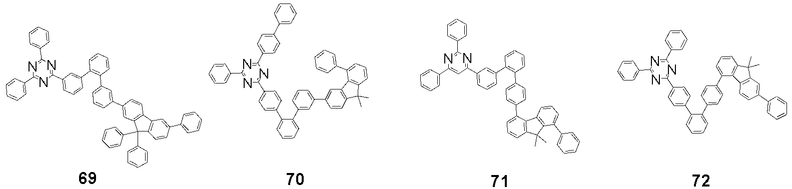

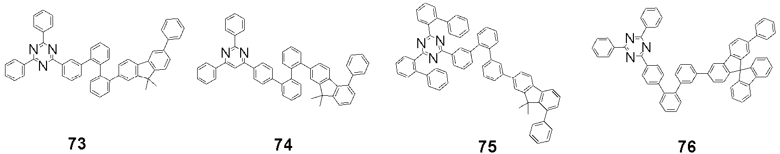

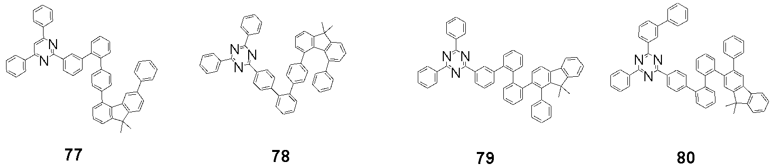

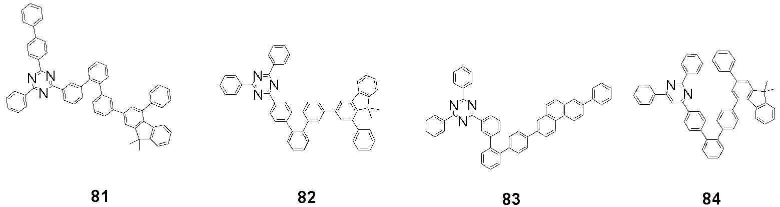

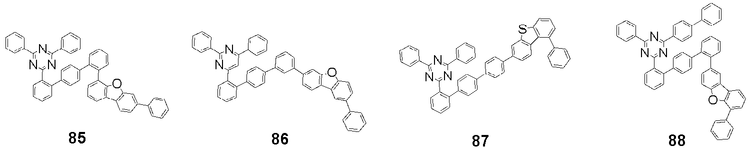

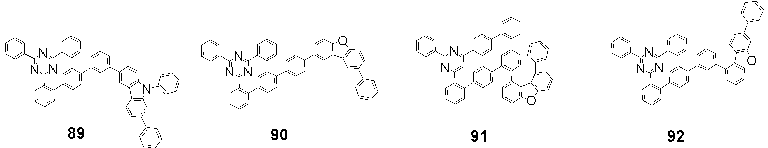

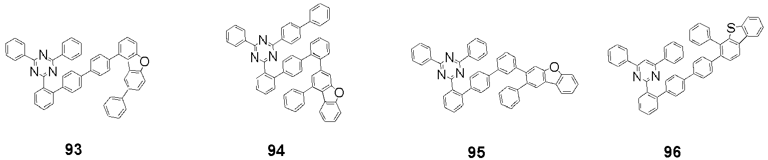

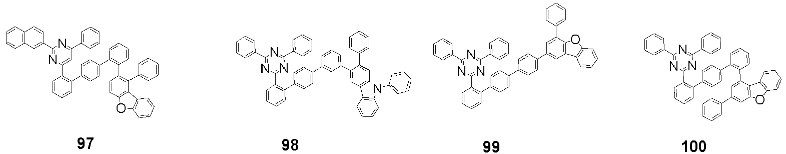

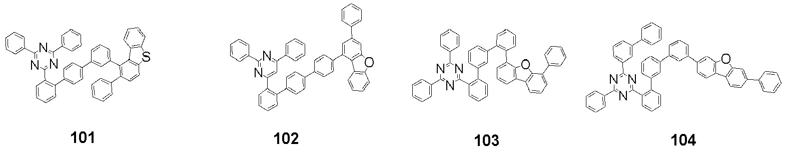

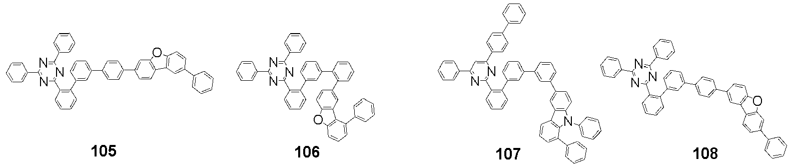

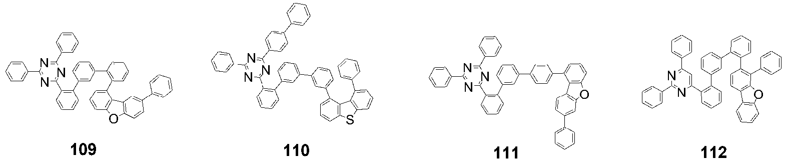

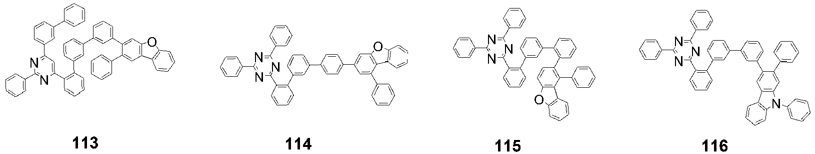

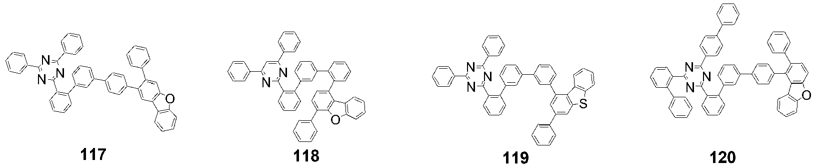

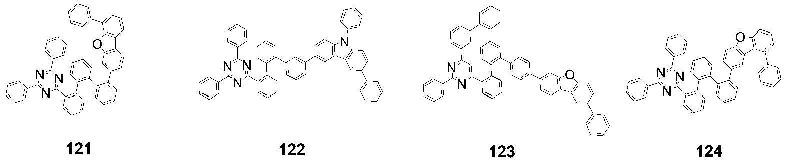

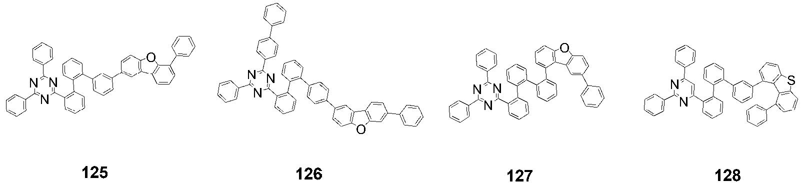

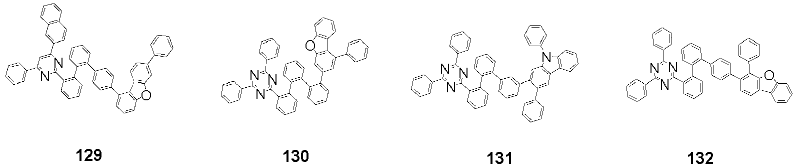

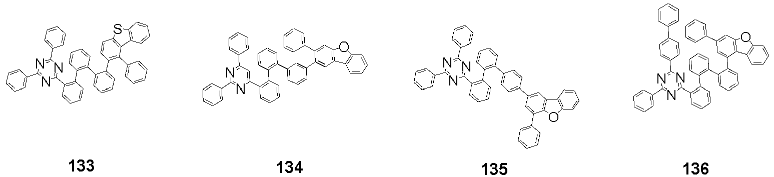

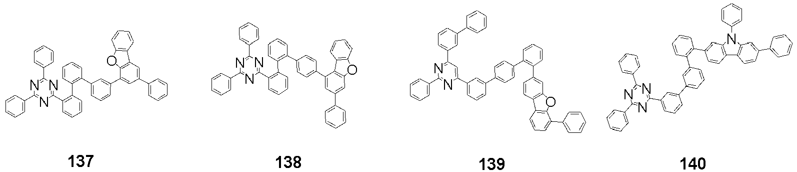

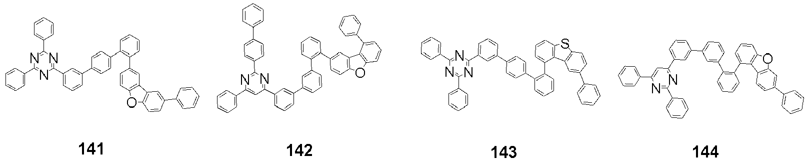

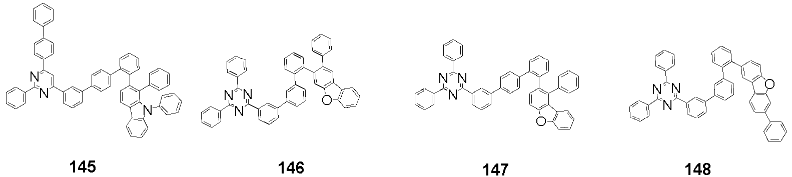

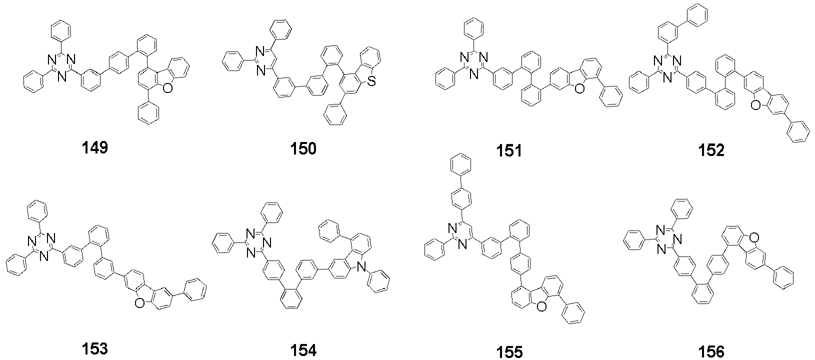

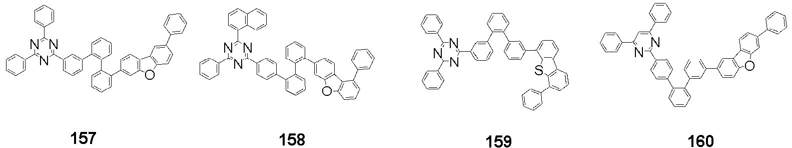

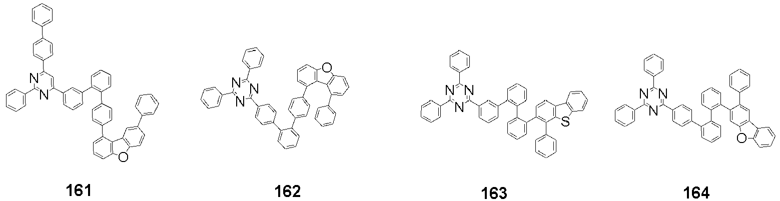

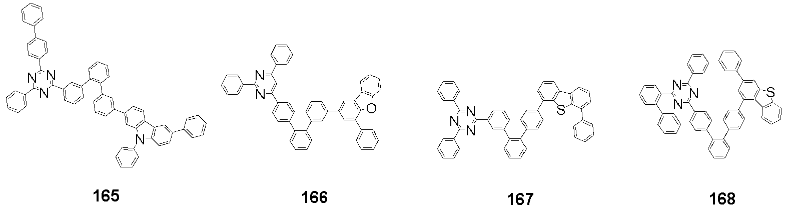

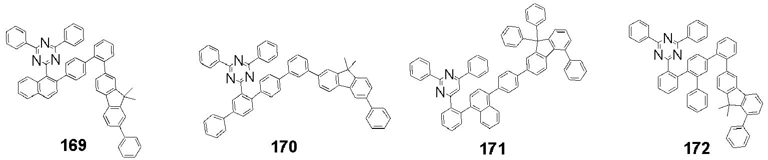

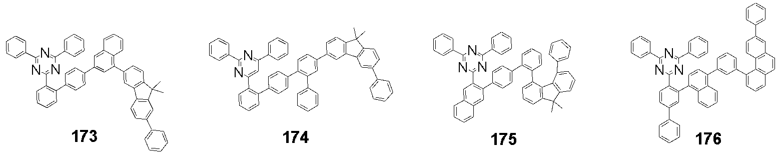

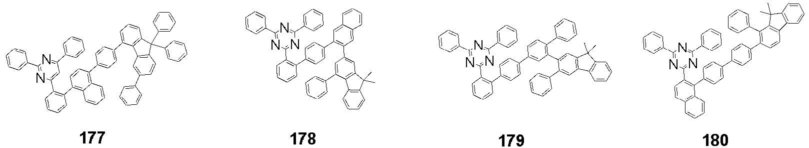

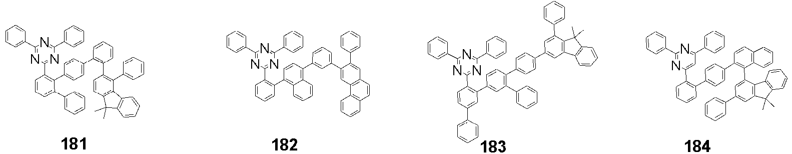

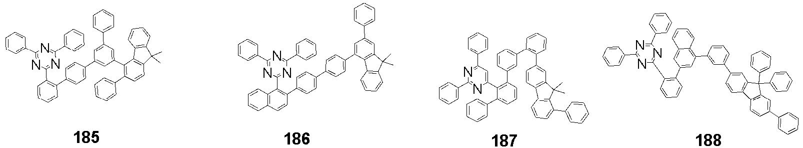

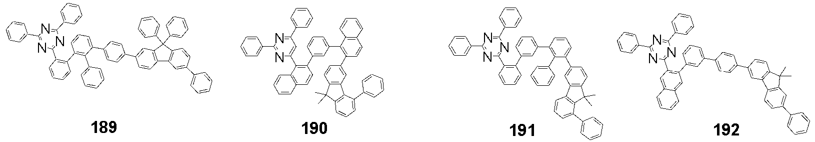

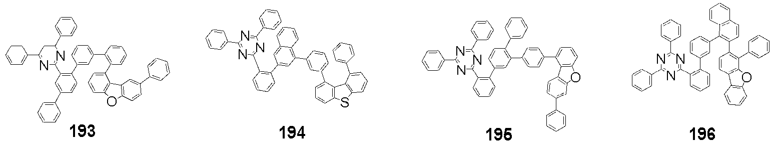

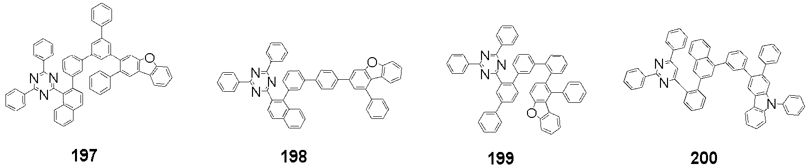

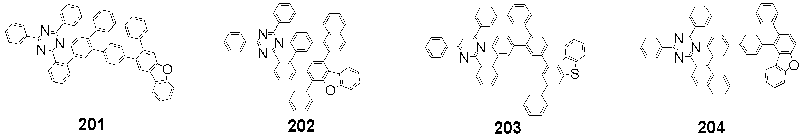

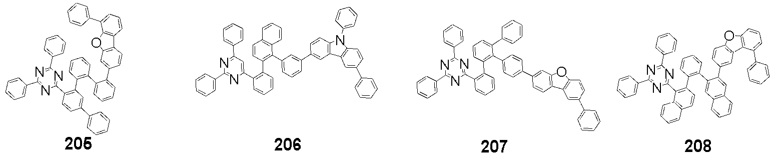

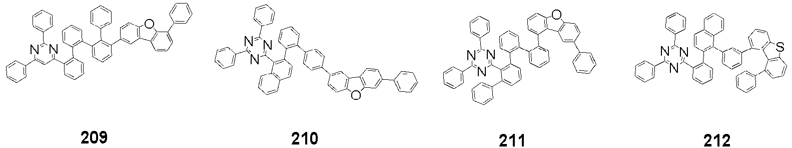

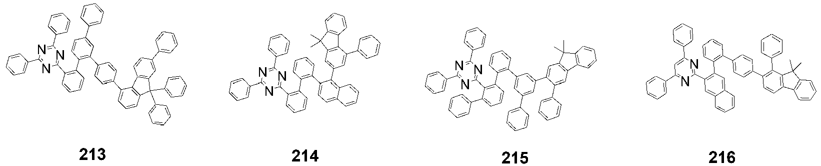

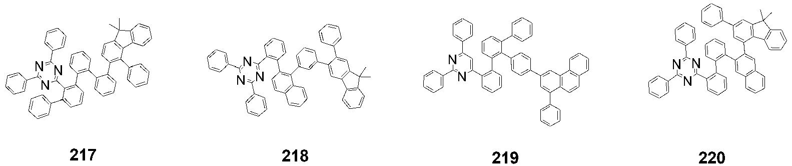

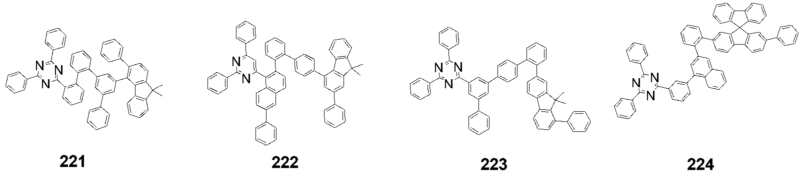

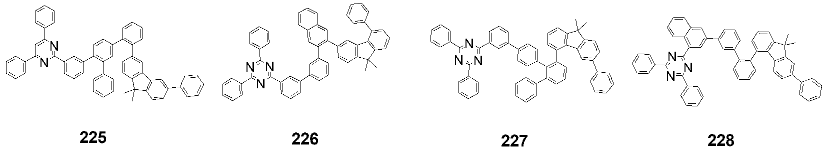

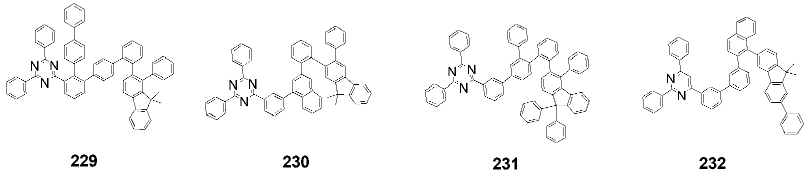

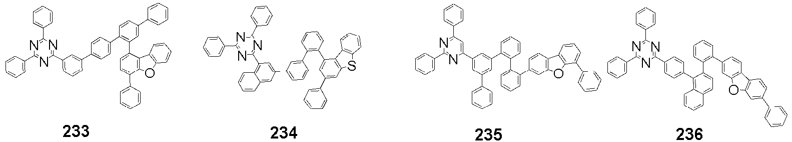

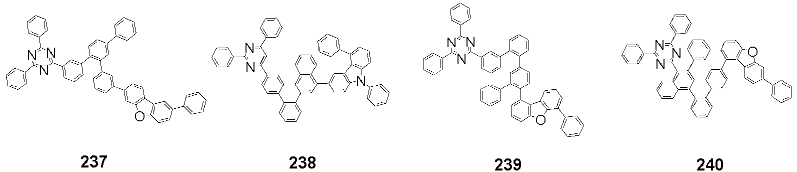

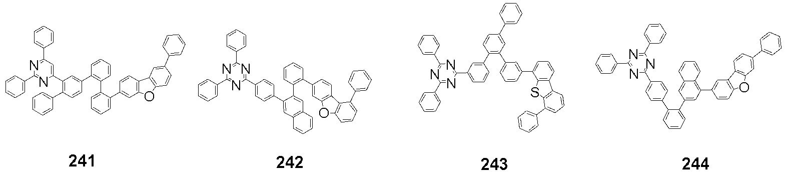

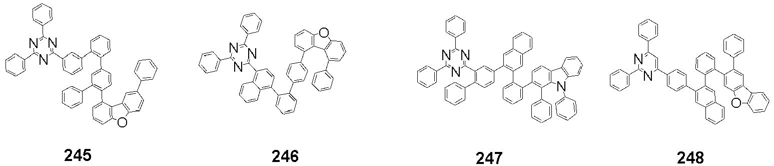

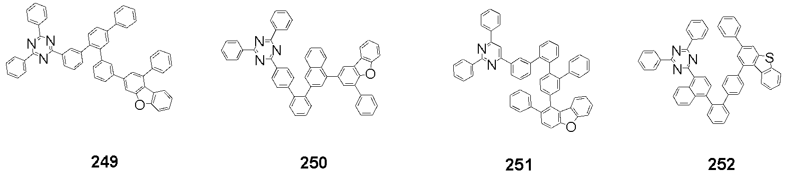

As used herein, "alkyl" refers to a monovalent substituent derived from a linear or branched chain saturated hydrocarbon having 1 to 40 carbon atoms. Examples of such alkyl may include, but not limited to, methyl, ethyl, propyl, isobutyl, sec-butyl, pentyl, iso-amyl, hexyl or the like.

As used herein, "alkenyl" refers to a monovalent substituent derived from a C₂ to C₄₀ linear or branched chain unsaturated hydrocarbon, having at least one carbon-carbon double bond. Examples of such alkenyl may include, but not limited to, vinyl, allyl, isopropenyl, 2-butenyl or the like.

As used herein, "alkynyl" refers to a monovalent substituent derived from a C₂ to C₄₀ linear or branched chain unsaturated hydrocarbon, having at least one carbon-carbon triple bond. Examples of such alkynyl may include, but not limited to, ethynyl, 2-propynyl or the like.

As used herein, "cycloalkyl" refers to a monovalent substituent derived from a C₃ to C₄₀ monocyclic or polycyclic non-aromatic hydrocarbon. Examples of such cycloalkyl may include, but not limited to, cyclopropyl, cyclopentyl, cyclohexyl, norbornyl, adamantine or the like.

As used herein, "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, O, S or Se. Examples of such heterocycloalkyl may include, but not limited to, morpholine, piperazine or the like. Here, the term "the number of nuclear atoms" refers to the number of atoms constituting a ring, i.e., the number of atoms forming the ring.

As used herein, "aryl" refers to a monovalent substituent derived from a C₆ to C₄₀ aromatic hydrocarbon having a structure with a single ring or two or more rings combined with each other. In addition, a form in which two or more rings are pendant (e.g., simply attached) to or fused with each other may also be included. Examples of such aryl may include, but not limited to, phenyl, naphthyl, phenanthryl, anthryl or the like.

As used herein, "heteroaryl" refers to a monovalent substituent derived from a monoheterocyclic or polyheterocyclic aromatic hydrocarbon having 5 to 40 nuclear atoms. In such an embodiment, one or more carbons in the ring, preferably one to three carbons, are substituted with a heteroatom such as N, O, S or Se. In addition, a form in which two or more rings are pendant to or fused with each other may be included and a form fused with an aryl group may be included. Examples of such heteroaryl may include, but not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole, and carbazolyl; 2-furanyl; N-imidazolyl; 2-isoxazolyl; 2-pyridinyl; 2-pyrimidinyl or the like. Here, the term "the number of nuclear atoms" refers to the number of atoms constituting a ring, i.e., the number of atoms forming the ring.

As used herein, "alkyloxy" refers to a monovalent substituent represented by R'O-, where R' refers to a C₁ to C₄₀ alkyl. Such alkyloxy may include a linear, branched or cyclic structure. Examples of such alkyloxy may include, but not limited to, methoxy, ethoxy, n-propoxy, 1-propoxy, t-butoxy, n-butoxy, pentoxy or the like.

As used herein, "aryloxy" is a monovalent substituent represented by RO-, where R refers to a C₅ to C₄₀ aryl. Examples of such aryloxy may include, but not limited to, phenyloxy, naphthyloxy, diphenyloxy or the like.

As used herein, "alkylsilyl" refers to silyl substituted with a C1 to C40 alkyl, and includes mono-, di-, and tri-alkylsilyl.

As used herein, "arylsilyl" refers to silyl substituted with a C₅ to C₄₀ aryl, and includes not only mono-arylsilyl but also polyarylsilyl such as di- and triarylsilyl.

As used herein, "alkylboron group" refers to a boron group substituted with a C₁ to C₄₀ alkyl, and "arylboron group" refers to a boron group substituted with a C₆ to C₆₀ aryl.

As used herein, "alkylphosphinyl group" refers to a phosphine group substituted with a C₁ to C₄₀ alkyl, and includes mono- and di-alkylphosphinyl groups.

As used herein, "arylphosphinyl group" refers to a phosphine group substituted with a C₆ to C₆₀ aryl, and includes mono- and di-arylphosphinyl groups.

As used herein, "arylphosphine oxide group" refers to a phosphine oxide group substituted with a C₆ to C₆₀ aryl, and includes mono- and di-arylphosphine oxide groups.

As used herein, "arylamine" refers to an amine substituted with a C₆ to C₆₀ aryl, and includes mono- and di-arylamines.

As used herein, "fused ring" refers to a fused aliphatic ring having 3 to 40 carbon atoms, a fused aromatic ring having 6 to 60 carbon atoms, a fused heteroaliphatic ring having 3 to 60 ring atoms, a fused heteroaromatic ring having 5 to 60 ring atoms, a C₃ to C₆₀ spiro ring, or a combination thereof. Here, the term "the number of nuclear atoms" refers to the number of atoms constituting a ring, i.e., the number of atoms forming the ring.

### <Organic electroluminescent device>

Another aspect of the present invention is directed to an organic electroluminescent device ("organic EL device" or "organic EL element") including the compound represented by Chemical Formula 1.

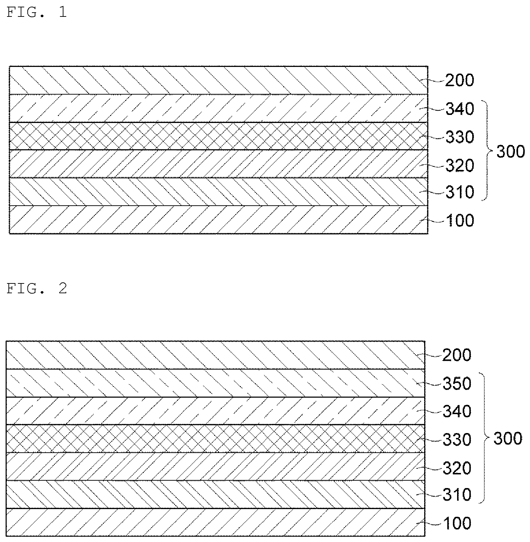

More specifically, the organic EL device according to the present invention, as shown in FIGS. 1 to 3, includes an anode 100, a cathode 200, and one or more organic layers 300 disposed between the anode and the cathode, and at least one of the one or more organic layers includes the compound represented by Chemical Formula 1. In this case, the compound may be used alone or in combination of two or more kinds thereof.

The one or more organic layers 300 may include at least one selected from a hole injection layer 310, a hole transport layer 320, a light emitting layer 330, an electron transport layer 340, and an electron injection layer 350, and may optionally further include at least one selected from an electron transport auxiliary layer 360 and a hole transport auxiliary layer (not shown). At least one of the organic layers 300 includes the compound represented by Chemical Formula 1. Specifically, the organic layer including the compound represented by Chemical Formula 1 may be the electron transport layer 340 or the electron transport auxiliary layer 360.

In one embodiment, the one or more organic layers include a hole injection layer, a hole transport layer, a light emitting layer, an electron transport layer, and an electron injection layer, and may optionally further include at least one selected from an electron transport auxiliary layer and a hole transport auxiliary layer. The electron transport layer includes the compound represented by Chemical Formula 1. In this case, the compound represented by Chemical Formula 1 is included in the organic electroluminescent device as an electron transport layer material. In the organic electroluminescent device, electrons may be easily injected from the cathode or the electron injection layer into the electron transport layer due to the compound represented by Chemical Formula 1, and may also rapidly move from the electron transport layer to the light emitting layer, thereby enhancing the recombination efficiency of holes and electrons in the light emitting layer. Accordingly, the organic electroluminescent device of the present invention may exhibit excellent light emission efficiency, power efficiency, and luminance. In addition, the compound represented by Chemical Formula 1 has excellent thermal stability and electrochemical stability, thereby improving performance of the organic electroluminescent device.

The compound represented by Chemical Formula 1 may be used alone or may be used in combination with an electron transport layer material known in the art.

In the present invention, the electron transport layer material that may be used in combination with the compound represented by Chemical Formula 1 may include an electron transport material or an n-type dopant commonly known in the art. Non-limiting examples of the electron transport material include oxazole-based compounds, isoxazole-based compounds, triazole-based compounds, isothiazole-based compounds, oxadiazole-based compounds, thiadiazole-based compounds, perylene-based compounds, aluminum complexes (e.g., Alq₃ (tris(8-quinolinolato)aluminum)), and gallium complexes (e.g., Gaq'2OPiv, Gaq'2OAc, and 2(Gaq'2)). These may be used alone or two or more thereof may be used in combination. In the present invention, examples of the n-type dopant that may be used include metals (e.g., alkali metals or alkaline earth metals) or complexes thereof, and specifically may include LiQ (lithium quinolate).

In the present invention, when the compound represented by Chemical Formula 1 is used in combination with the electron transport layer material, the mixing ratio thereof is not particularly limited and may be appropriately adjusted within a range known in the art.

In another embodiment, the one or more organic layers include a hole injection layer, a hole transport layer, a light emitting layer, an electron transport auxiliary layer, an electron transport layer, and an electron injection layer, and the electron transport auxiliary layer includes the compound represented by Chemical Formula 1. In this case, the compound represented by Chemical Formula 1 is included in the organic electroluminescent device as a material of the electron transport auxiliary layer. When the compound represented by Chemical Formula 1 is included as a material of the electron transport auxiliary layer, the efficiency of the organic electroluminescent device may be improved due to a triplet-triplet fusion (TTF) effect. In addition, the compound represented by Chemical Formula 1 may prevent diffusion of excitons or holes generated in the light emitting layer to the electron transport layer adjacent to the light emitting layer. Therefore, the number of excitons contributing to light emission in the light emitting layer may be increased, thereby improving the light emission efficiency of the device, and the durability and stability of the device may be enhanced, resulting in an efficient increase in the lifespan of the device.

The compound represented by Chemical Formula 1 may be used alone or may be used in combination with an electron transport auxiliary layer material known in the art.

In the present invention, the electron transport auxiliary layer material that may be used in combination with the compound represented by Chemical Formula 1 may include an electron transport material commonly known in the art. Non-limiting examples thereof include oxadiazole derivatives, triazole derivatives, phenanthroline derivatives (e.g., BCP), and nitrogen-containing heterocyclic derivatives, but are not limited thereto.

The structure of the organic electroluminescent device of the present invention described above is not particularly limited. For example, an anode 100, one or more organic layers 300, and a cathode 200 may be sequentially stacked on a substrate (see FIGS. 1 to 3). In addition, although not illustrated, the device may have a structure in which an insulating layer or an adhesive layer is inserted at an interface between an electrode and the organic layers.

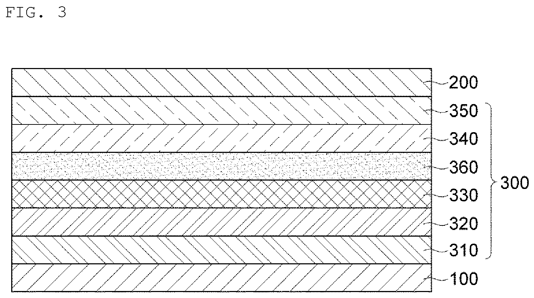

In one embodiment, as shown in FIG. 1, the organic electroluminescent device may have a structure in which an anode 100, a hole injection layer 310, a hole transport layer 320, a light emitting layer 330, an electron transport layer 340, and a cathode 200 are sequentially stacked on a substrate. Optionally, as shown in FIG. 2, an electron injection layer 350 may be positioned between the electron transport layer 340 and the cathode 200. In addition, an electron transport auxiliary layer 360 may be positioned between the light emitting layer 330 and the electron transport layer 340 (see FIG. 3).

The organic EL device of the present invention may be manufactured using materials and methods known in the art to form organic layers and electrodes, except that at least one of the organic layers 300 (e.g., the electron transport layer 340) include the compound represented by Chemical Formula 1.

The organic layer may be formed by a vacuum deposition method or a solution coating method. Examples of the solution coating method may include, but not limited to, spin coating, dip coating, doctor blading, inkjet printing, thermal transfer or the like.

The substrate used in preparation of the organic EL device of the present invention is not particularly limited, and non-limiting examples thereof may include silicon wafers, quartz, glass plates, metal plates, plastic films, sheets or the like.

In addition, an anode material may use any anode material known in the art without limitation. Examples of the anode material may include, but not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly(3-methylthiophene), poly [3,4-(ethylene-1,2-dioxy) thiophene] (PEDT), polypyrrole or polyaniline; carbon black or the like.

In addition, a cathode material may use any cathode material known in the art without limitation. Examples of the cathode material may include, but not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, materials for the hole injection layer, the hole transport layer, the light emitting layer, the electron injection layer, and the hole transport auxiliary layer are not particularly limited, and conventional materials known in the art may be used without limitation.

Hereinafter, the present invention will be described in detail through examples. However, the following examples are only to illustrate the present invention, and the present invention is not limited by the following examples.

### [Preparation Example 1] Synthesis of TC-1

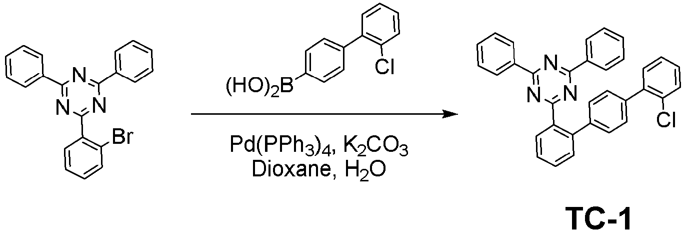

2-(2-bromophenyl)-4,6-diphenyl-1,3,5-triazine (12.0g, 30.9mmol), (2'-chloro-[1,1'-biphenyl]-4-yl)boronic acid (7.2g, 30.9mmol), Pd(PPh₃)₄ (1.1g, 0.9mmol), and K₂CO₃ (8.5g, 61.9mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-1 (9.2 g, 60% yield) .
1H-NMR: δ 8.36(d, 4H), 7.96(d, 2H), 7.71(d, 1H), 7.61~7.60(m, 3H), 7.50(t, 6H), 7.38(t, 2H), 7.25(d, 4H)
Mass: [(M+H)+] : 497

### [Preparation Example 2] Synthesis of TC-2

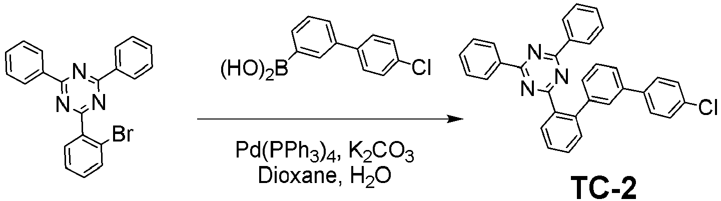

2-(2-bromophenyl)-4,6-diphenyl-1,3,5-triazine (12.0g, 30.9mmol), (4'-chloro-[1,1'-biphenyl]-3-yl)boronic acid (7.2g, 30.9mmol), Pd(PPh₃)₄ (1.1g, 0.9mmol), and K₂CO₃ (8.5g, 61.9mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-2 (8.9g, 58% yield) .
1H-NMR: δ 8.36(d, 4H), 8.10(d, 2H), 7.96~7.94(m, 3H), 7.73(t, 1H), 7.62~7.50(m, 12H)
Mass: [(M+H)+] : 497

### [Preparation Example 3] Synthesis of TC-3

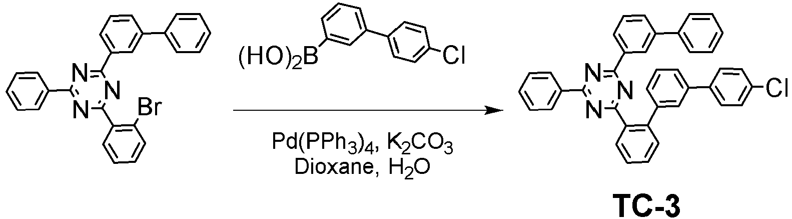

2-([1,1'-biphenyl]-3-yl)-4-(2-bromophenyl)-6-phenyl-1,3,5-triazine (12.0g, 25.9mmol), (4'-chloro-[1,1'-biphenyl]-3-yl)boronic acid (6.0g, 25.9mmol), Pd(PPh₃)₄ (0.9g, 0.8mmol), and K₂CO₃ (7.1g, 51.7mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-3 (8.7g, 59% yield).
1H-NMR: δ 8.38(d, 1H), 8.36(d, 2H), 8.10(d, 2H), 7.96~7.94(m, 4H), 7.75~7.73(m, 4H), 7.62~7.60(m, 7H), 7.50~7.41(m, 6H)
Mass: [(M+H)+] : 573

### [Preparation Example 4] Synthesis of TC-4

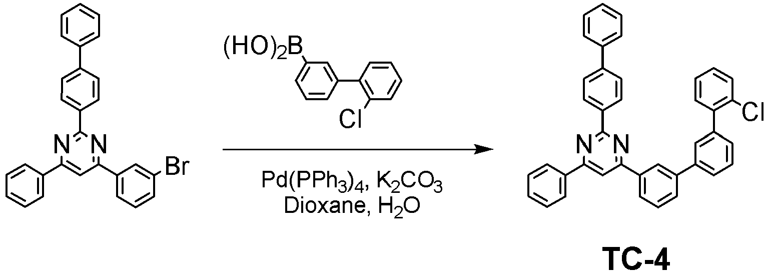

2-([1,1'-biphenyl]-4-yl)-4-(3-bromophenyl)-6-phenylpyrimidine (12.0g, 25.9mmol), (2'-chloro-[1,1'-biphenyl]-3-yl)boronic acid (6.0g, 25.9mmol), Pd(PPh₃)₄ (0.9g, 0.8mmol), and K₂CO₃ (7.1g, 51.7mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-4 (8.3g, 56% yield).
1H-NMR: δ 8.23(s, 1H), 7.96~7.94(m, 7H), 7,75~7.71(m, 5H), 7.61~7.38(m, 12H), 7.25(2, 2H)~
Mass: [(M+H)+] : 572

### [Preparation Example 5] Synthesis of TC-5

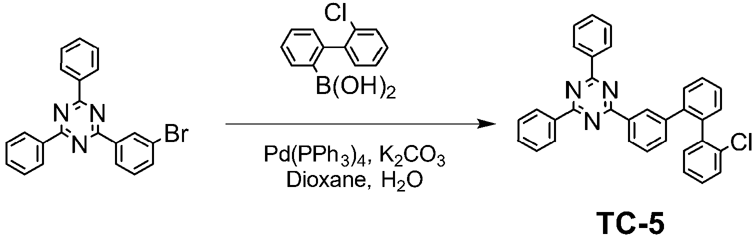

2-(3-bromophenyl)-4,6-diphenyl-1,3,5-triazine (12.0g, 30.9mmol), (2'-chloro-[1,1'-biphenyl]-2-yl)boronic acid (7.2g, 30.9mmol), Pd(PPh₃)₄ (1.1g, 0.9mmol), and K₂CO₃ (8.5g, 61.9mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-5 (8.9g, 58% yield) .
1H-NMR: δ 8.40(d, 1H), 8.36(d, 4H), 7.96~7.94(m, 3H), 7.73(t, 1H), 7.71(d, 1H), 7.61~7.60(m, 4H), 7.50(m, 6H), 7.38(t, 2H)
Mass: [(M+H)+] : 497

### [Preparation Example 6] Synthesis of TC-6

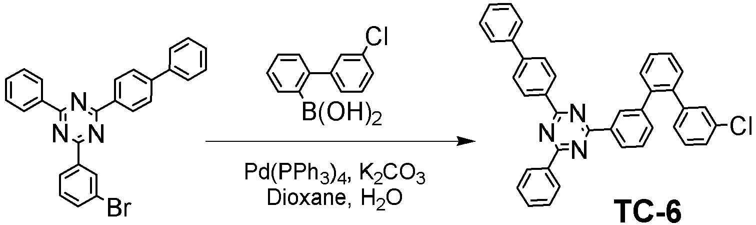

2-([1,1'-biphenyl]-4-yl)-4-(3-bromophenyl)-6-phenyl-1,3,5-triazine (12.0g, 25.9mmol), (3'-chloro-[1,1'-biphenyl]-2-yl)boronic acid (6.0g, 25.9mmol), Pd(PPh₃)₄ (0.9g, 0.8mmol), and K₂CO₃ (7.1g, 51.7mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-6 (8.6g, 58% yield).
1H-NMR: δ 8.38(d, 1H), 8.36(d, 2H), 7.96~7.94(m, 6H), 7.75~7.73(m, 3H), 7.60~7.61(m, 3H), 7.50~7.41(m, 9H), 7.25(d, 2H)
Mass: [(M+H)+] : 573

### [Preparation Example 7] Synthesis of TC-7

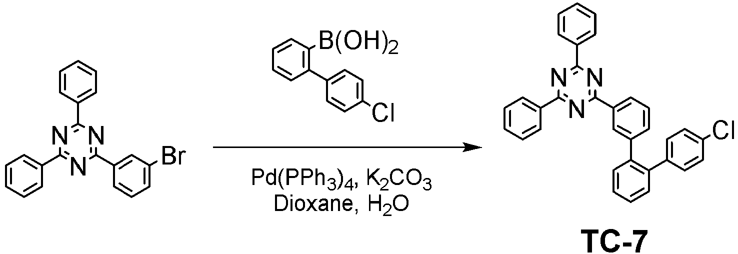

2-(3-bromophenyl)-4,6-diphenyl-1,3,5-triazine (12.0g, 30.9mmol), (4'-chloro-[1,1'-biphenyl]-2-yl)boronic acid (7.2g, 30.9mmol), Pd(PPh₃)₄ (1.1g, 0.9mmol), and K₂CO₃ (8.5g, 61.9mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-7 (9.2g, 60% yield) .
1H-NMR: δ 8.38(d, 1H), 8.36(d, 4H), 7.96(d, 2H), 7.94(s, 1H), 7.78~7.73(m, 3H), 7.62~7.60(m, 5H), 7.50(m, 6H)
Mass: [(M+H)+] : 497

### [Preparation Example 8] Synthesis of TC-8

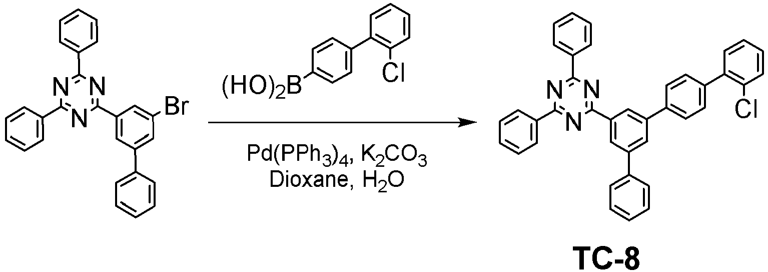

2-(5-bromo-[1,1'-biphenyl]-3-yl)-4,6-diphenyl-1,3,5-triazine (12.0g, 25.9mmol), (2'-chloro-[1,1'-biphenyl]-4-yl)boronic acid (6.0g, 25.9mmol), Pd(PPh₃)₄ (0.9g, 0.8mmol), and K₂CO₃ (7.1g, 51.7mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-8 (8.2g, 55% yield).
1H-NMR: δ 8.36(d, 4H), 8.04(s, 1H), 7.75~7.71(m, 3H), 7.61(d, 1H), 7.50~7.38(m, 11H), 7.25(d,4H)
Mass: [(M+H)+] : 573

### [Preparation Example 9] Synthesis of TC-9

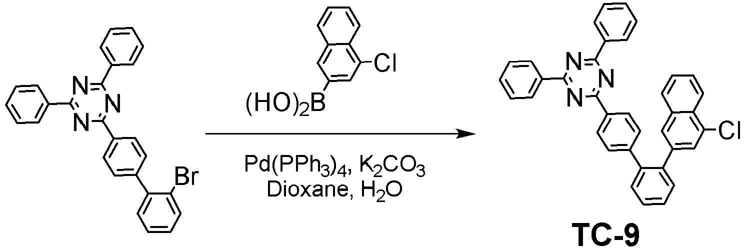

2-(2'-bromo-[1,1'-biphenyl]-4-yl)-4,6-diphenyl-1,3,5-triazine (12.0g, 25.9mmol), (4-chloronaphthalen-2-yl)boronic acid (5.3g, 25.9mmol), Pd(PPh₃)₄ (0.9g, 0.8mmol), and K₂CO₃ (7.1g, 51.7mmol) were added to 160 ml of Dioxane and 40 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain TC-9 (8.1g, 57% yield).
1H-NMR: δ 8.36(d, 4H), 8.32(d, 1H), 8.11(d, 1H), 7.96~7.92(m, 5H), 7.60~7.50(m, 11H), 7.25(d, 2H)
Mass: [(M+H)+] : 547

### [Synthesis Example 1] Synthesis of Compound 1

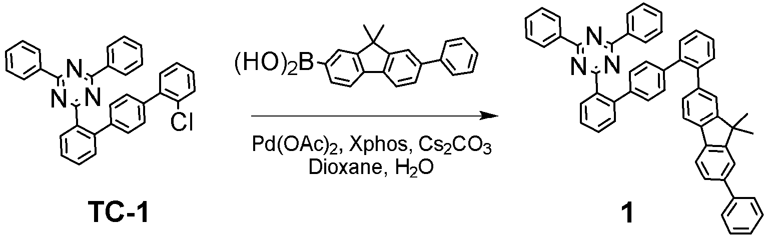

TC-1 (5.0g, 10.1mmol) obtained in Preparation Example 1, (9,9-dimethyl-7-phenyl-9H-fluoren-2-yl)boronic acid (3.2g, 10.1mmol), Pd(OAc)₂ (0.1g, 0.3mmol), XPhos (0.3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 1 (3.2g, 44% yield).

Mass: [(M+H)+] : 731

### [Synthesis Example 2] Synthesis of Compound 21

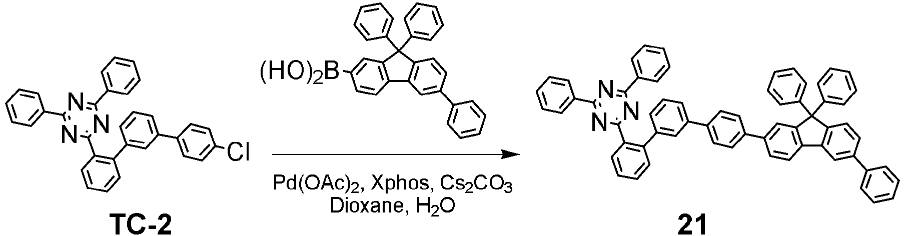

TC-2 (5.0g, 10.1mmol) obtained in Preparation Example 2, (6,9,9-triphenyl-9H-fluoren-2-yl)boronic acid (4.4g, 10.1mmol), Pd(OAc)₂ (0.1g, 0.3mmol), XPhos (0. 3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 21 (4.0g, 46% yield).

Mass: [(M+H)+] : 855

### [Synthesis Example 3] Synthesis of Compound 30

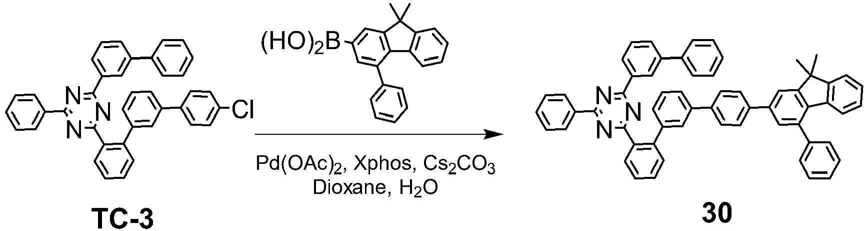

TC-3 (5.0g, 8.7mmol) obtained in Preparation Example 3, (9,9-dimethyl-4-phenyl-9H-fluoren-2-yl)boronic acid (2.7g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 30 (2.9g, 41% yield).

Mass: [(M+H)+] : 807

### [Synthesis Example 4] Synthesis of Compound 58

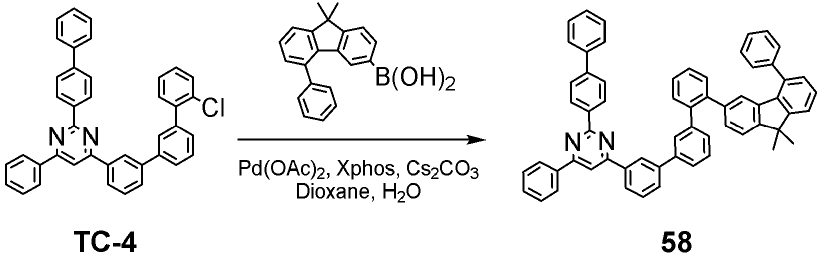

TC-4 (5.0g, 8.7mmol) obtained in Preparation Example 4, (9,9-dimethyl-5-phenyl-9H-fluoren-3-yl)boronic acid (2.7g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 58 (3.0g, 41% yield).

Mass: [(M+H)+] : 806

### [Synthesis Example 5] Synthesis of Compound 67

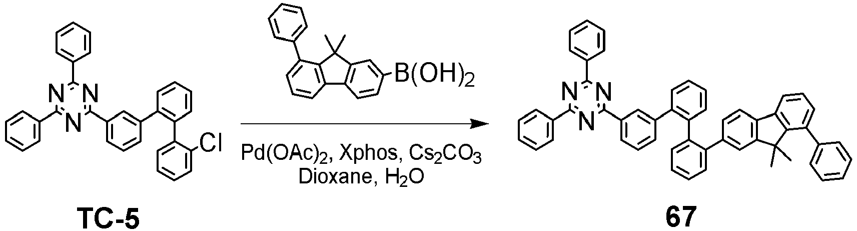

TC-5 (5.0g, 10.1mmol) obtained in Preparation Example 5, (9,9-dimethyl-8-phenyl-9H-fluoren-2-yl)boronic acid (3.2g, 10.1mmol), Pd(OAc)₂ (0.1g, 0.3mmol), XPhos (0.3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 67 (3.2g, 44% yield).

Mass: [(M+H)+] : 731

### [Synthesis Example 6] Synthesis of Compound 81

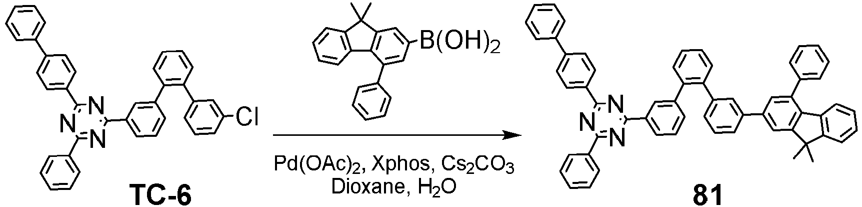

TC-6 (5.0g, 8.7mmol) obtained in Preparation Example 6, (9,9-dimethyl-4-phenyl-9H-fluoren-2-yl)boronic acid (2.7g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 81 (3.4g, 48% yield).

Mass: [(M+H)+] : 807

### [Synthesis Example 7] Synthesis of Compound 83

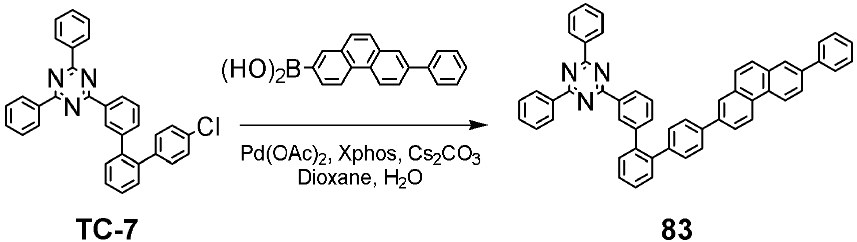

TC-7 (5.0g, 10.1mmol) obtained in Preparation Example 7, (7-phenylphenanthren-2-yl)boronic acid (3.0g, 10.1mmol), Pd(OAc)₂ (0.1g, 0.3mmol), XPhos (0.3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 83 (3.2g, 45% yield).

Mass: [(M+H)+] : 715

### [Synthesis Example 8] Synthesis of Compound 85

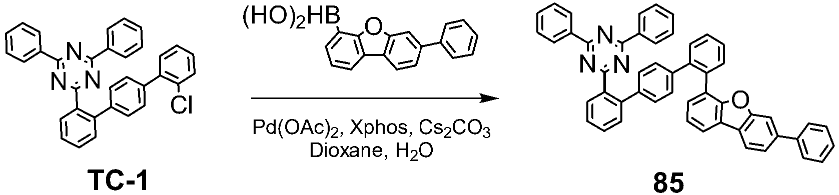

TC-1 (5.0g, 10.1mmol) obtained in Preparation Example 1, (7-phenyldibenzo[b,d]furan-4-yl)boronic acid (2.9g, 10.1mmol), Pd(OAc)₂ (0.1g, 0.3mmol), XPhos (0.3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 85 (3.3g, 46% yield).

Mass: [(M+H)+] : 705

### [Synthesis Example 9] Synthesis of Compound 142

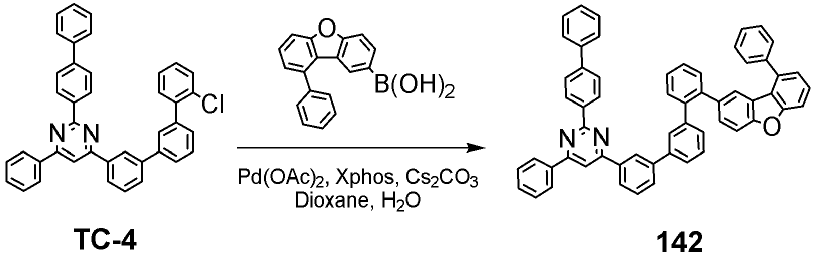

TC-4 (5.0g, 8.7mmol) obtained in Preparation Example 4, (9-phenyldibenzo[b,d]furan-2-yl)boronic acid (2.5g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 142 (3.2g, 47% yield).

Mass: [(M+H)+] : 780

### [Synthesis Example 10] Synthesis of Compound 165

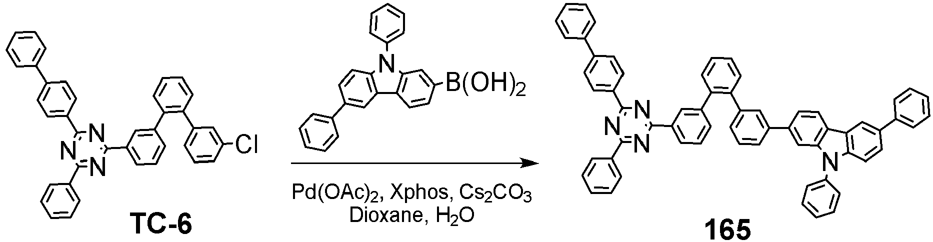

TC-6 (5.0g, 8.7mmol) obtained in Preparation Example 6, (6,9-diphenyl-9H-carbazol-2-yl)boronic acid (3.2g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 165 (3.4g, 45% yield).

Mass: [(M+H)+] : 856

### [Synthesis Example 11] Synthesis of Compound 167

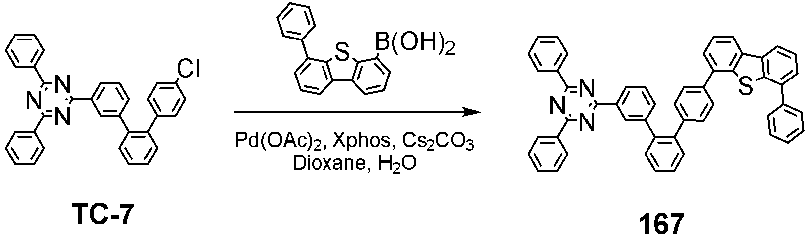

TC-7 (5.0g, 10.1mmol) obtained in Preparation Example 7, (6-phenyldibenzo[b,d]thiophen-4-yl)boronic acid (3.1g, 10.1mmol), Pd(OAc)₂ (0.1g, 0.3mmol), XPhos (0.3g, 0.6mmol), and Cs₂CO₃ (6.6g, 20.2mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 167 (3.1g, 43% yield).

Mass: [(M+H)+] : 721

### [Synthesis Example 12] Synthesis of Compound 223

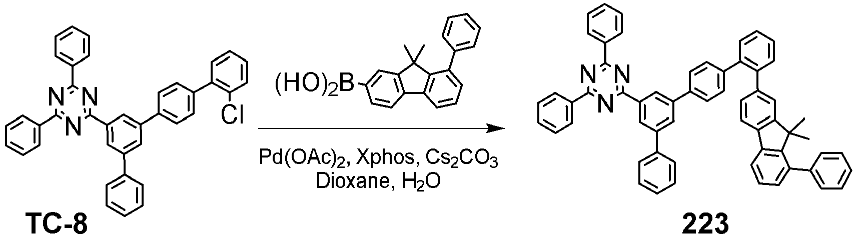

TC-8 (5.0g, 8.7mmol) obtained in Preparation Example 8, (9,9-dimethyl-8-phenyl-9H-fluoren-2-yl)boronic acid (2.7g, 8.7mmol), Pd(OAc)₂ (0.1g, 0.3mmol), XPhos (0.3g, 0.5mmol), and Cs₂CO₃ (5.7g, 17.5mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 223 (3.0g, 43% yield).

Mass: [(M+H)+] : 806

### [Synthesis Example 13] Synthesis of Compound 250

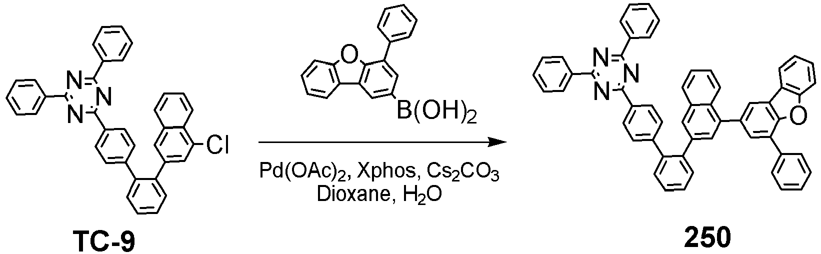

TC-9 (5.0g, 9.2mmol) obtained in Preparation Example 9, (4-phenyldibenzo[b,d]furan-2-yl)boronic acid (2.6g, 9.2mmol), Pd(OAc)₂ (0.1g, 0.3mmol), XPhos (0.3g, 0.5mmol), and Cs₂CO₃ (6.0g, 18.3mmol) were added to 80 ml of Dioxane and 20 ml of H₂O, and the resulting mixture was heated under reflux with stirring for 3 hours. After the reaction was completed, the reaction mixture was quenched with a sufficient amount of water. The resulting solid was filtered to remove the solution and then dried in an oven. The dried solid was purified by column chromatography to obtain Compound 250 (2.6g, 38% yield).

Mass: [(M+H)+] : 755

### [Example 1] Manufacturing of Blue organic EL device

The compound 1 synthesized in the above synthesis example 1 were subjected to high-purity sublimation purification in a conventionally known method, and then blue organic EL device (OLED) was manufactured as follows.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, 98 wt% HI + 2 wt% HAT-CN6 (10 nm)/HI(140 nm) /EB (5 nm)/98 wt% BH + 2 wt% BD (20nm)/Compound 1 + Liq (1:1) (30 nm)/LiF (1 nm)/Al (100 nm) were stacked in order to manufacture an organic EL device. The structures of HI, HAT-CN6, EB, BH, BD, and Liq used in Example 1 below are as follows, respectively.

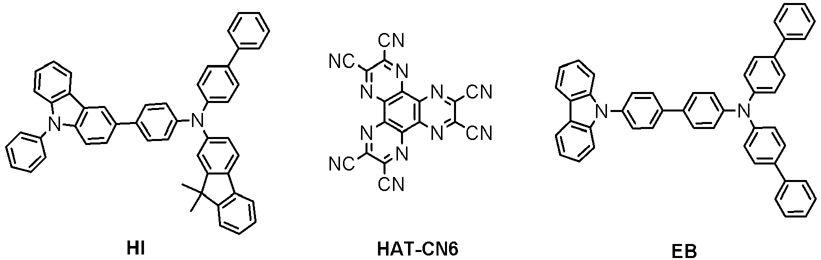

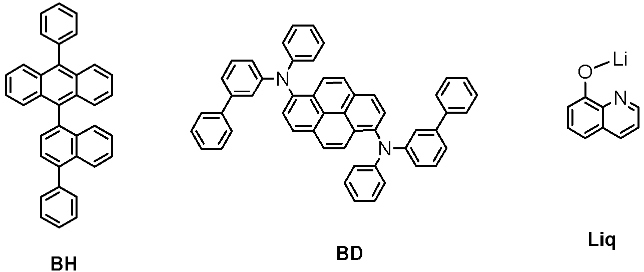

### [Examples 2 to 13] Manufacturing of Blue organic EL device

Blue organic EL devices were manufactured in the same manner as Example 1, except that each of the compounds listed in Table 1 below was used as an electron transport layer material instead of Compound 1 used in Example 1.

### [Comparative Examples 1 to 7] Manufacturing of blue organic EL device

Blue organic EL devices were manufactured in the same manner as Example 1, except that each of Alq₃, TP-1 to TP-6 was used as an electron transport layer material instead of Compound 1 used in Example 1. The structures of Alq₃, TP-1 to TP-6 used in Comparative Examples are as follows, respectively.

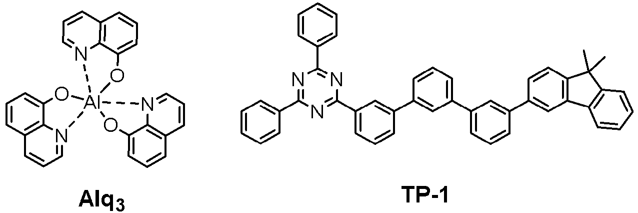

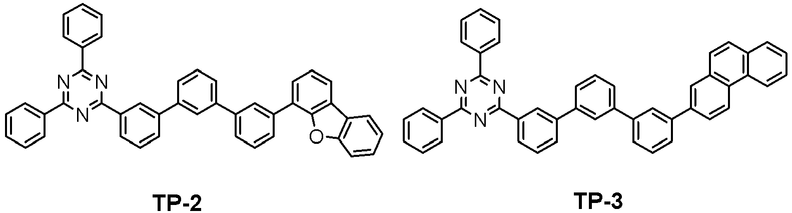

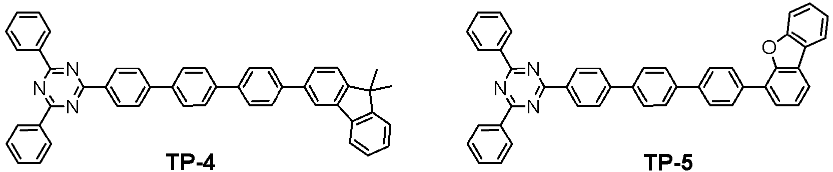

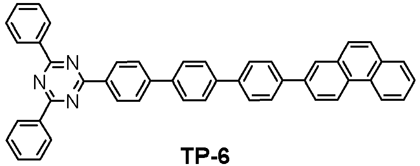

### [Evaluation example 1]

For each of the organic EL devices manufactured in Examples (Ex.) 1 to 13 and Comparative Examples (Comp. Ex.) 1 to 7, a driving voltage, a current efficiency at a current density of 10 mA/cm2, and an emission peak were measured, and the results are shown in Table 1 below.

**[Table 1]**

| Sample | Electron transport layer | Driving voltage (V) | EL peak (nm) | Current efficiency (cd/A) |
|---|---|---|---|---|
| Ex. 1 | Compound 1 | 3.4 | 454 | 7.0 |
| Ex. 2 | Compound 21 | 3.3 | 455 | 7.1 |
| Ex. 3 | Compound 30 | 3.2 | 453 | 7.0 |
| Ex. 4 | Compound 58 | 3.4 | 454 | 7.1 |
| Ex. 5 | Compound 67 | 3.2 | 454 | 6.9 |
| Ex. 6 | Compound 81 | 3.3 | 453 | 6.8 |
| Ex. 7 | Compound 83 | 3.4 | 455 | 7.0 |
| Ex. 8 | Compound 85 | 3.5 | 455 | 7.0 |
| Ex. 9 | Compound 142 | 3.3 | 454 | 7.1 |
| Ex. 10 | Compound 165 | 3.2 | 454 | 7.0 |
| Ex. 11 | Compound 167 | 3.2 | 455 | 7.0 |
| Ex. 12 | Compound 223 | 3.3 | 453 | 6.9 |
| Ex. 13 | Compound 250 | 3.2 | 454 | 7.1 |
| Comp. Ex. 1 | Alq₃ | 4. 6 | 457 | 5.1 |
| Comp. Ex. 2 | TP-1 | 4.0 | 458 | 6.0 |
| Comp. Ex. 3 | TP-2 | 4.1 | 457 | 6.1 |
| Comp. Ex. 4 | TP-3 | 4. 6 | 456 | 6.1 |
| Comp. Ex. 5 | TP-4 | 4.0 | 457 | 6.0 |
| Comp. Ex. 6 | TP-5 | 4.1 | 457 | 6.0 |
| Comp. Ex. 7 | TP-6 | 4. 6 | 457 | 6.1 |

As shown in Table 1, it was confirmed that the organic EL devices of Examples 1 to 13 exhibit significantly excellent performance in terms of driving voltage, emission peak and current efficiency compared to the organic EL device of Comparative Examples 1 to 7.

### [Example 14] Manufacturing of Blue organic EL device

The compound 1 synthesized in the above synthesis example 1 were subjected to high-purity sublimation purification in a conventionally known method, and then blue organic EL device (OLED) was manufactured as follows.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, 98 wt% HI + 2 wt% HAT-CN6 (10 nm)/HI(140 nm)/EB (5 nm)/98wt% BH + 2 wt% BD (20nm)/Compound 1 (5 nm)/ET + Liq (1:1) (30 nm)/LiF (1 nm)/Al (100 nm) were stacked in order to manufacture an organic EL device. The structures of HI, HAT-CN6, EB, BH, BD, ET, and Liq used in Example 14 below are as follows, respectively.

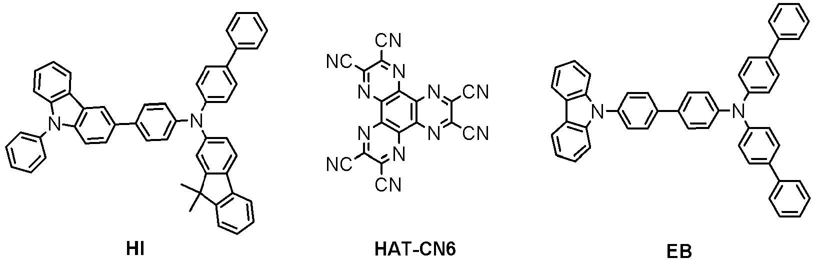

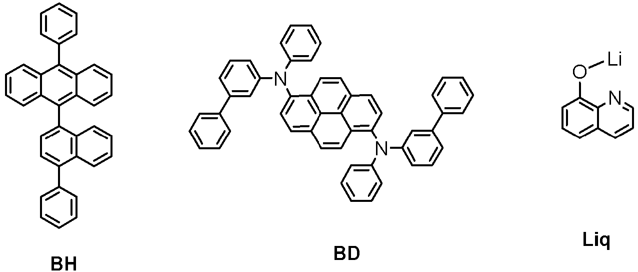

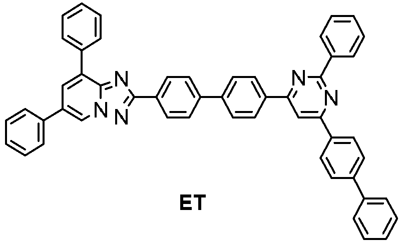

### [Examples 15 to 26]

Blue organic EL devices were manufactured in the same manner as Example 14, except that each of the compounds listed in Table 2 below was used as an electron transport auxiliary layer material instead of Compound 1 used in Example 14.

### [Comparative Example 8]

Blue organic EL device was manufactured in the same manner as Example 14, except that Compound 1 used as a material for the electron transport auxiliary layer in Example 14 was not used.

### [Comparative Examples 9 to 14] Manufacturing of blue organic EL device

Blue organic EL devices were manufactured in the same manner as Example 14, except that each of TP-1 to TP-6 was used as an electron transport auxiliary layer material instead of Compound 1 used in Example 14. The structures of TP-1 to TP-6 used in Comparative Examples are as follows, respectively.

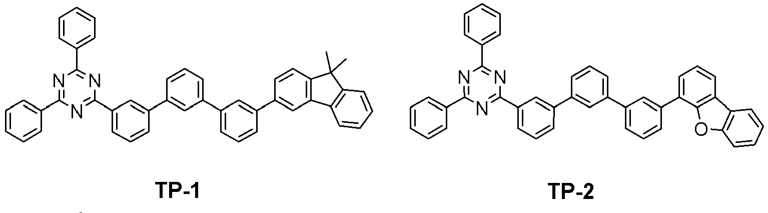

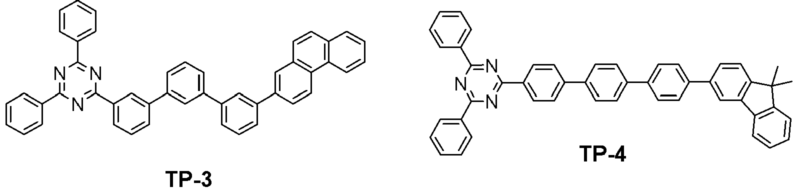

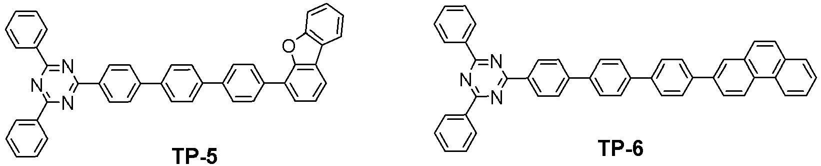

### [Evaluation example 2]

For each of the organic EL devices manufactured in Examples (Ex.) 14 to 26 and Comparative Examples (Comp. Ex.) 8 to 14, a driving voltage, a current efficiency at a current density of 10 mA/cm2, and an emission peak were measured, and the results are shown in Table 2 below.

**[Table 2]**

| Sample | Electron transport auxiliary layer | Driving voltage (V) | EL peak (nm) | Current efficiency (cd/A) |
|---|---|---|---|---|
| Ex. 14 | Compound 1 | 3.1 | 455 | 7.4 |
| Ex. 15 | Compound 21 | 3.2 | 454 | 7.3 |
| Ex. 16 | Compound 30 | 3.1 | 454 | 7.4 |
| Ex. 17 | Compound 58 | 3.1 | 455 | 7.5 |
| Ex. 18 | Compound 67 | 3.2 | 455 | 7.4 |
| Ex. 19 | Compound 81 | 3.1 | 454 | 7.3 |
| Ex. 20 | Compound 83 | 3.2 | 455 | 7.4 |
| Ex. 21 | Compound 85 | 3.3 | 455 | 7.3 |
| Ex. 22 | Compound 142 | 3.3 | 454 | 7.3 |
| Ex. 23 | Compound 165 | 3.2 | 455 | 7.4 |
| Ex. 24 | Compound 167 | 3.2 | 455 | 7.3 |
| Ex. 25 | Compound 223 | 3.2 | 454 | 7.3 |
| Ex. 26 | Compound 250 | 3.1 | 454 | 7.2 |
| Comp. Ex. 8 | - | 4.6 | 456 | 5.6 |
| Comp. Ex. 9 | TP-1 | 3.8 | 455 | 6.0 |
| Comp. Ex. 10 | TP-2 | 4.1 | 456 | 6.1 |
| Comp. Ex. 11 | TP-3 | 4.5 | 456 | 6.1 |
| Comp. Ex. 12 | TP-4 | 4.0 | 456 | 6.1 |
| Comp. Ex. 13 | TP-5 | 3.9 | 456 | 6.1 |
| Comp. Ex. 14 | TP-6 | 4.5 | 456 | 6.0 |

As shown in Table 2, it was confirmed that the organic EL devices of Examples 14 to 26 exhibit significantly excellent performance in terms of driving voltage, emission peak and current efficiency compared to the organic EL device of Comparative Examples 8 to 14.

## Claims

1. A compound represented by Chemical Formula 1 below: 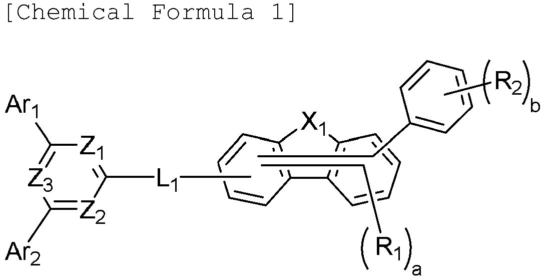 (wherein in Chemical Formula 1,
Z₁ to Z₃ are the same as or different from each other and are each independently N or C(Ar₃), provided that at least two of Z₁ to Z₃ are N;
Ar₁ to Ar₂ are the same as or different from each other and are each independently selected from the group consisting of a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group and a C₆-C₆₀ arylamine group,
L₁ is a linker represented by the following Chemical Formula L1-1 or L1-2, 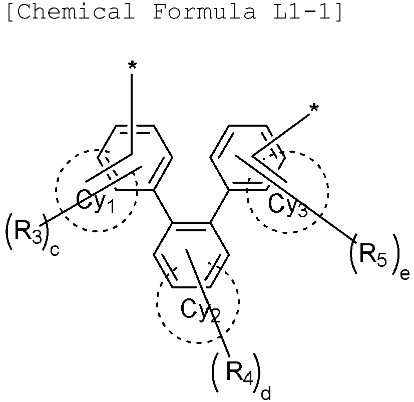 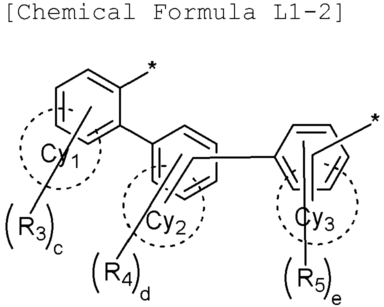
wherein in Chemical Formula L1-1 or L1-2,
a dotted line indicates that each ring is present or absent,
Cy₁ to Cy₃ are the same as or different from each other and are each independently a 6-membered aromatic ring,
a, c, d and e are each independently an integer of 0 to 6,
b is an integer of 0 to 5,
R₁ to R₅ are the same as or different from each other and are each independently selected from the group consisting of a hydrogen, a deuterium (D), a halogen, a cyano group, a nitro group, an amino group, a hydroxy group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
X₁ is selected from the group consisting of O, S, C(Ar₄)(Ar₅), N(Ar₆) and 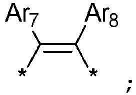
Ar₃ to Ar₈ are the same as or different from each other and are each independently selected from the group consisting of a hydrogen, a deuterium (D), a halogen, a cyano group, a nitro group, an amino group, a hydroxy group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₁-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group, and a C₆-C₆₀ arylamine group, or may be bonded to any adjacent group to form a fused ring,
the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, arylamine group and the fused ring of Ar₁ to Ar₈, and R₁ to R₅ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other) .

2. The compound of Claim 1, wherein L₁ is a linker represented by any one of the following Chemical Formulas L2-1 to L2-5: 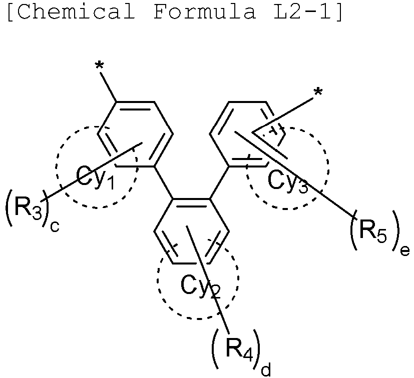 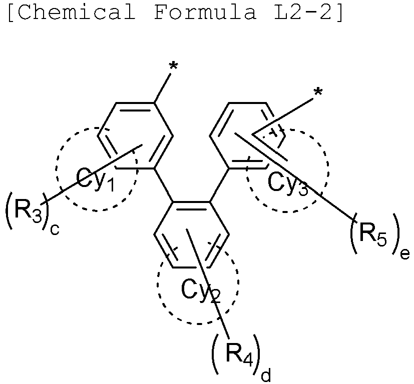 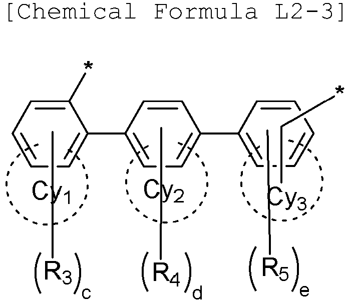 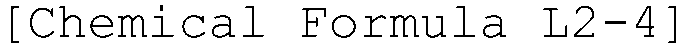 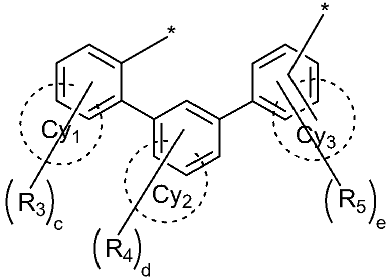 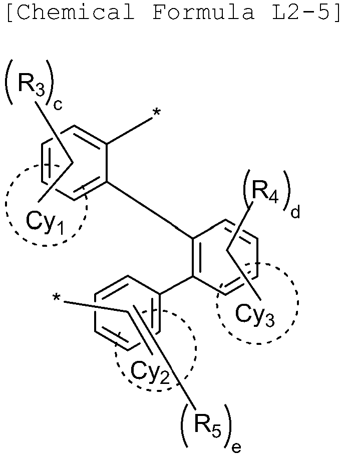
(wherein in Chemical Formulas L2-1 or L2-5,
the dotted line, Cy₁ to Cy₃, c, d, e, R₃ to R₄ are each as defined in claim 1).

3. The compound of Claim 1, wherein the compound represented by Chemical Formula 1 is represented by any one of Chemical Formulas 2 to 20: 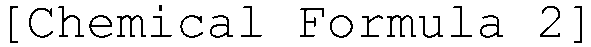 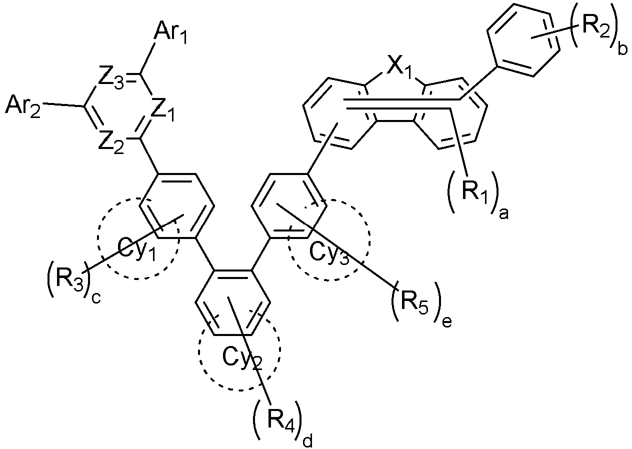 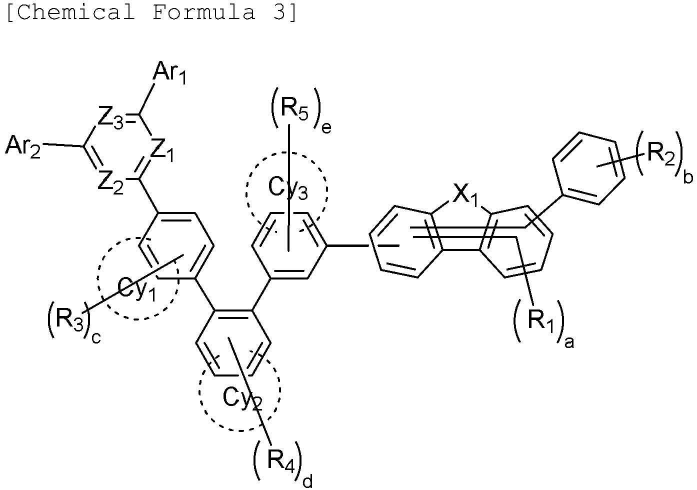 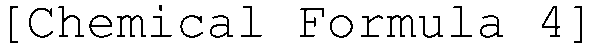 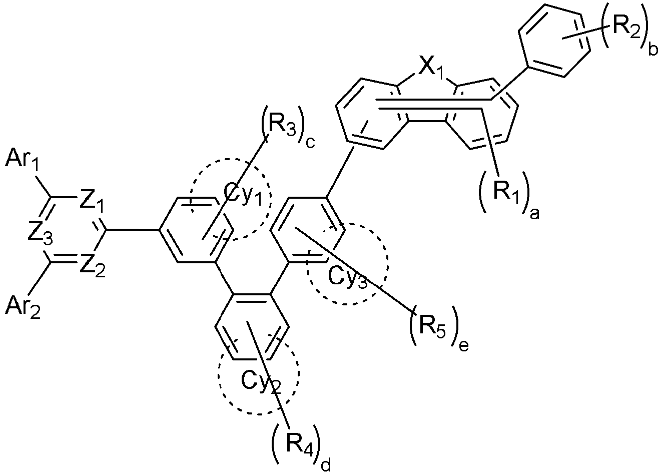 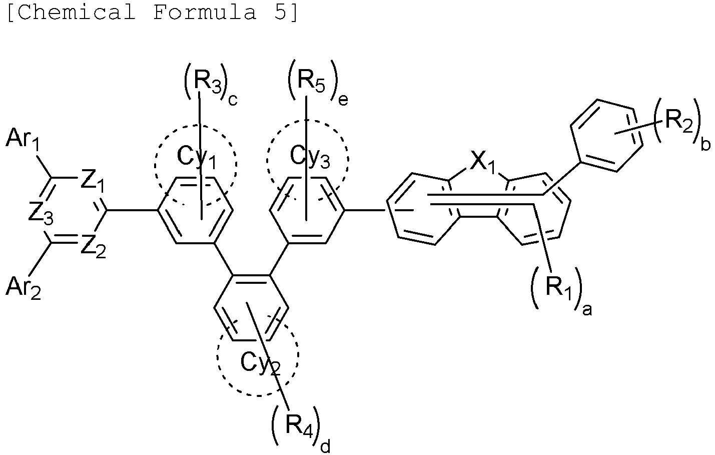 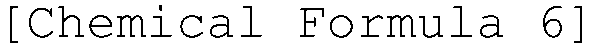 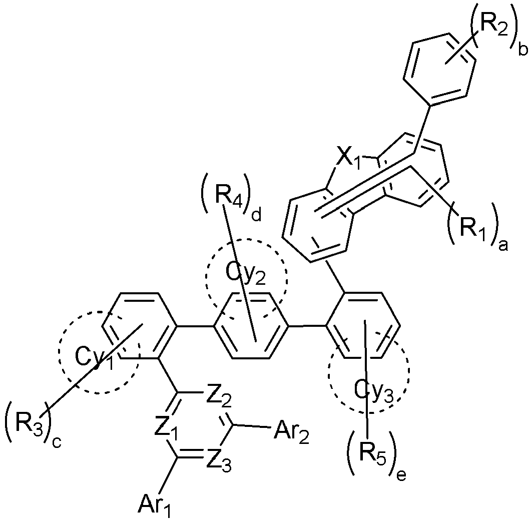 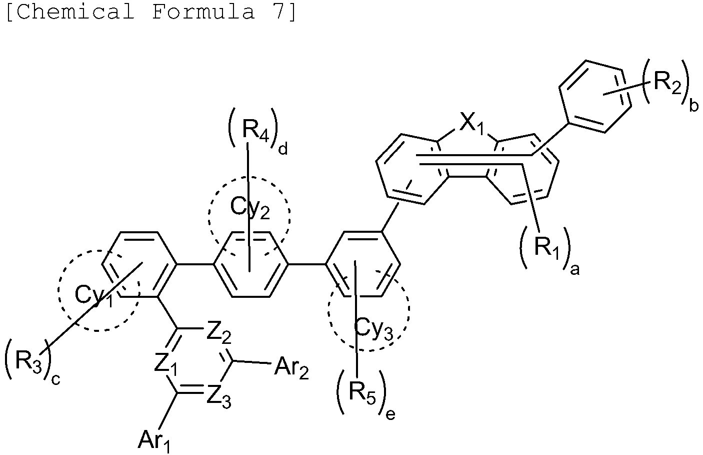 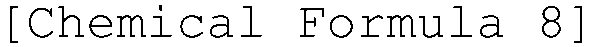 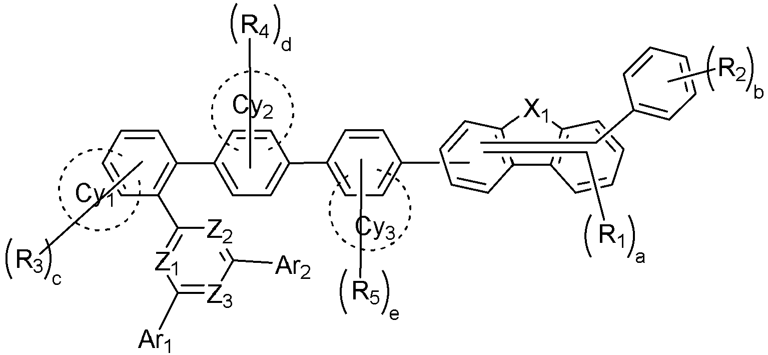 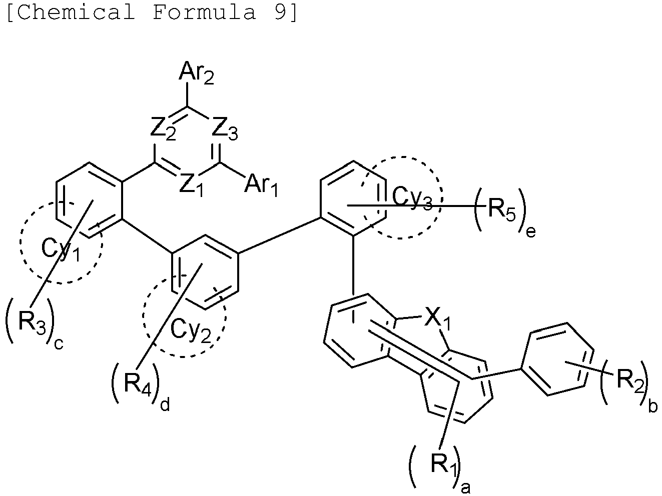 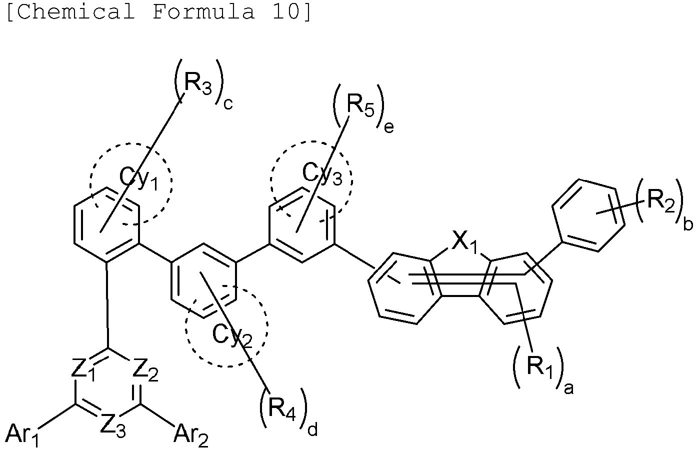 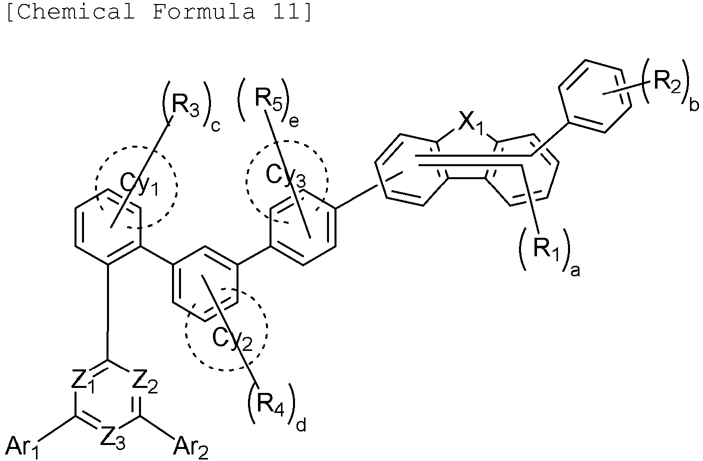 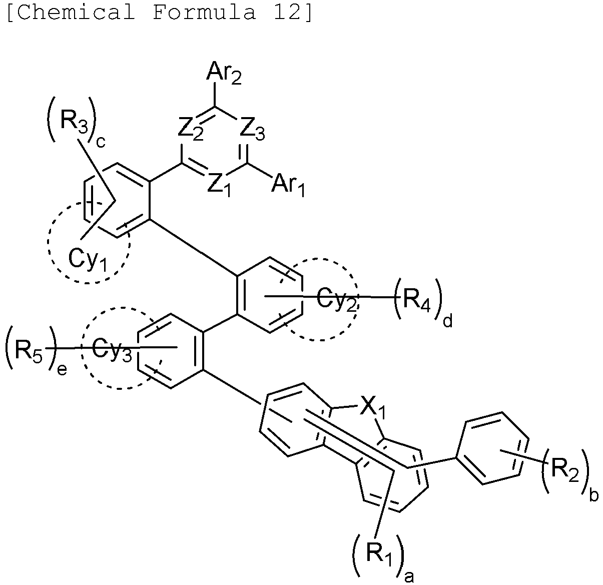 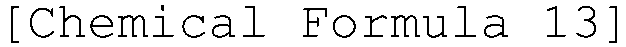 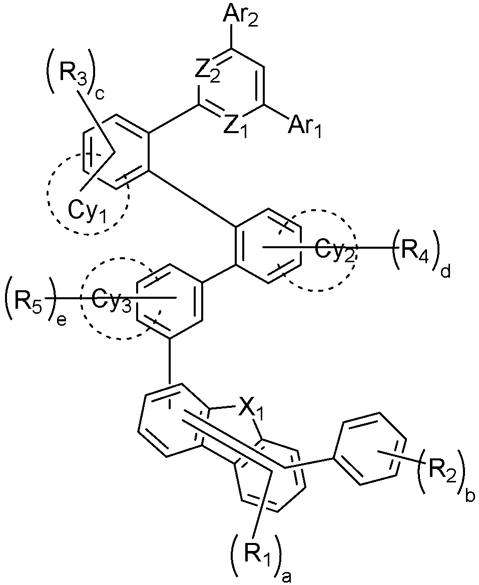 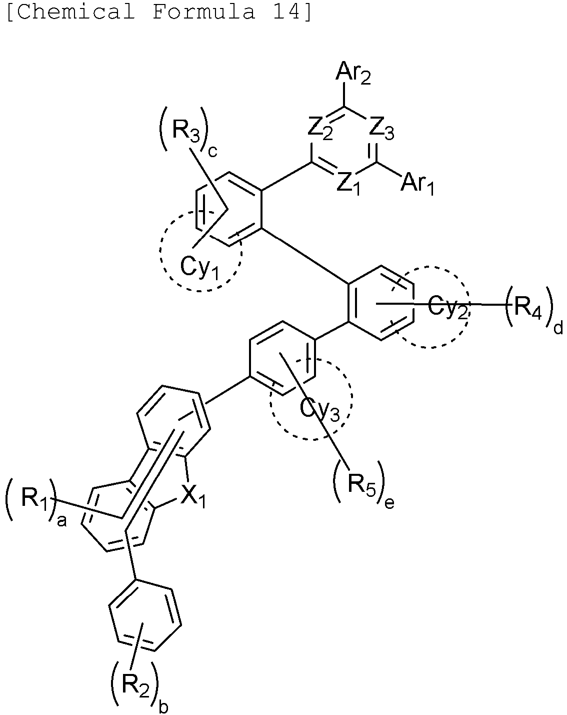 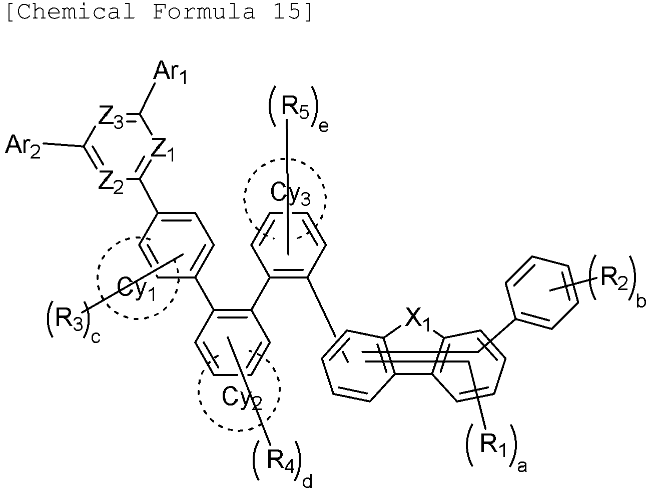 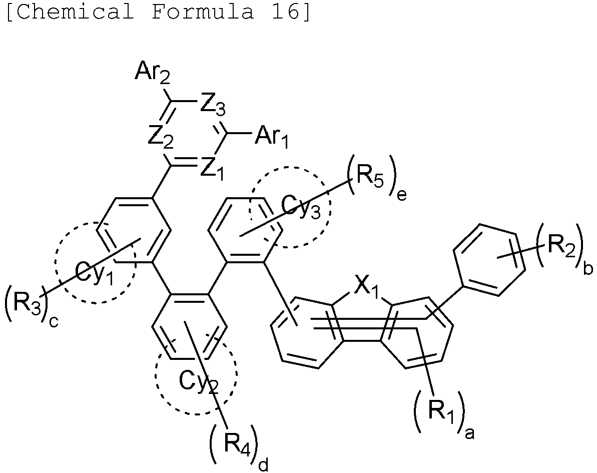 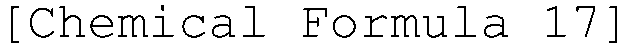 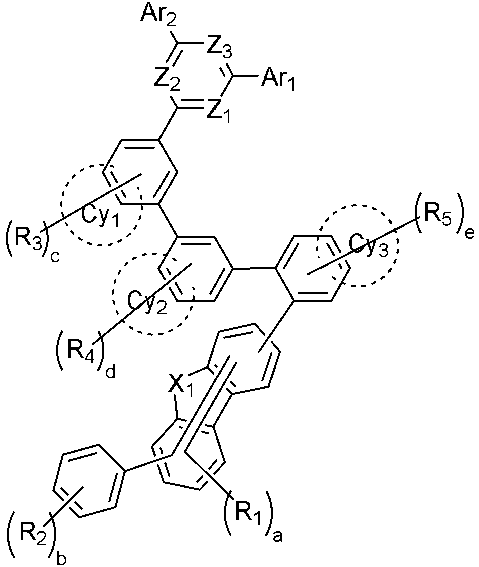 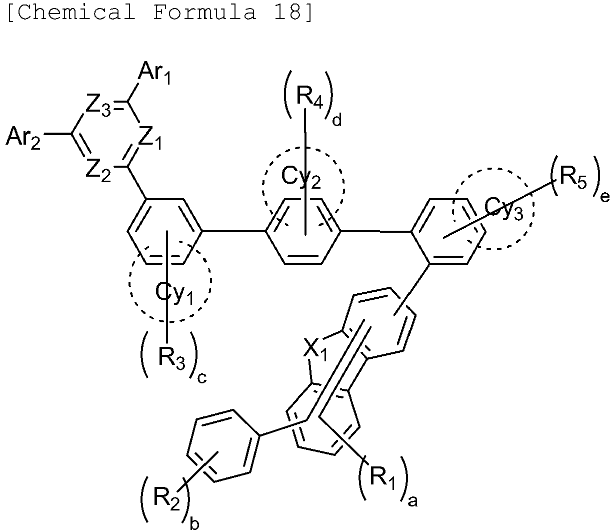 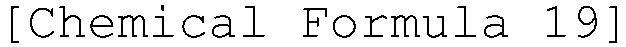 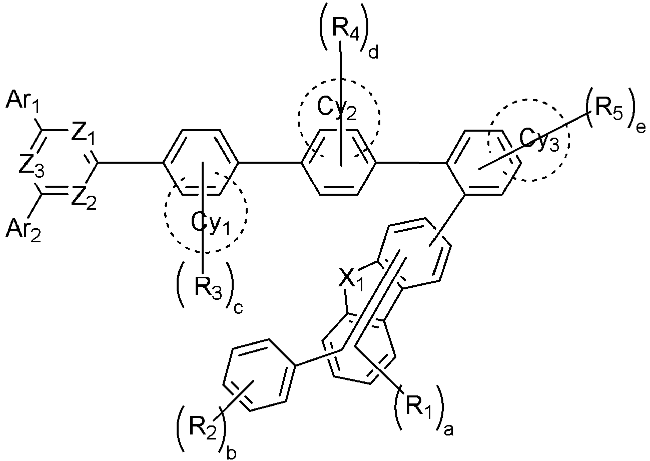 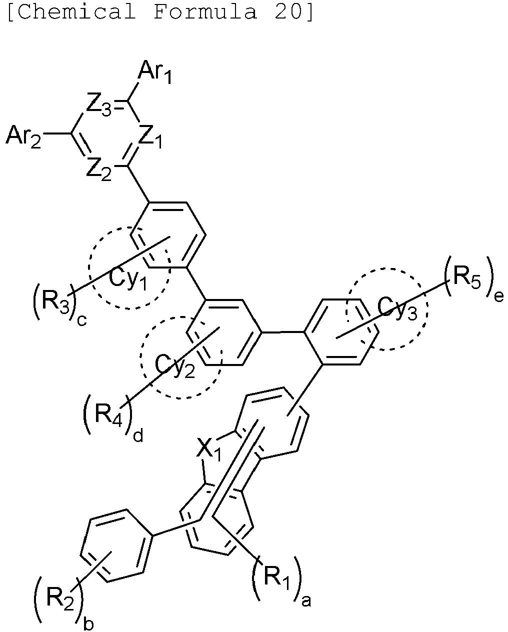
(wherein in Chemical Formulas 2 to 20,
Z₁ to Z₃, X₁, Ar₁, Ar₂, the dotted line, Cy₁ to Cy₃, a, b, c, d, e, R₁ to R₅ are each as defined in claim 1).

4. The compound of Claim 1,
wherein the moiety in Chemical Formula 1 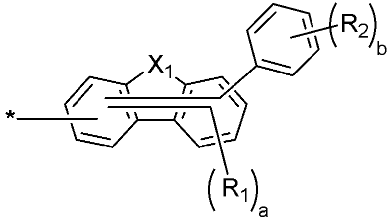 is a moiety selected from the group consisting of Mo1-1 to Mo1-10: 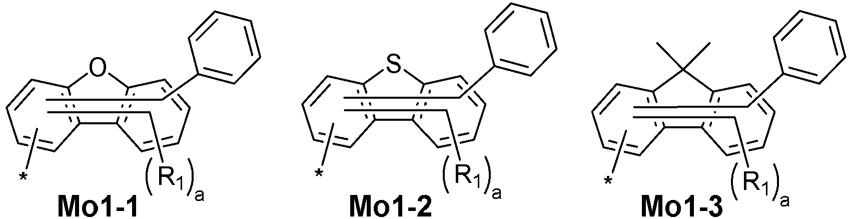 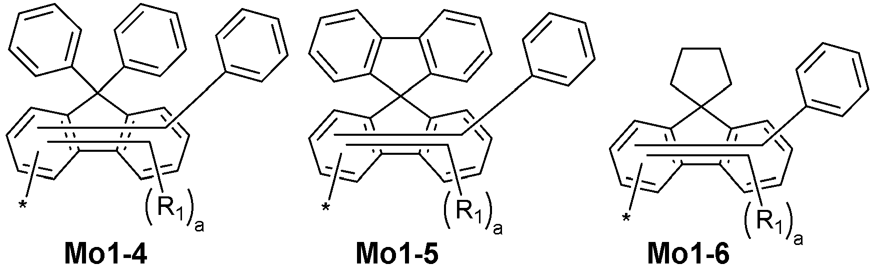 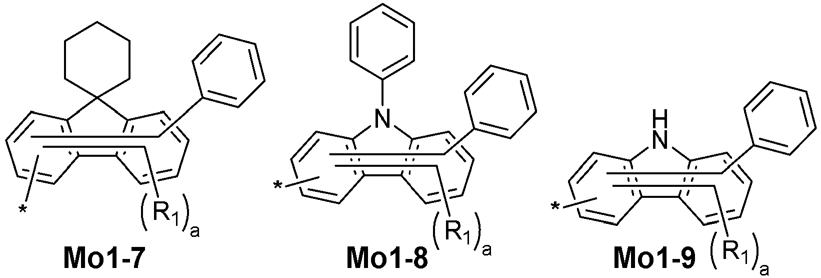 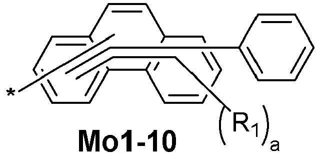
(wherein in moieties Mo1-1 to Mo1-10,
a, and R₁ are each as defined in claim 1).

5. The compound of Claim 1, wherein the compound represented by Chemical Formula 1 is represented by any one of Chemical Formulas 21 to 115: 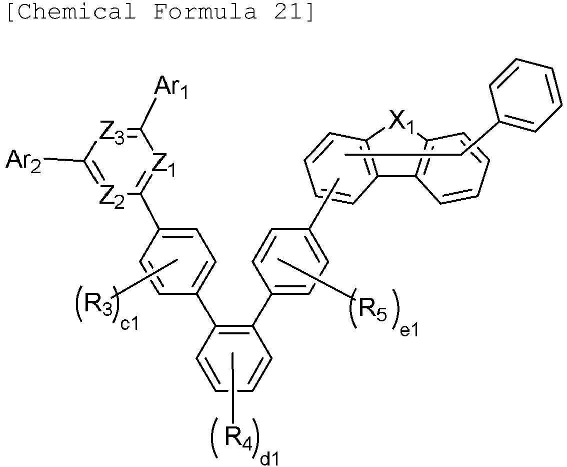 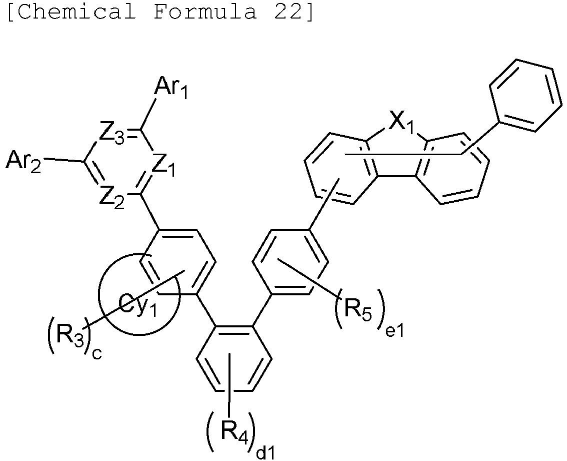 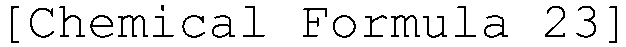 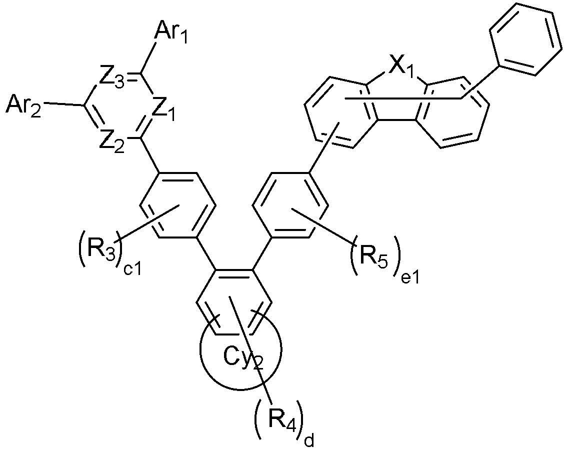 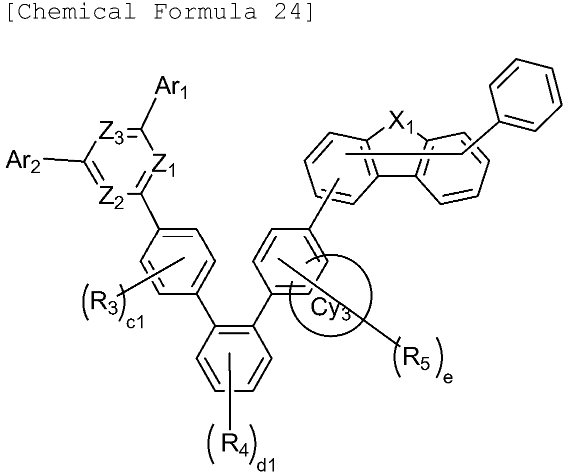 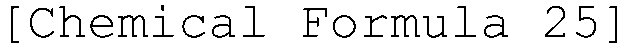 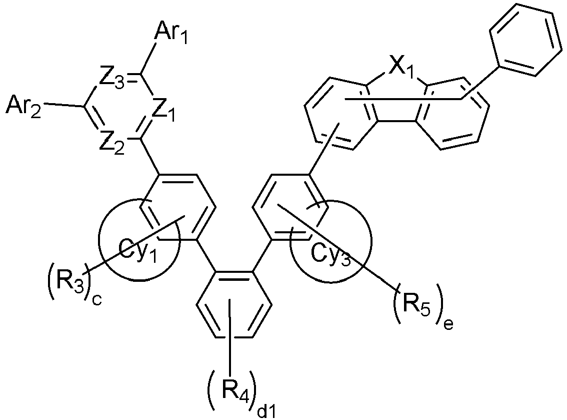 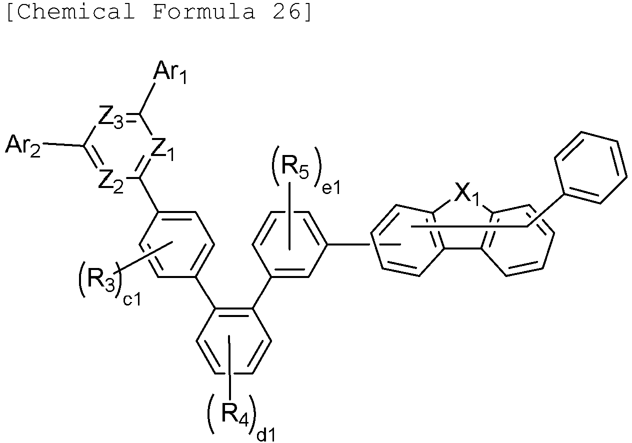 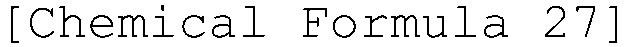 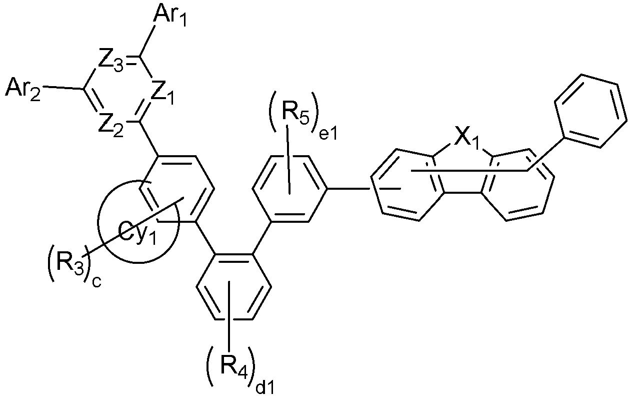 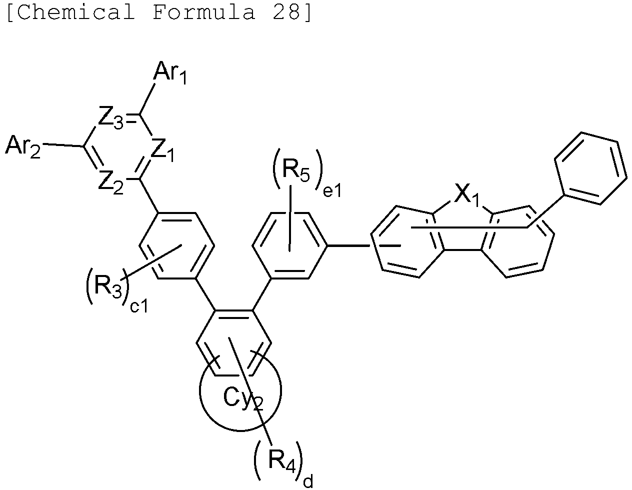 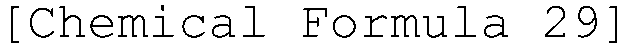 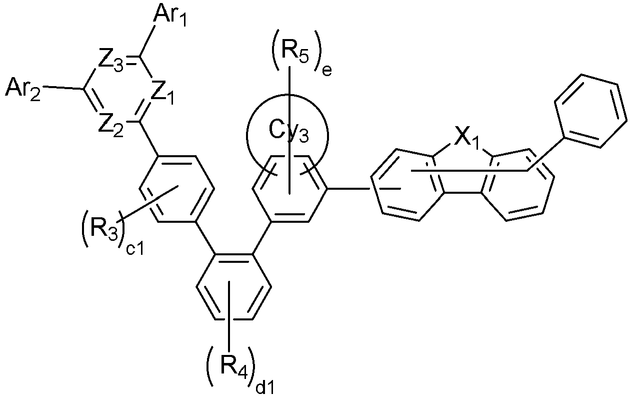 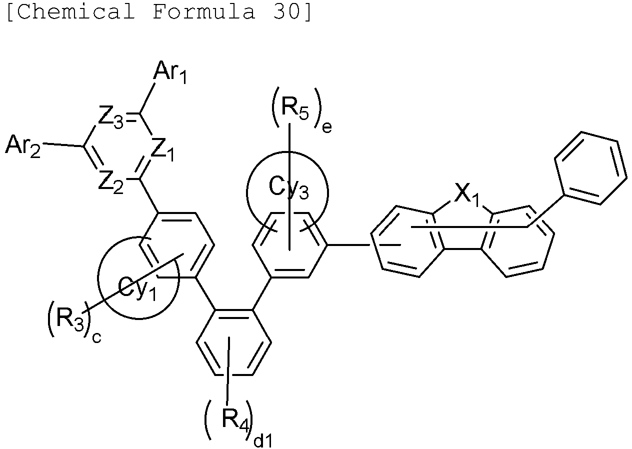 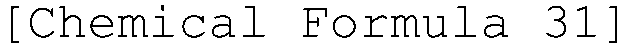 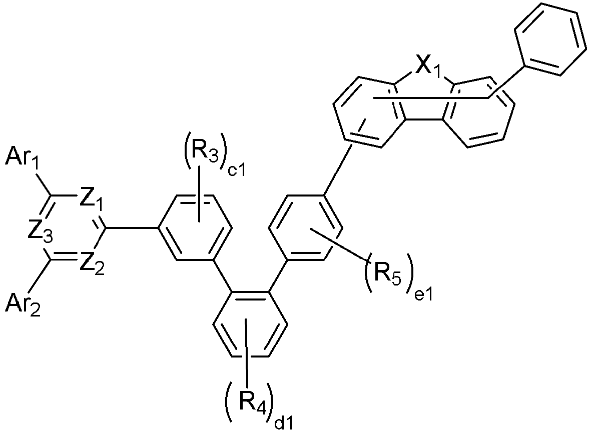 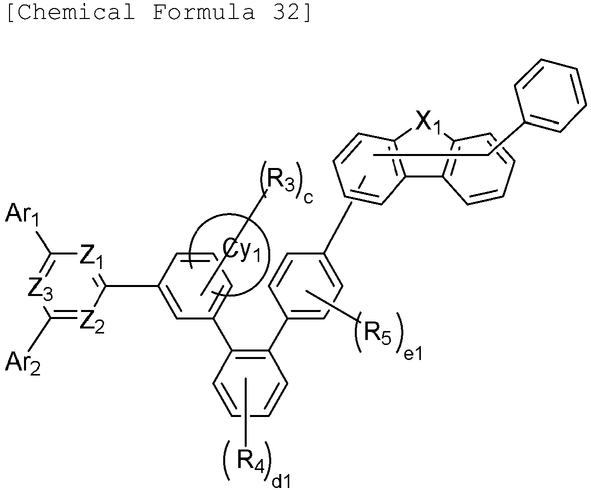 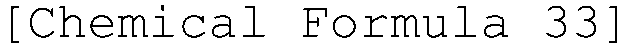 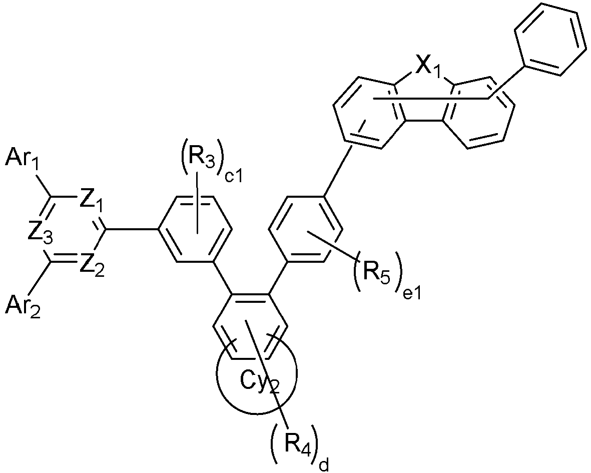 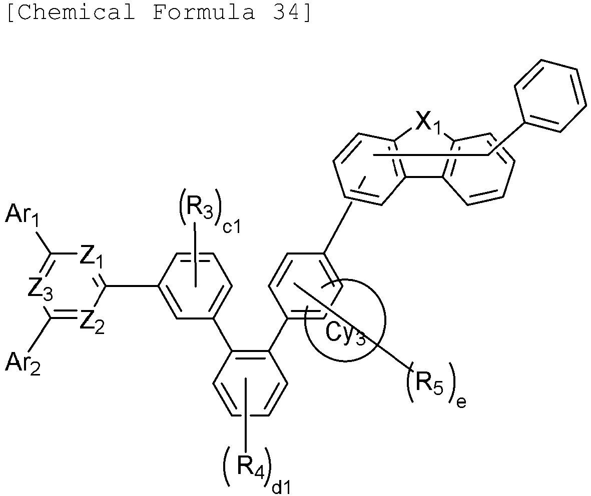 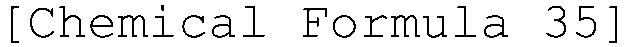 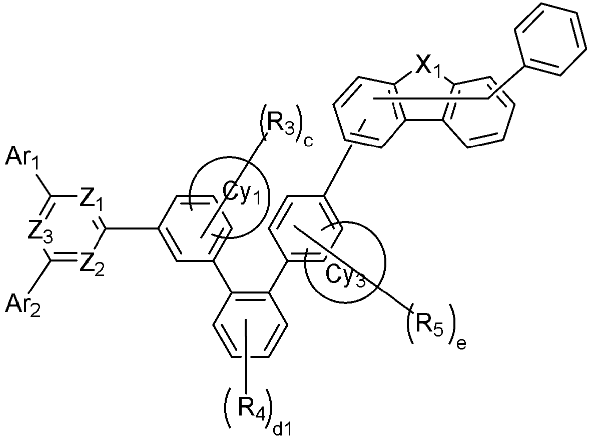 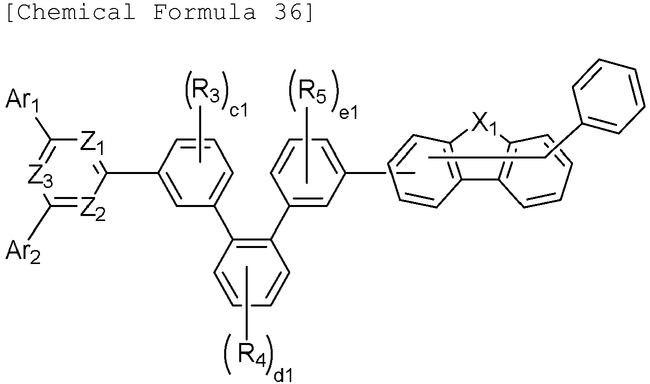 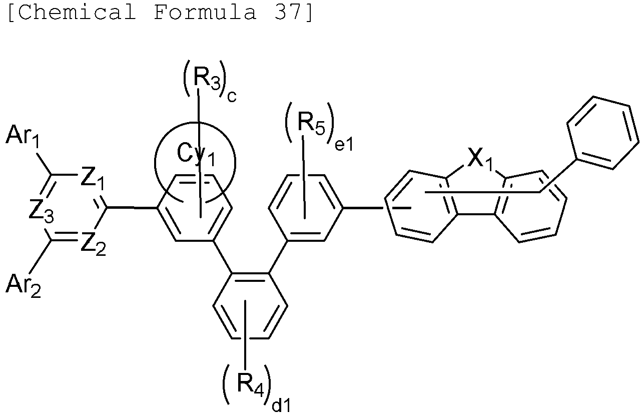 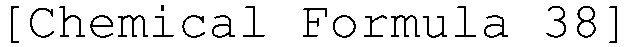 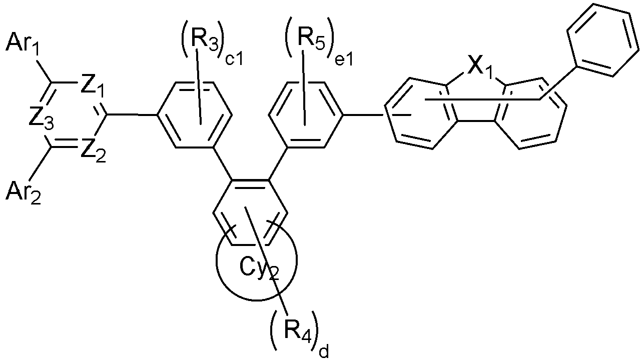 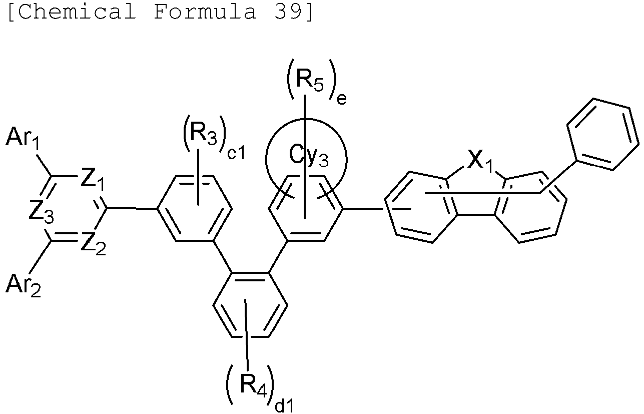 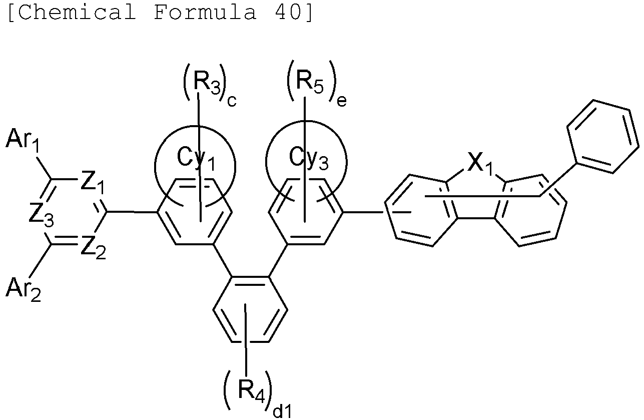 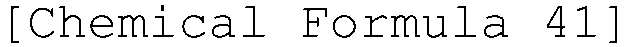 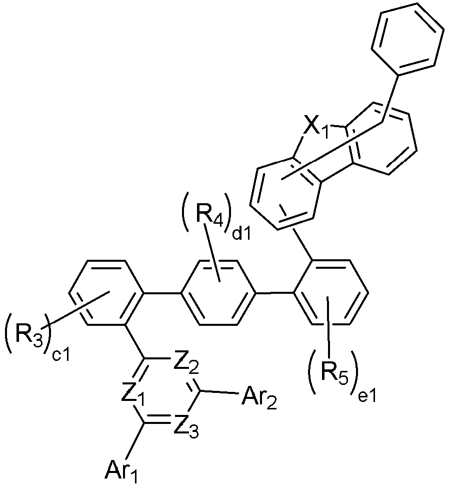 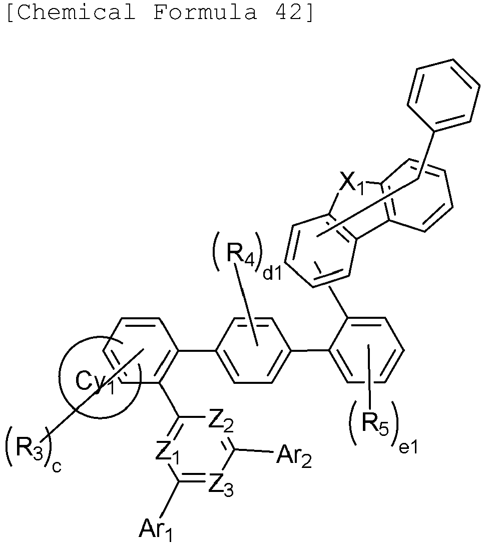 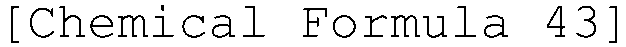 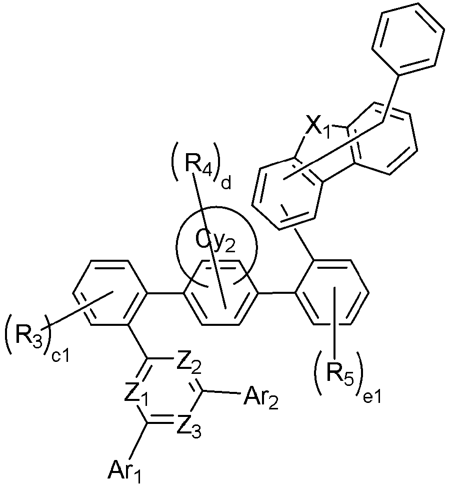 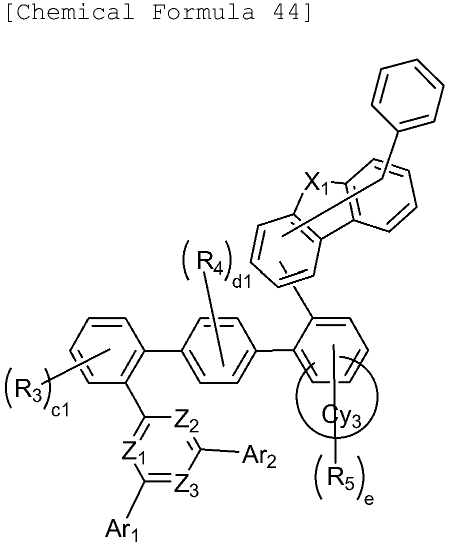 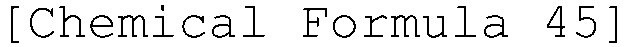 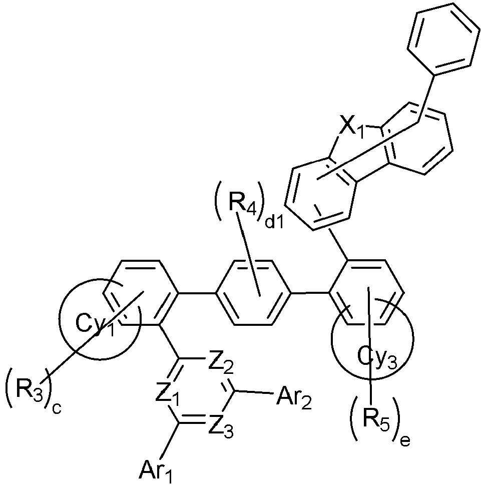 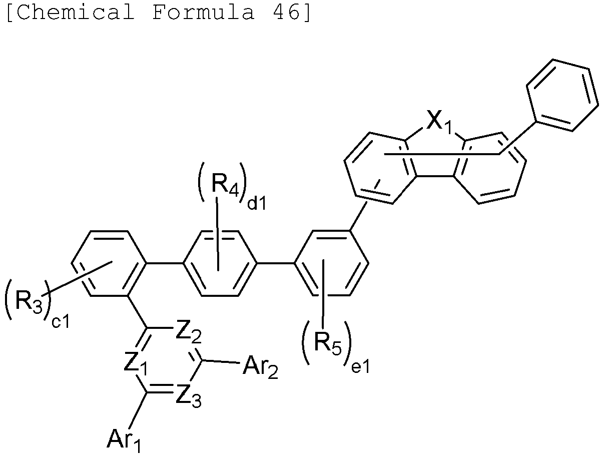 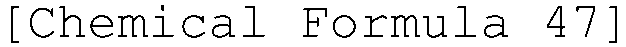 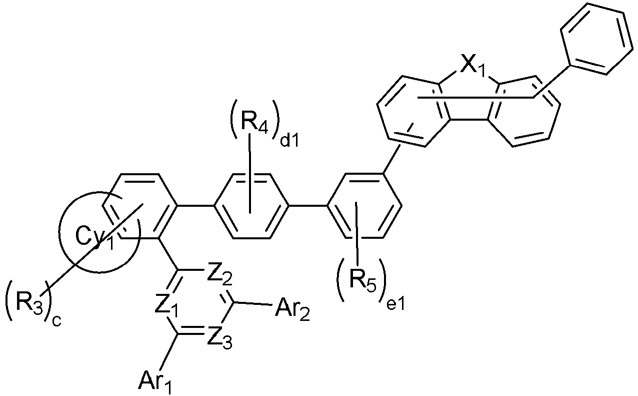 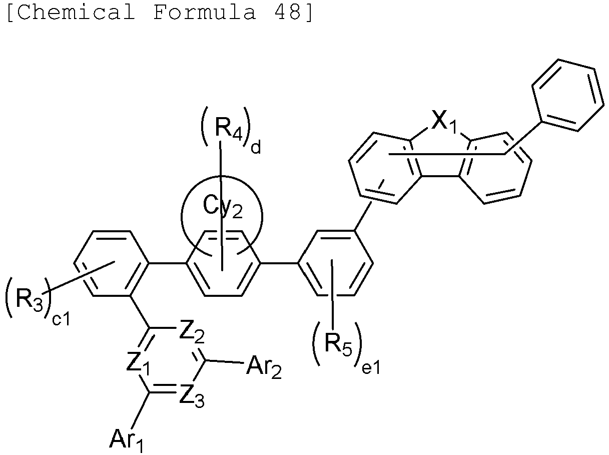 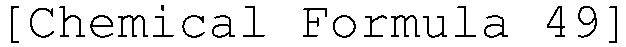 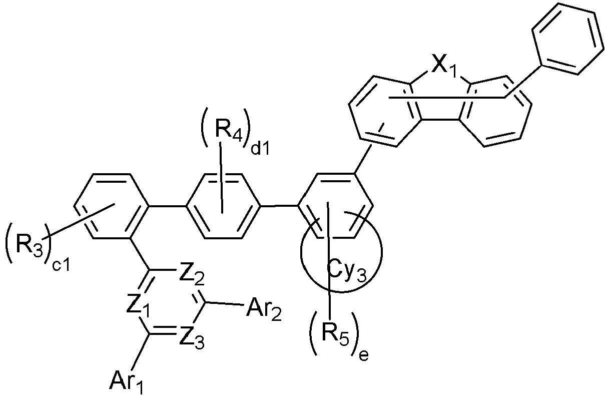 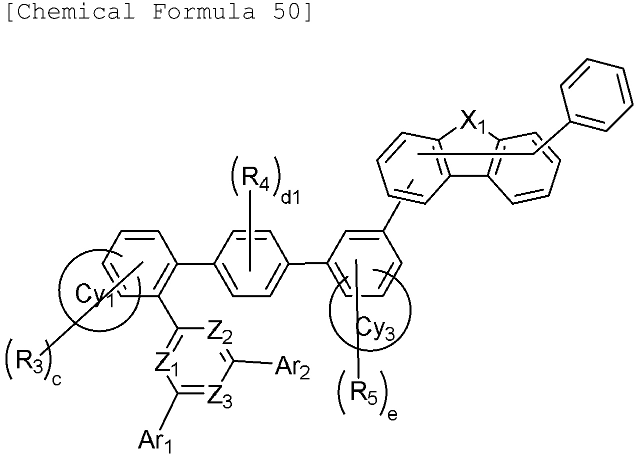 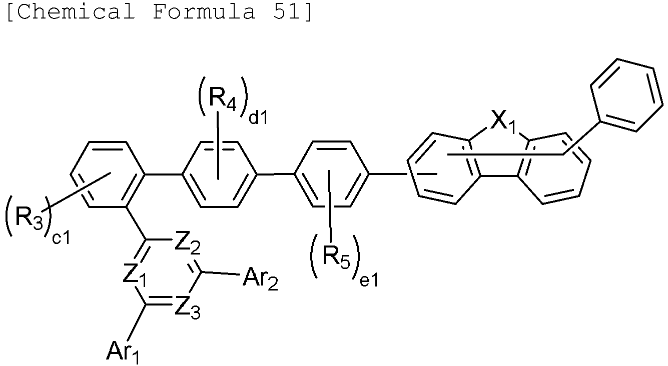 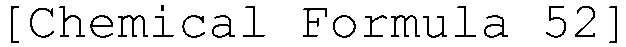 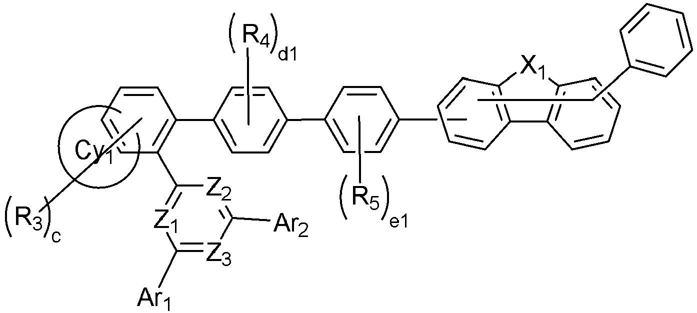 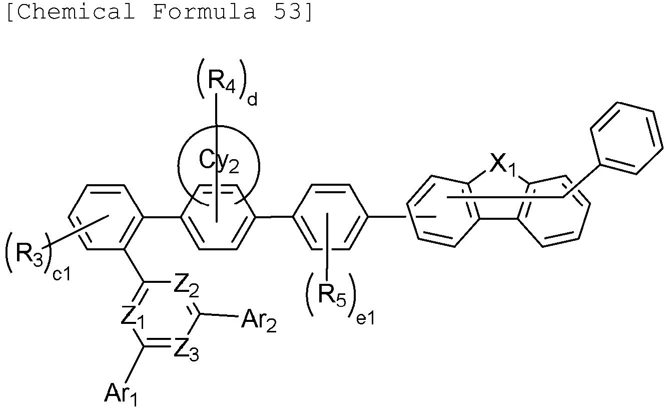 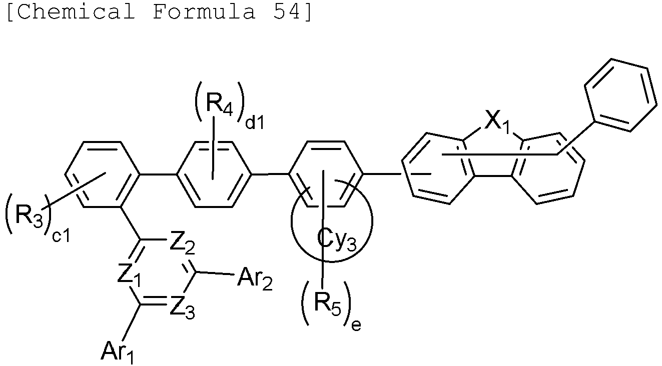 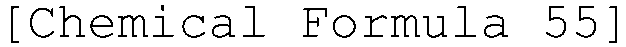 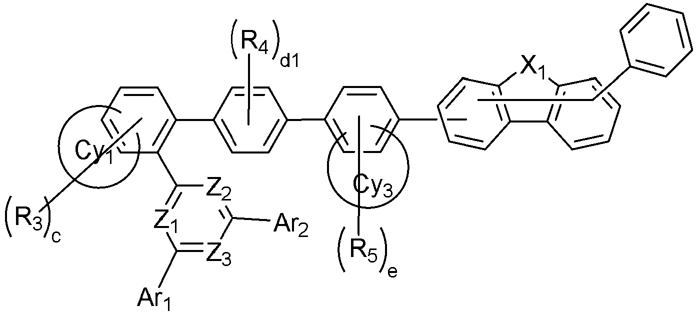 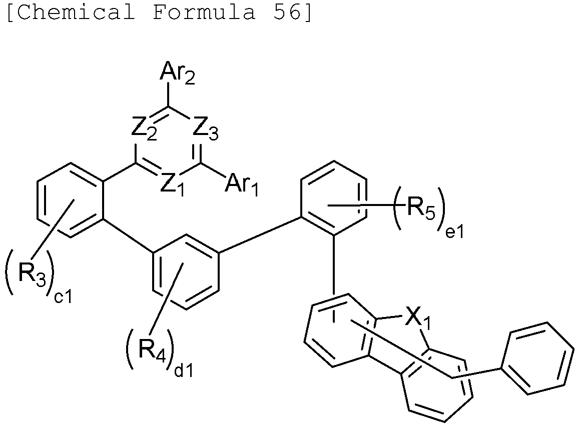 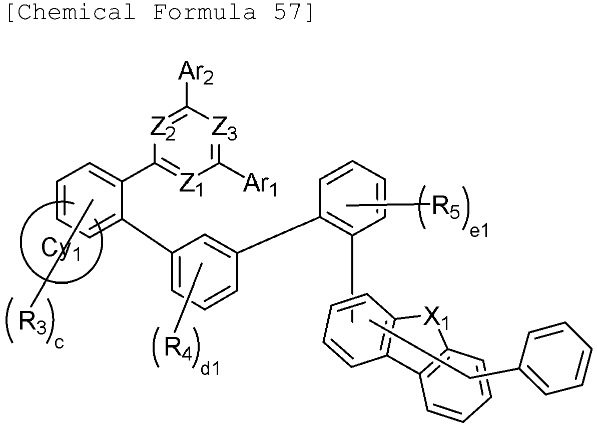 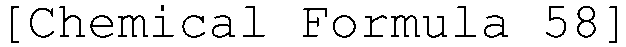 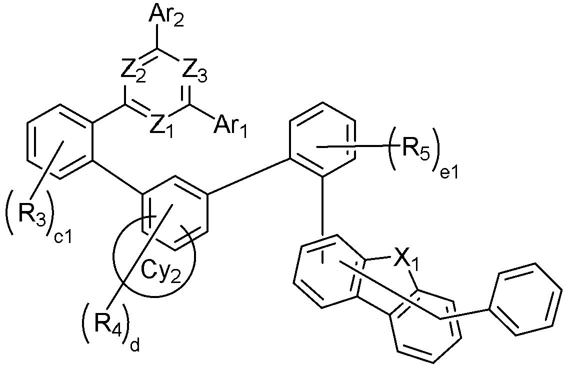 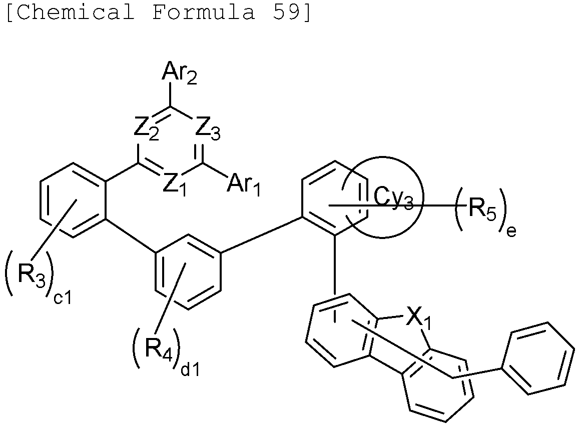 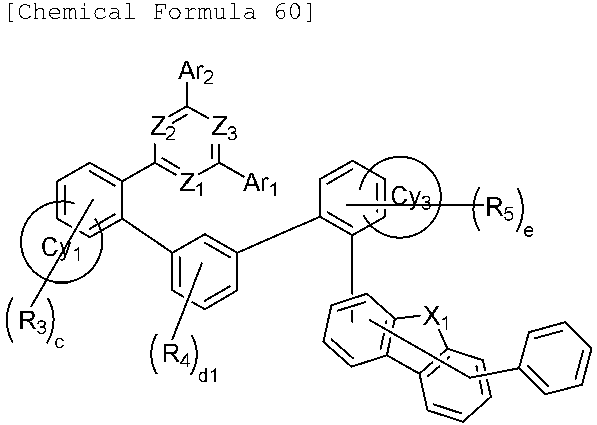 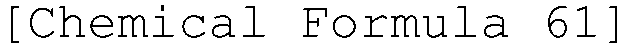 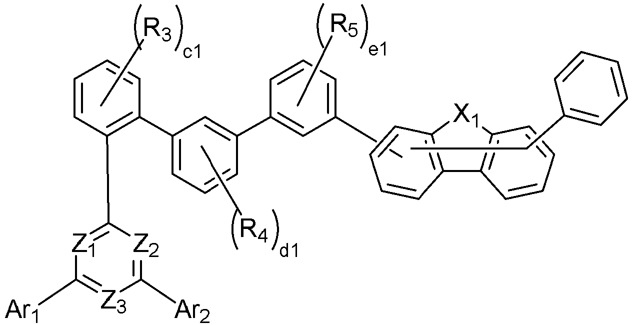 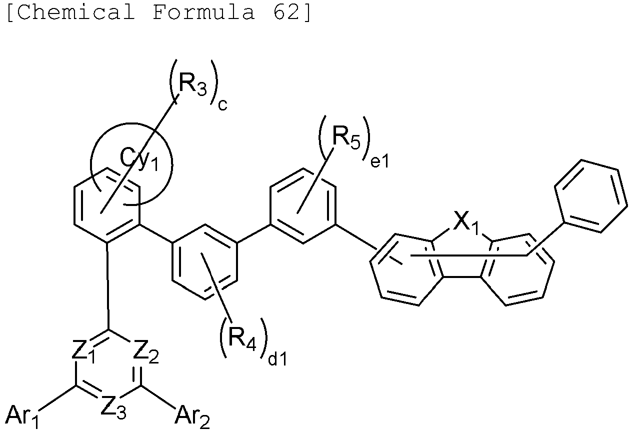 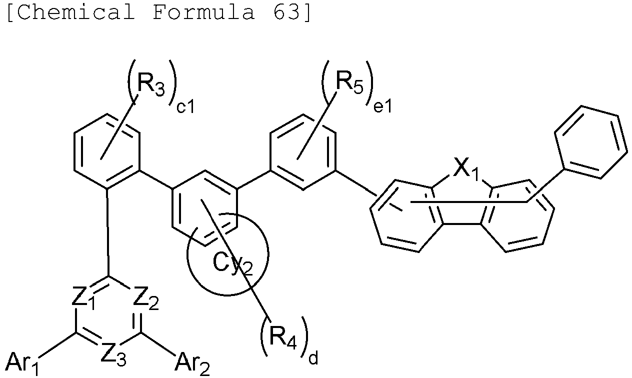 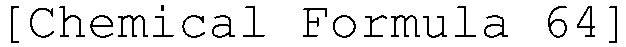 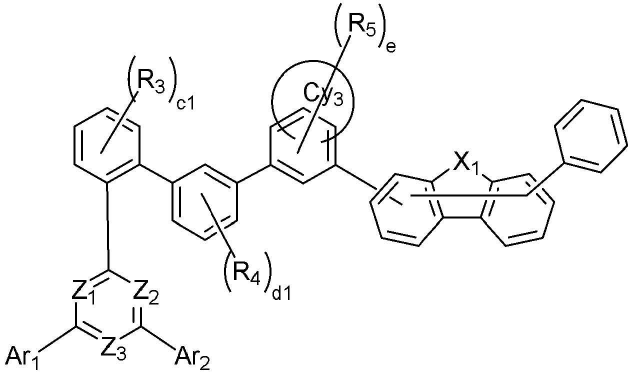 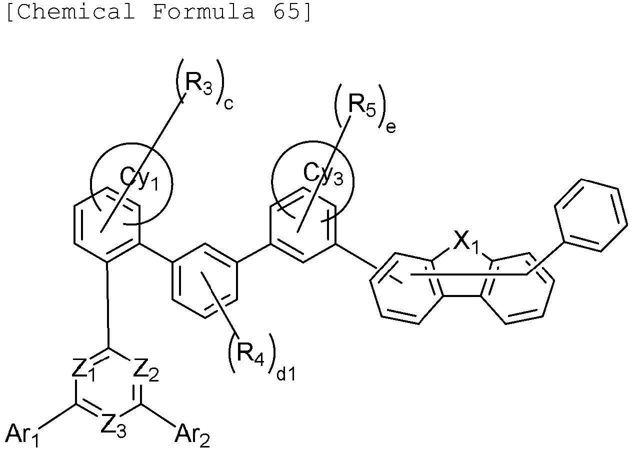 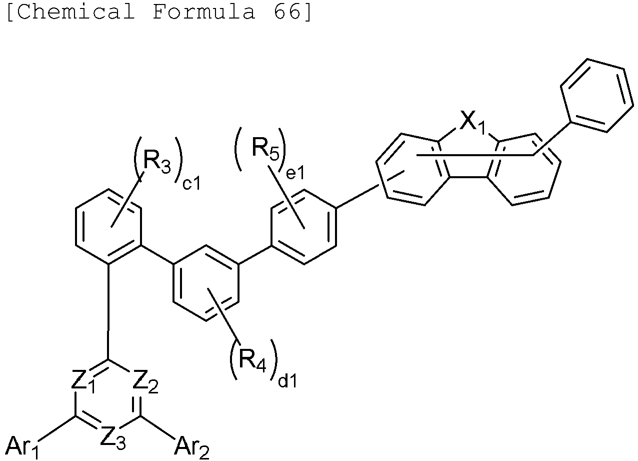 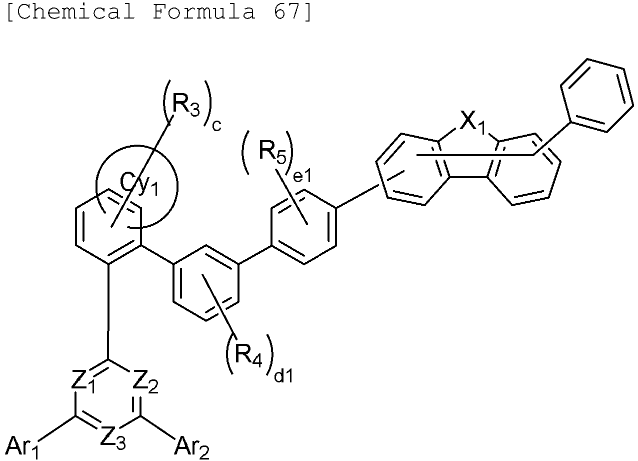 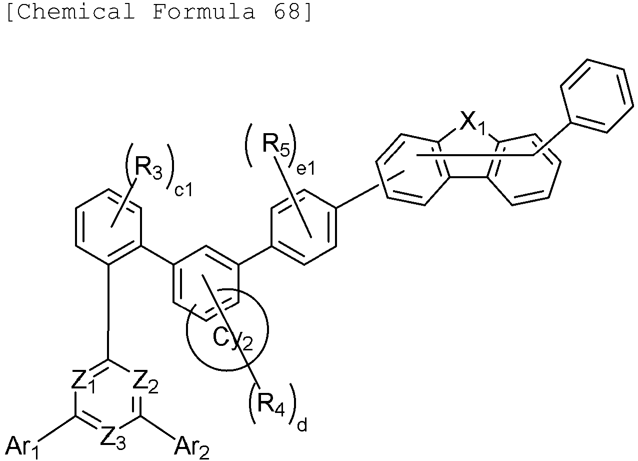 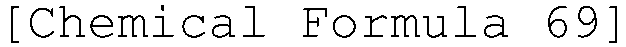 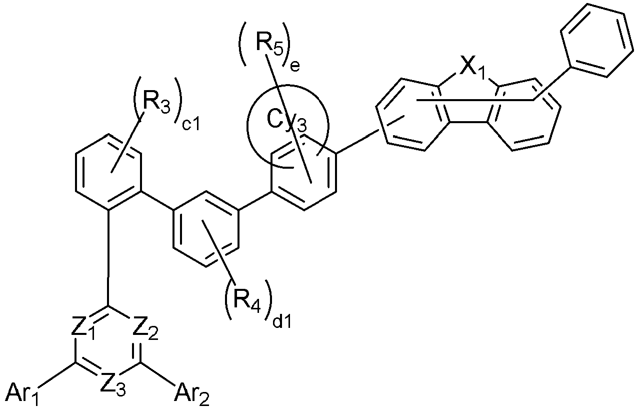 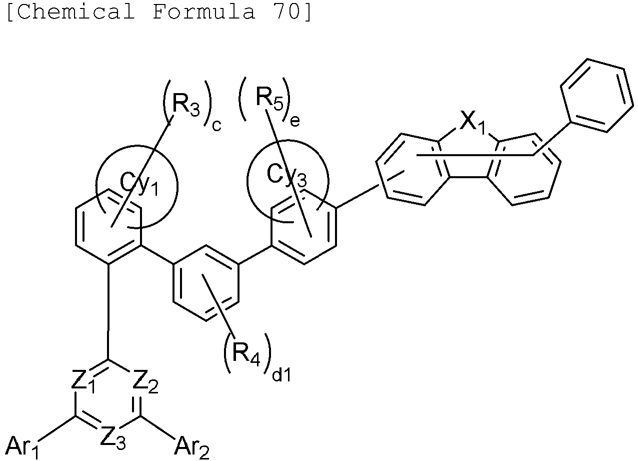 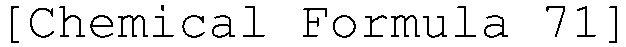 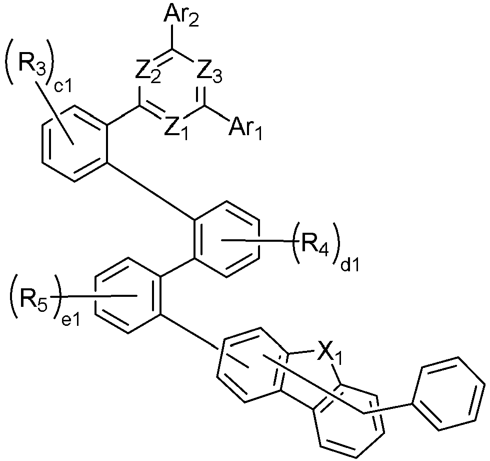 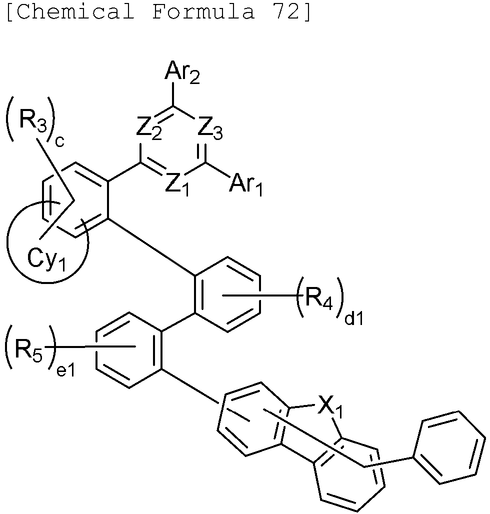 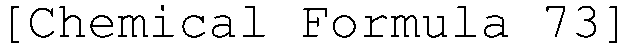 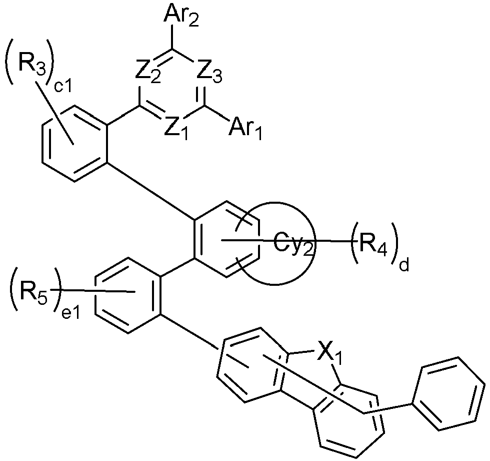 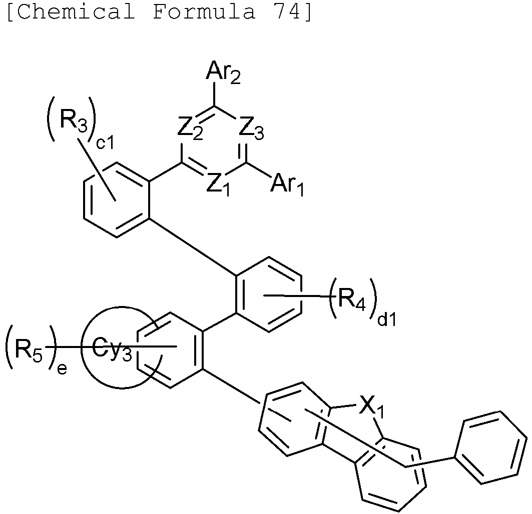 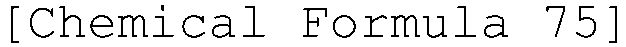 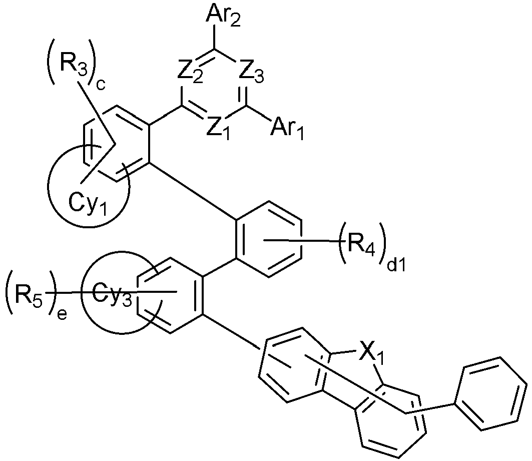 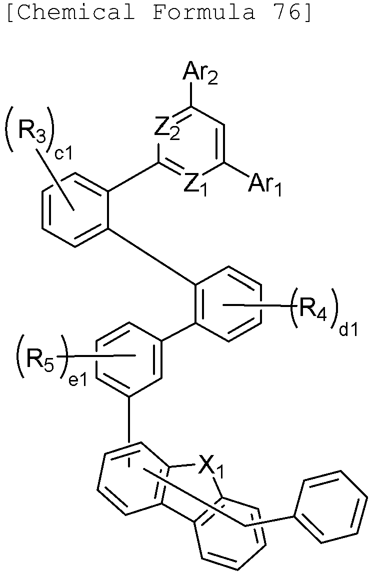 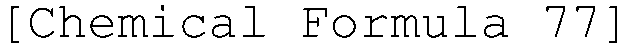 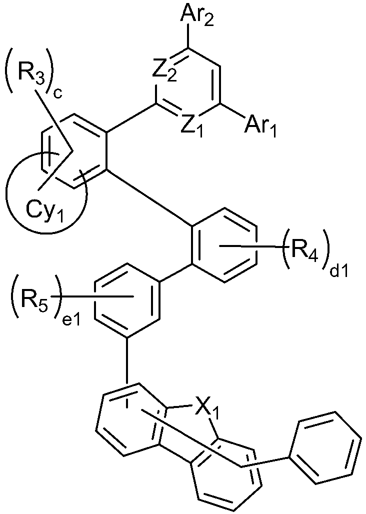 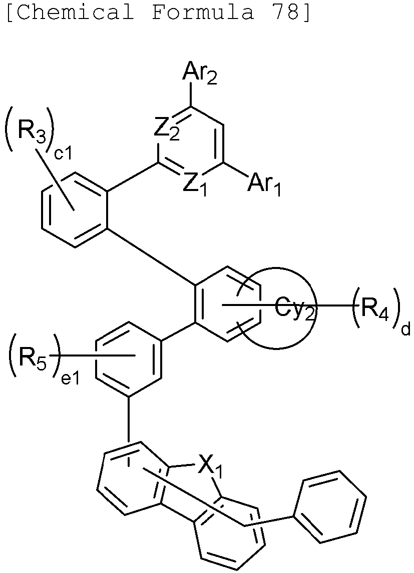 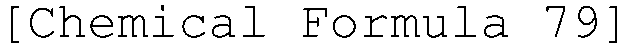 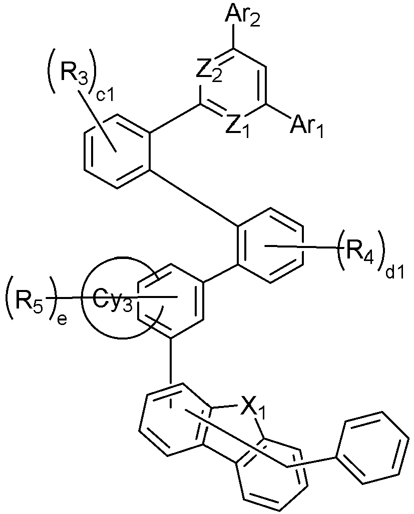 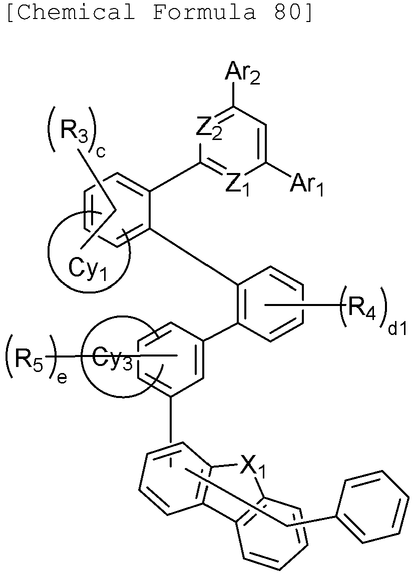 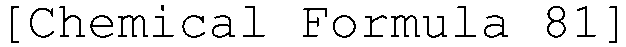 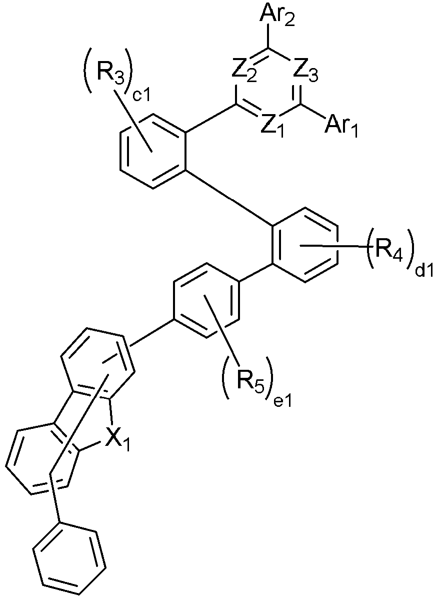 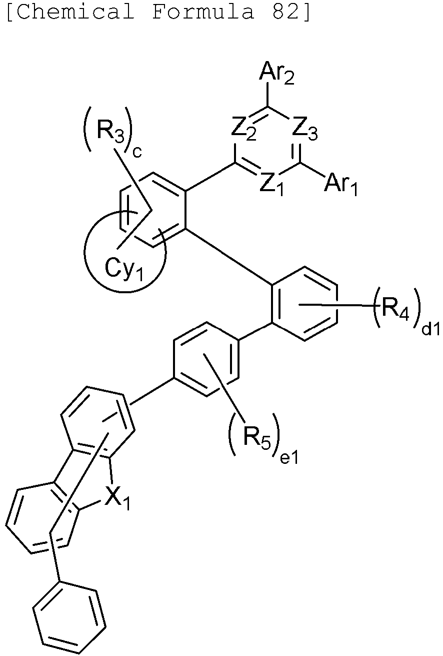 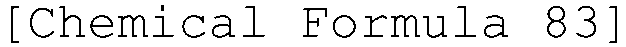 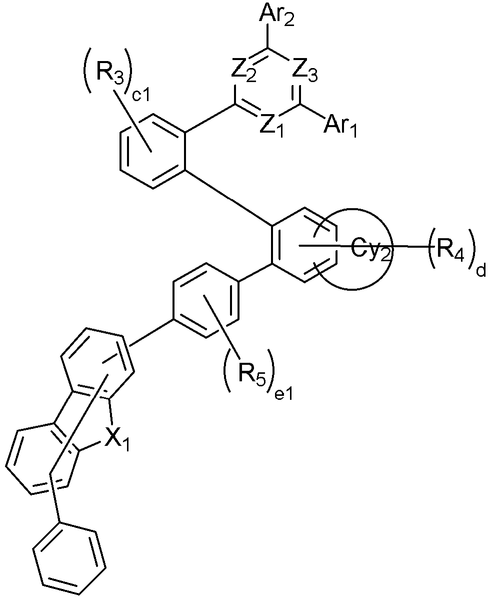 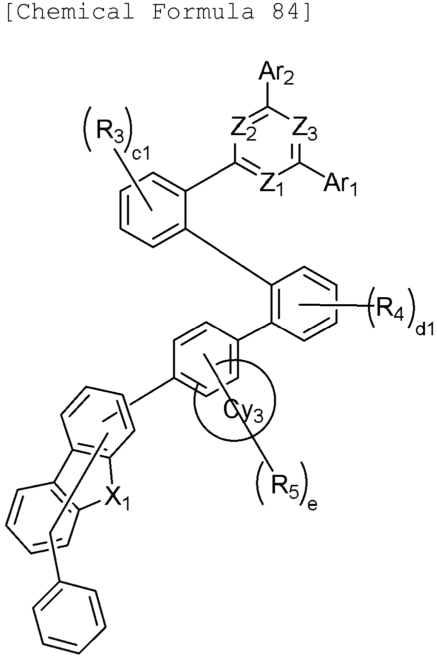 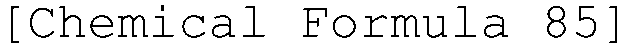 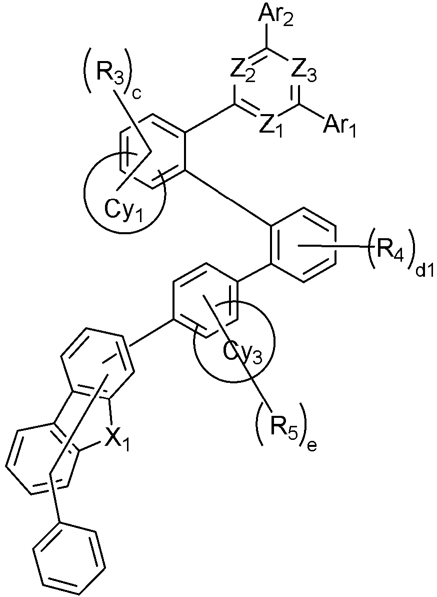 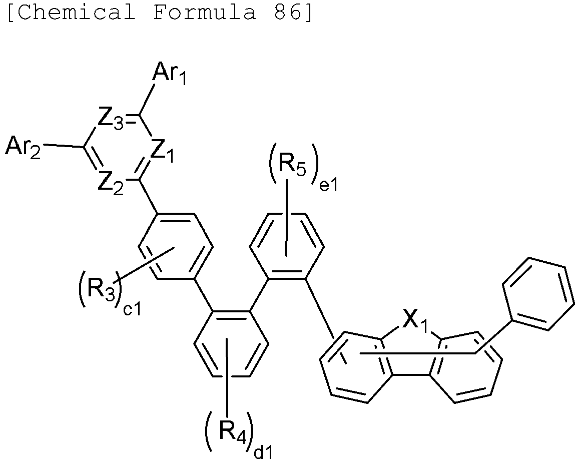 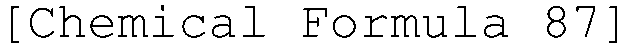 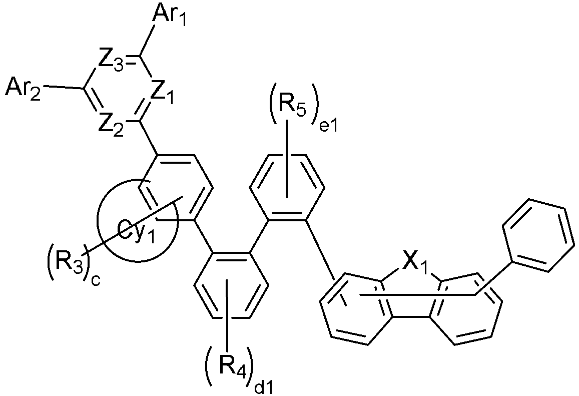 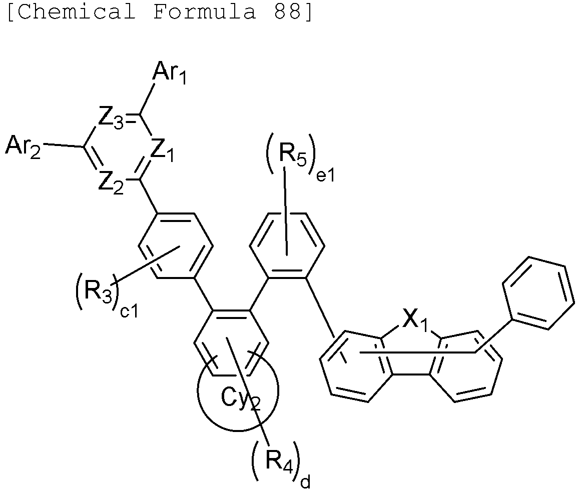 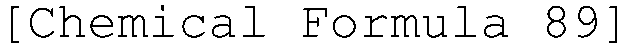 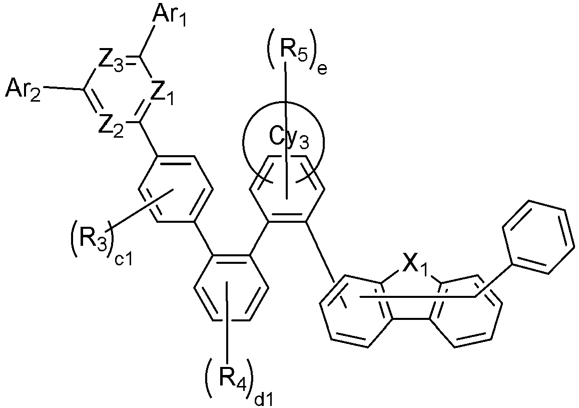 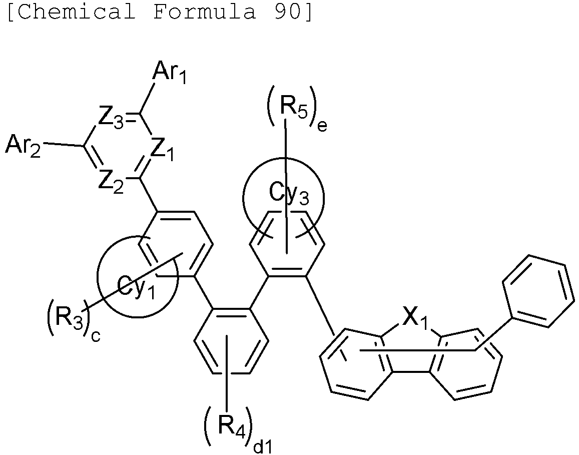 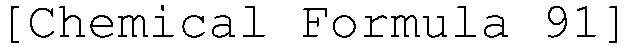 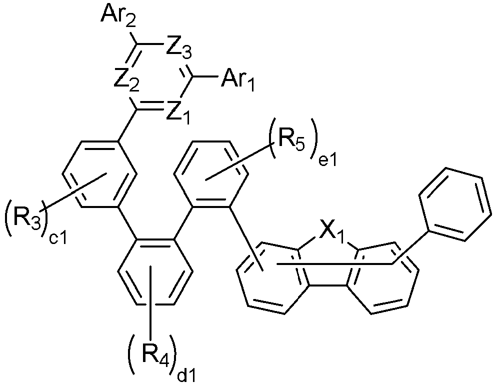 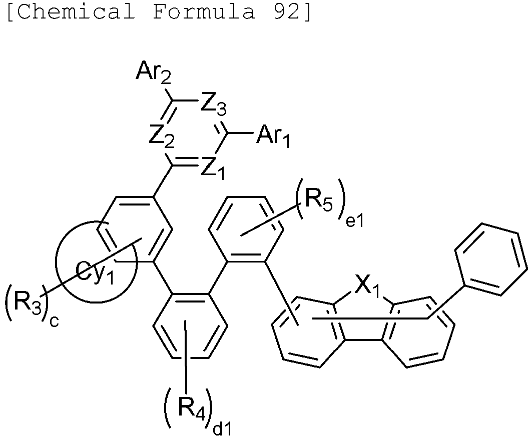 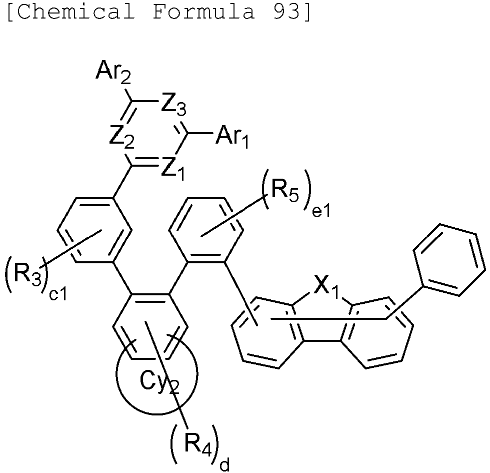 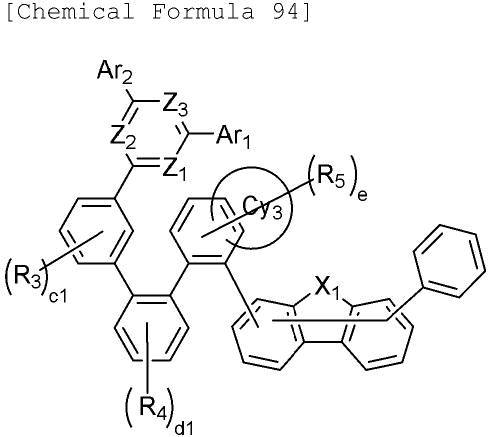 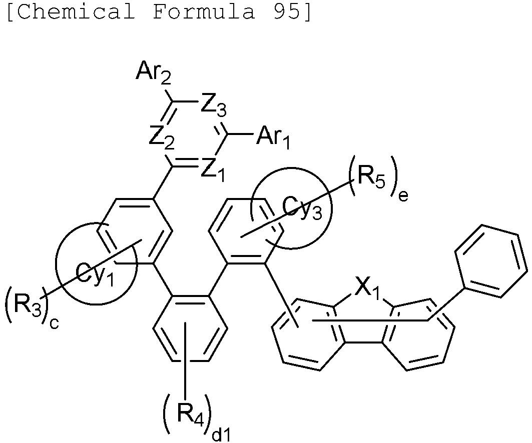 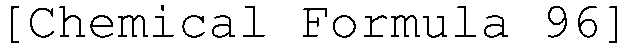 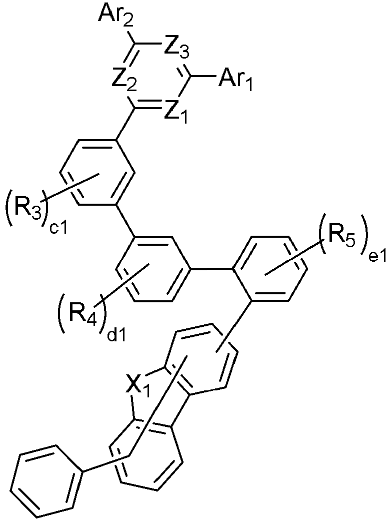 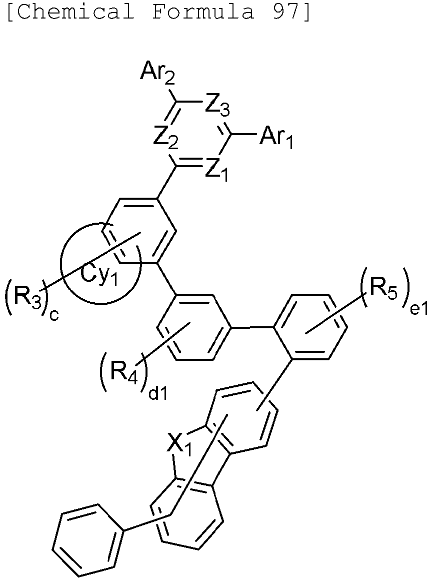 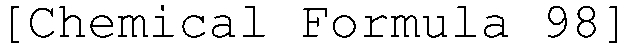 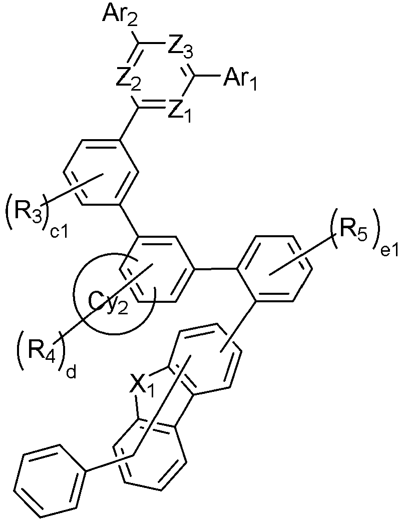 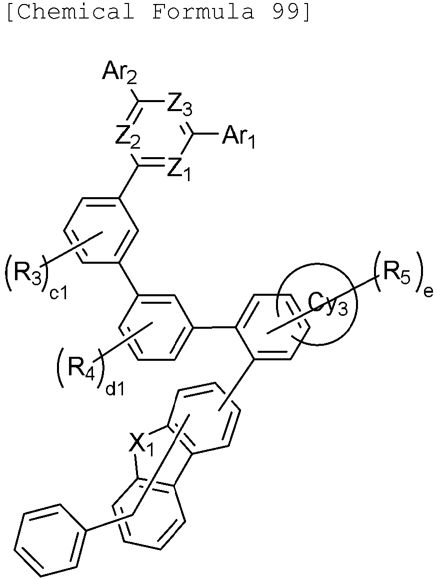 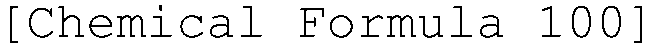 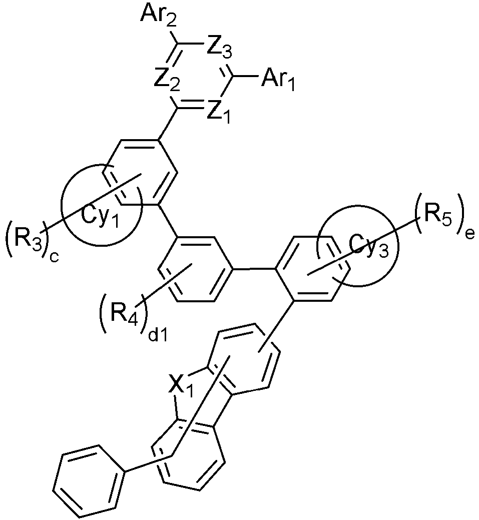 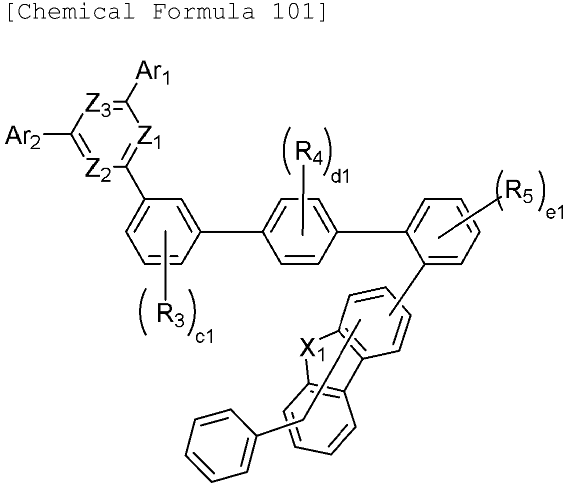 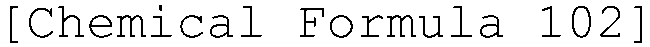 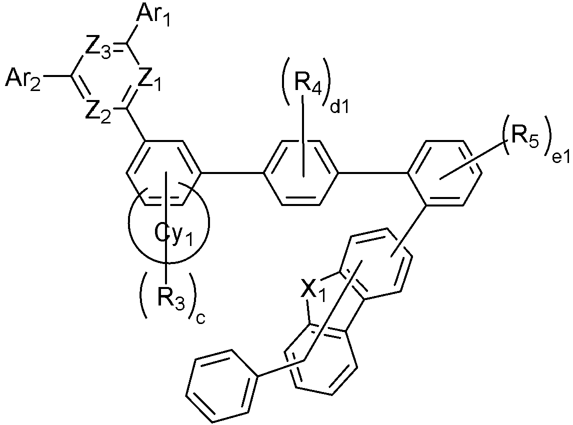 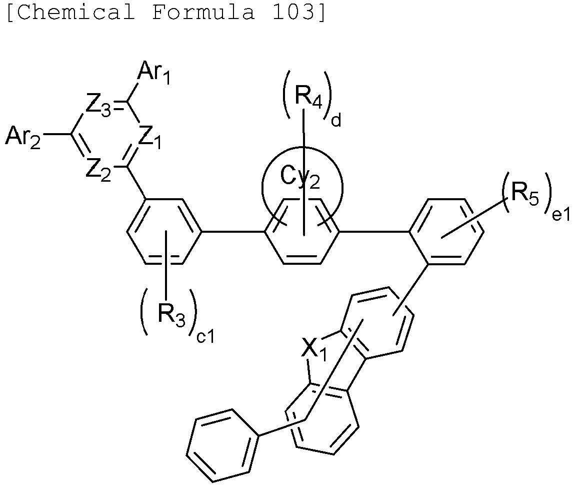 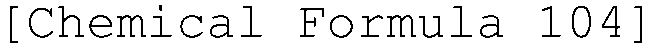 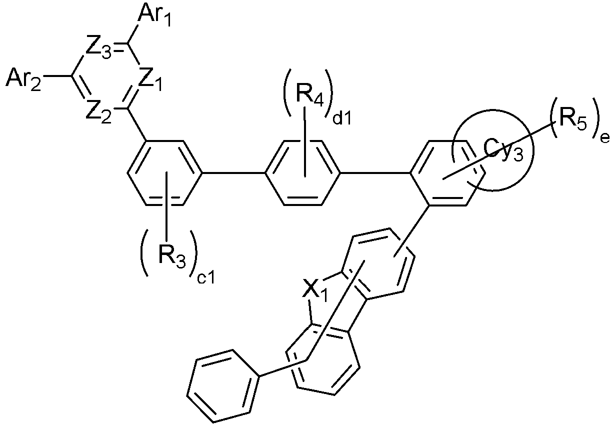 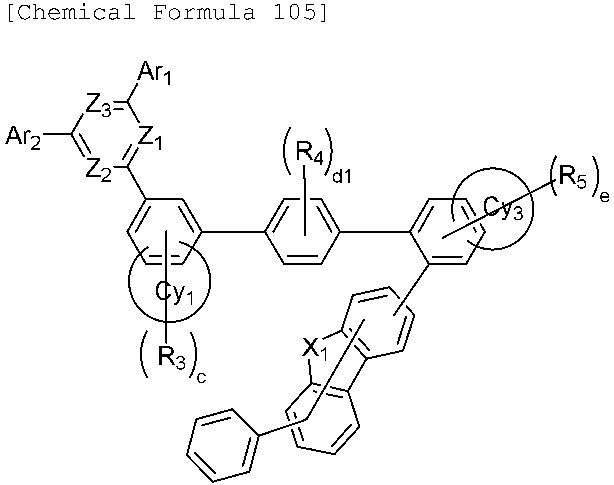 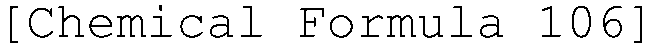 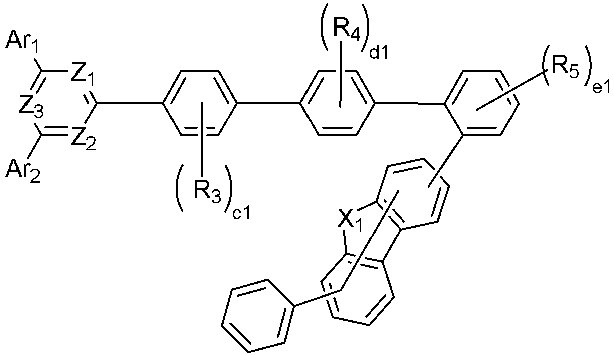 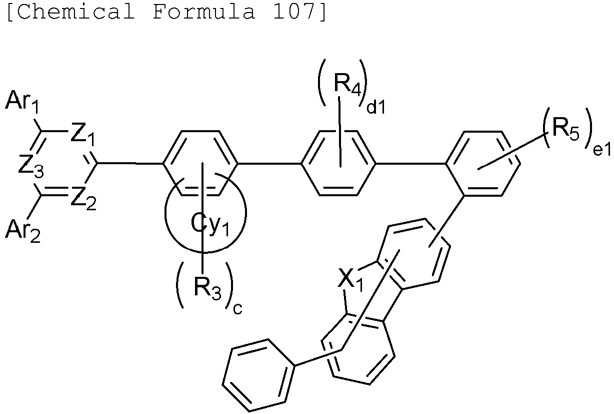 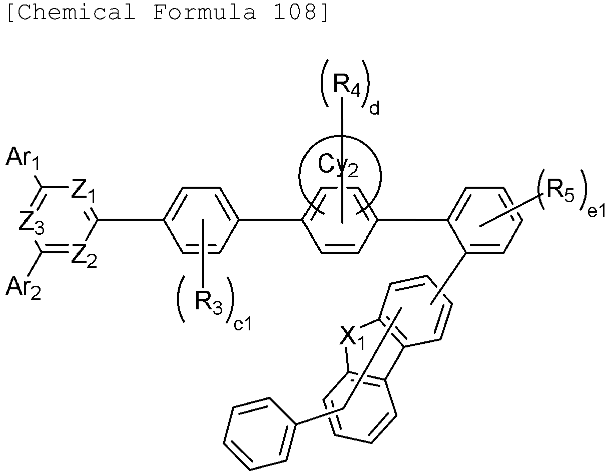 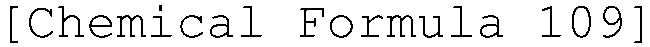 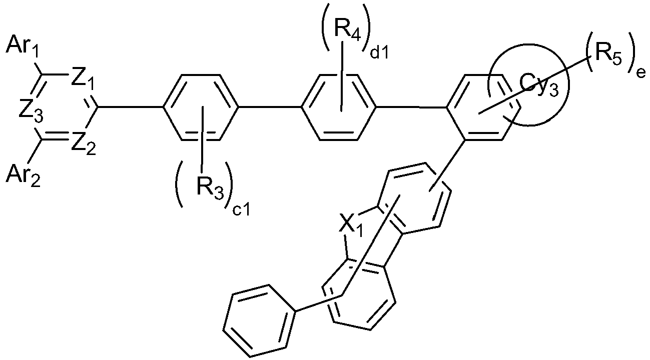 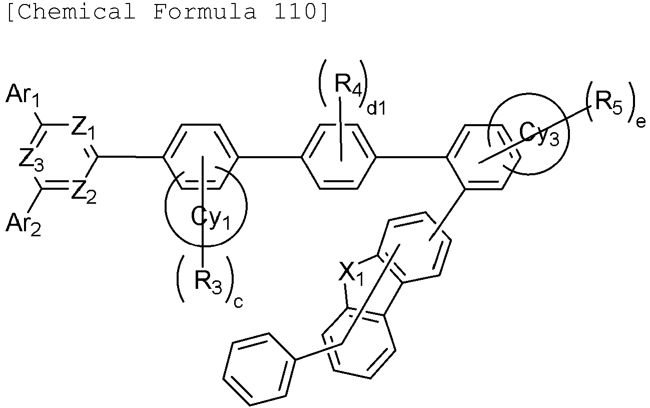 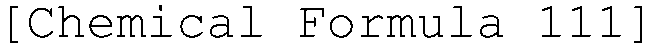 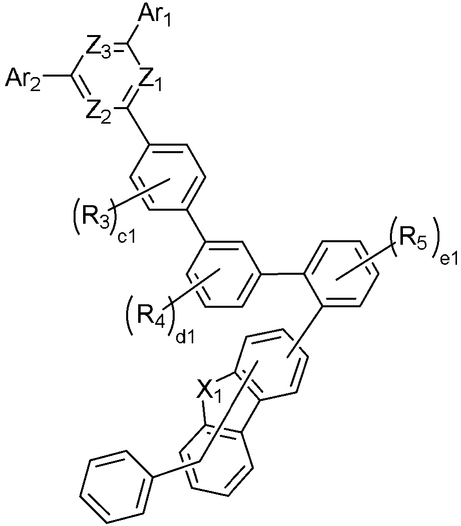 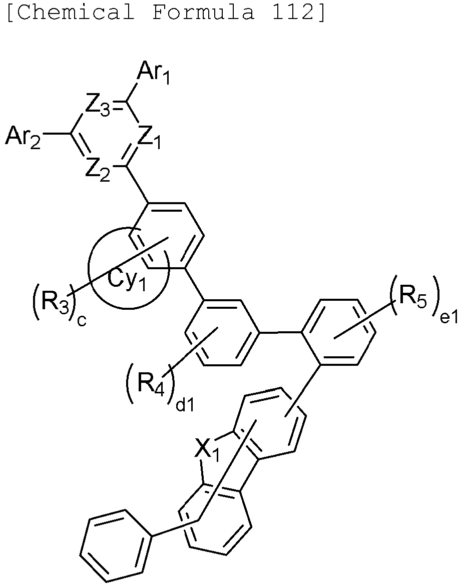 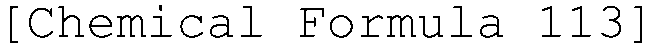 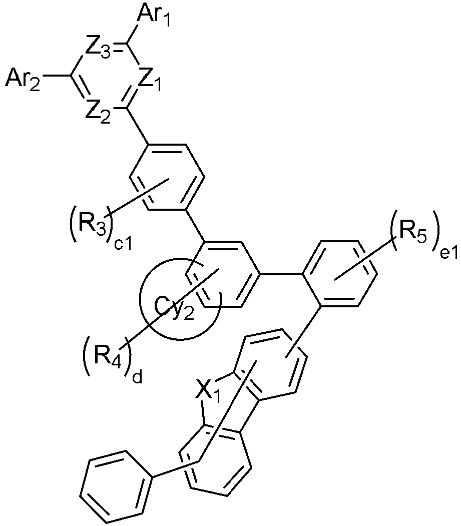 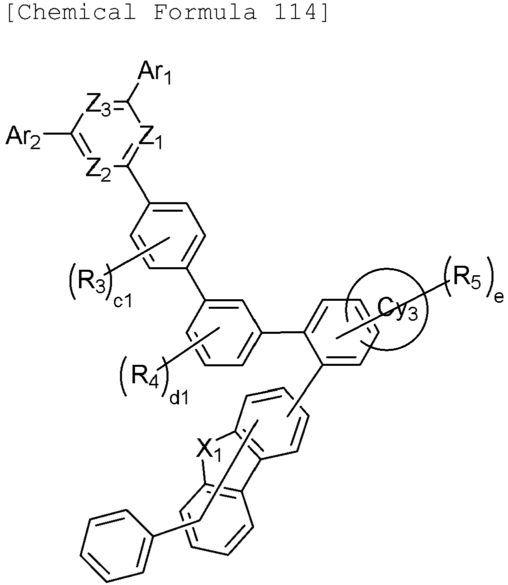 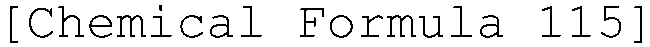 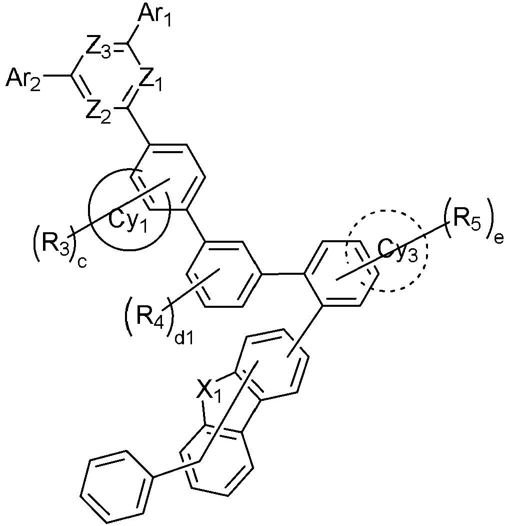
(wherein in Chemical Formulas 2 to 115,
Z₁ to Z₃, X₁, Ar₁, Ar₂, Cy₁ to Cy₃, c, d, e, R₃ to R₅ are each as defined in claim 1,
c1, d1 and e1 are each independently an integer of 0 to 4).

6. The compound of Claim 1, wherein the compound represented by Chemical Formula 1 is selected from the group consisting of the following compounds 1 to 252: 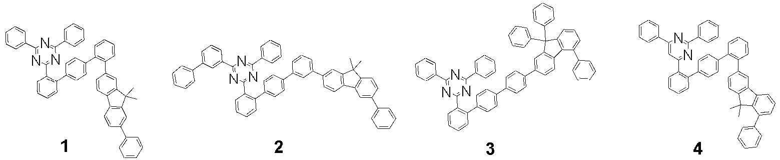 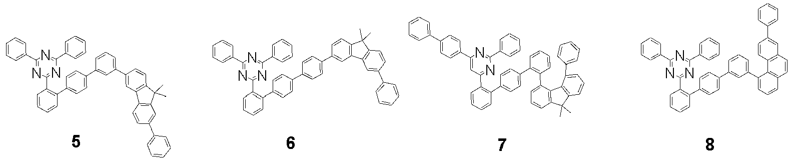 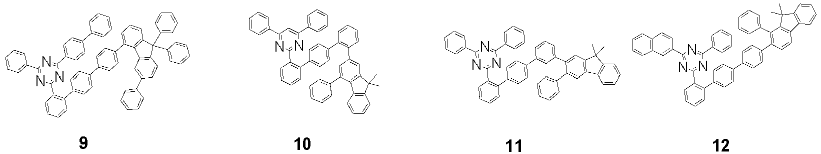 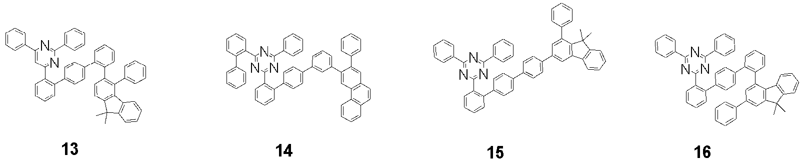 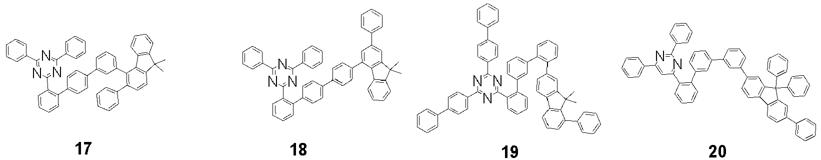 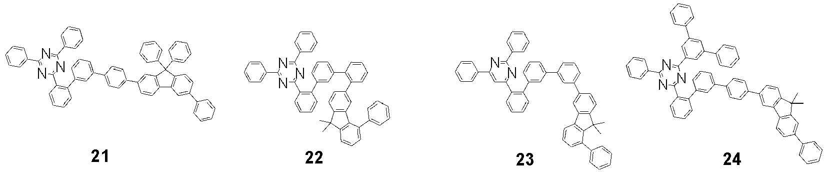 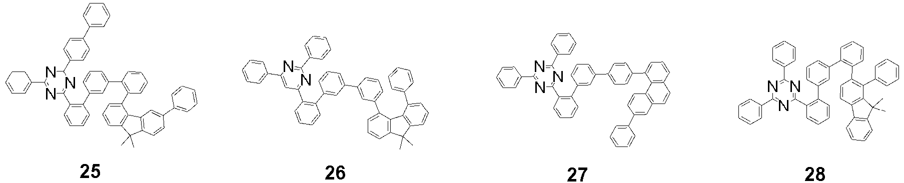 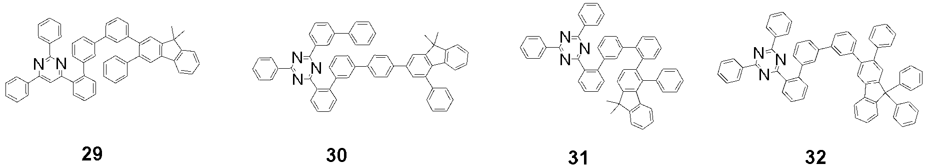 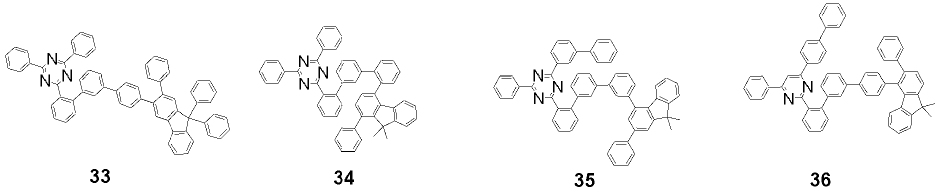 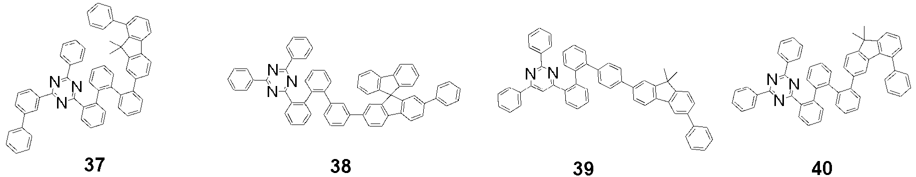 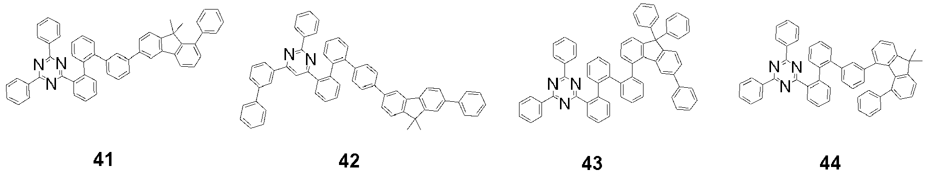 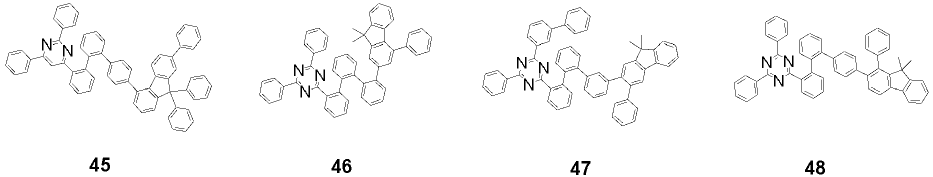 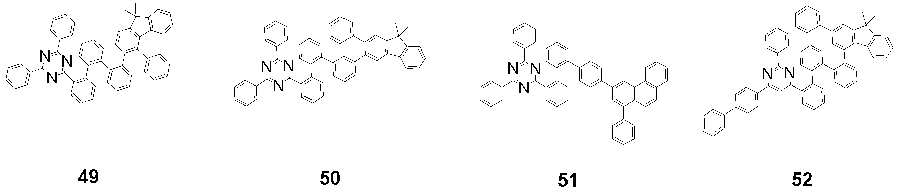 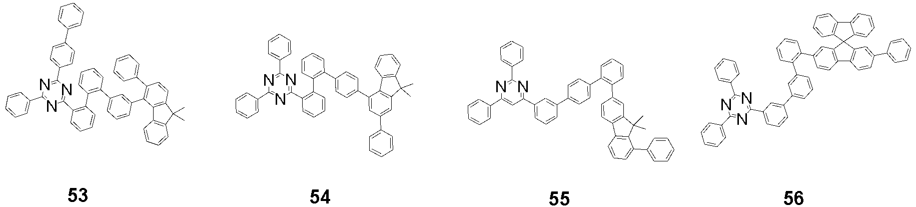 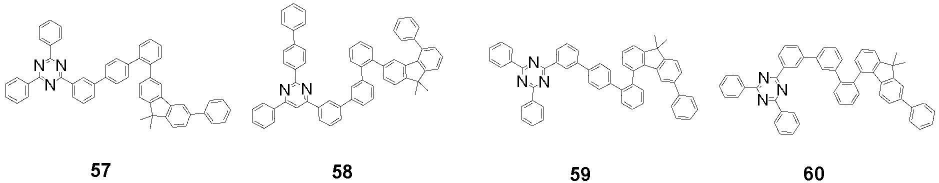 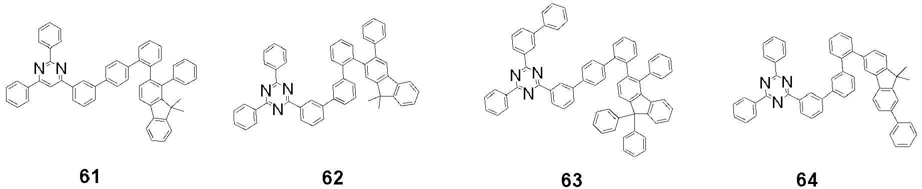 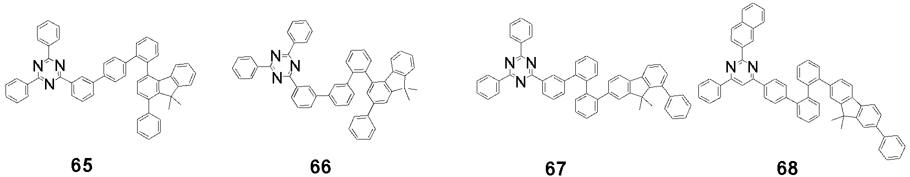 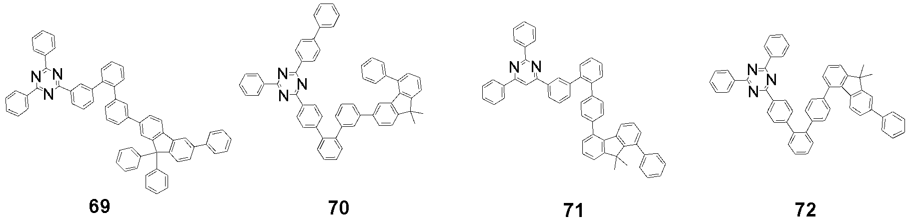 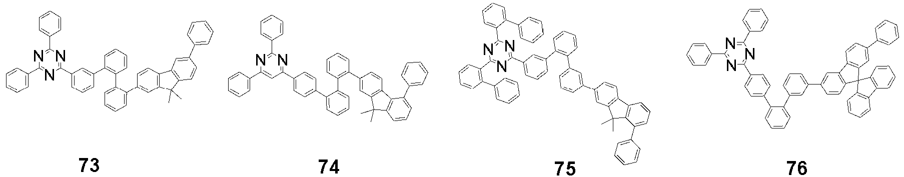 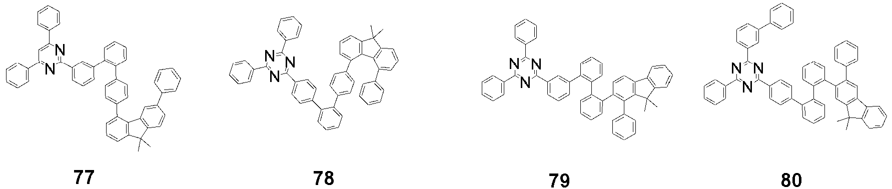 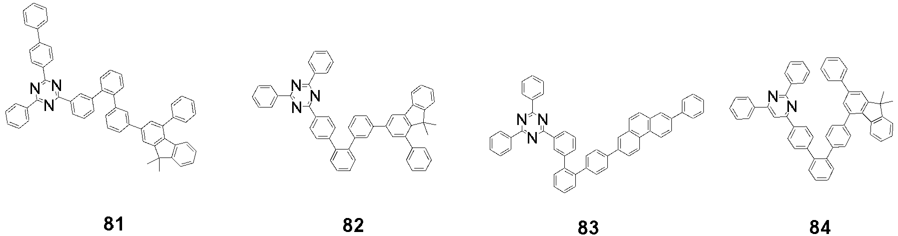 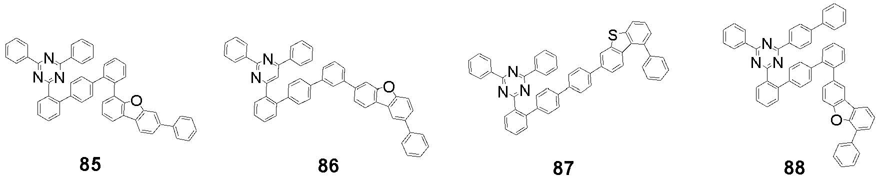 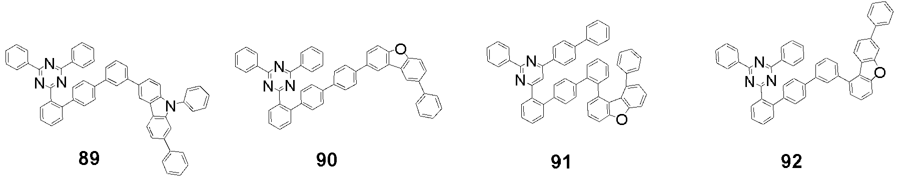 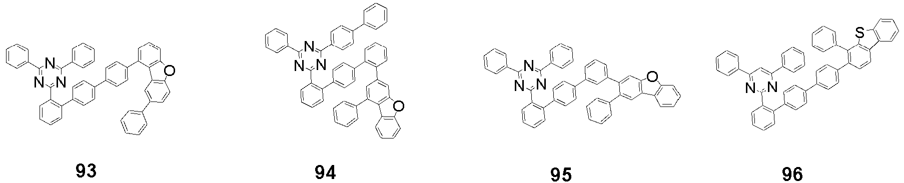 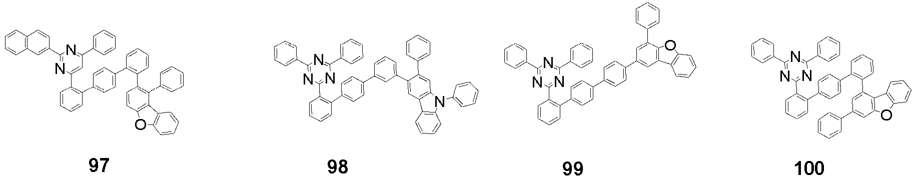 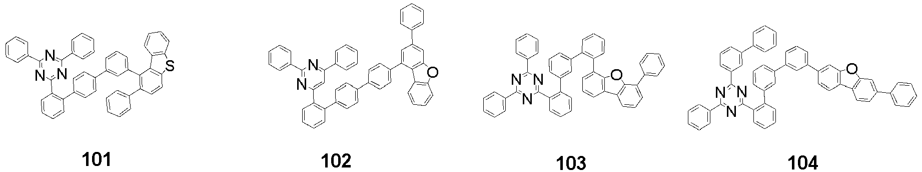 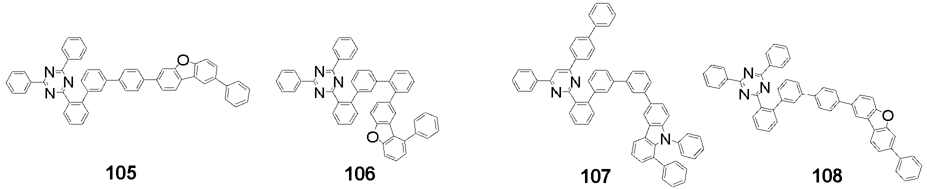 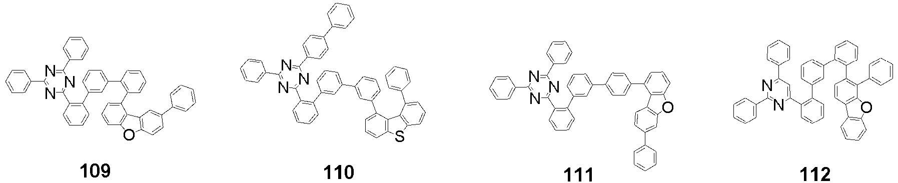 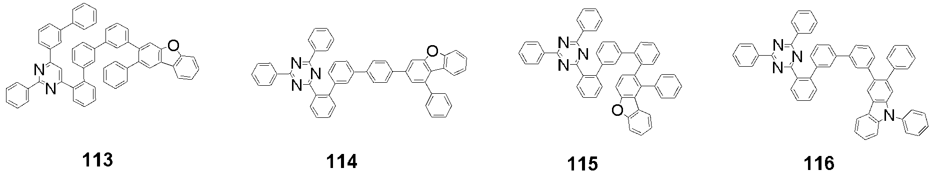 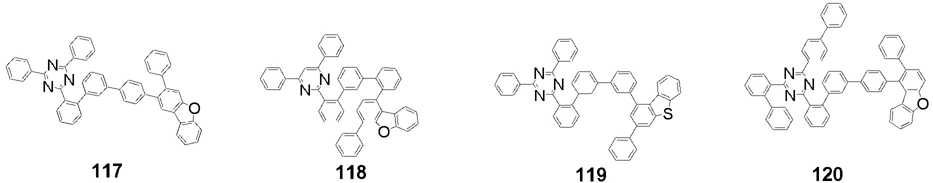 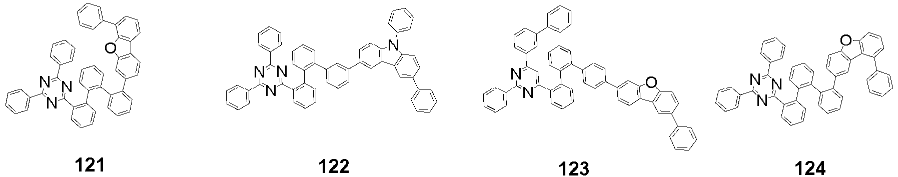 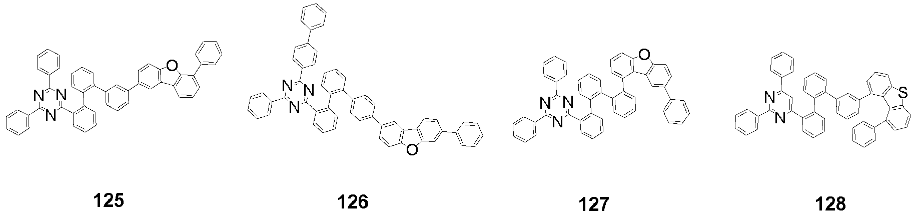 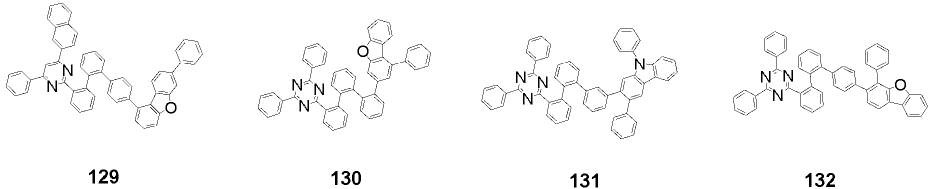 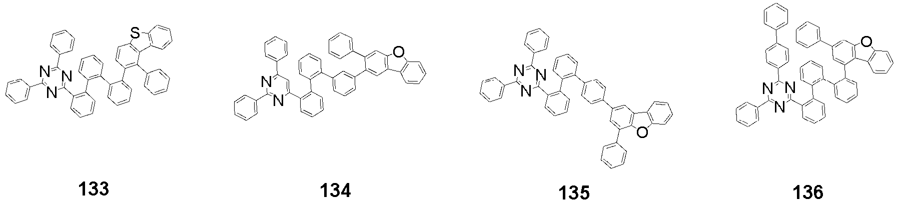 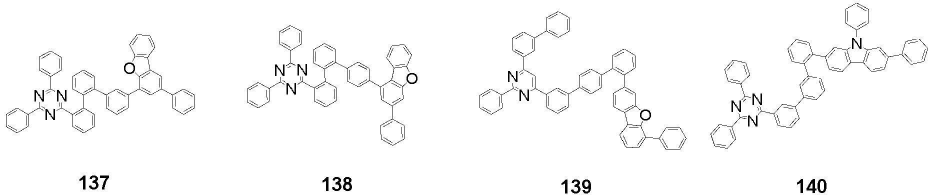 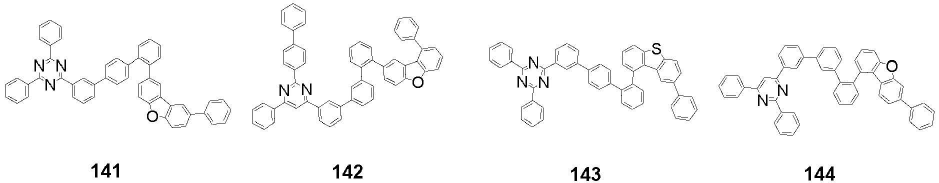 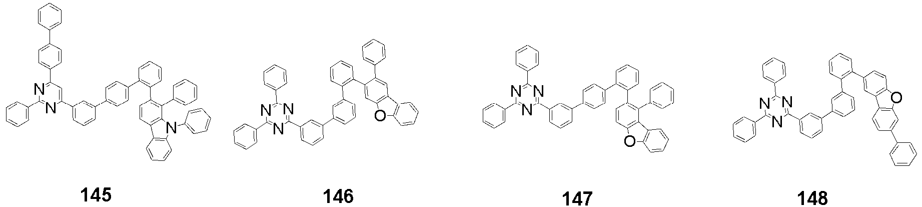 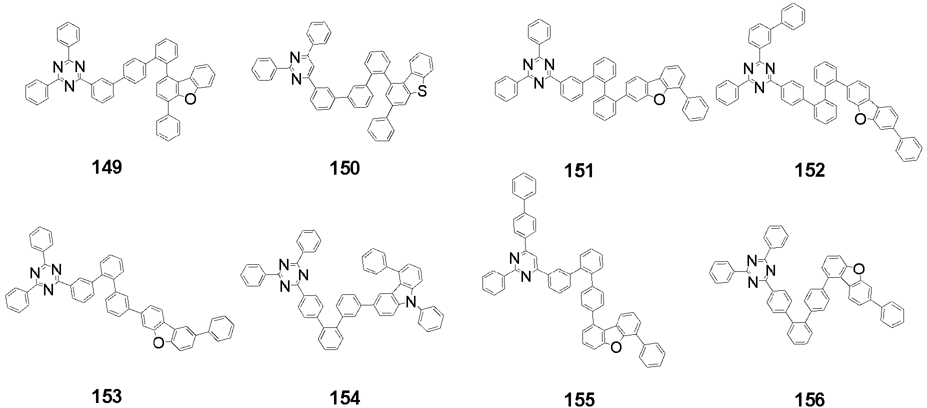 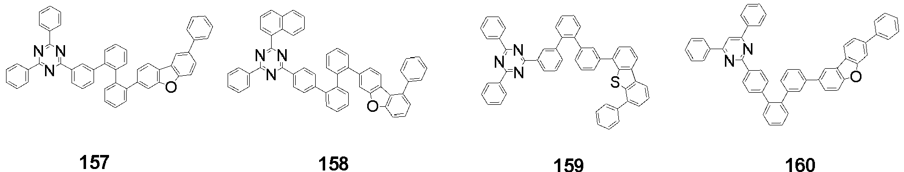 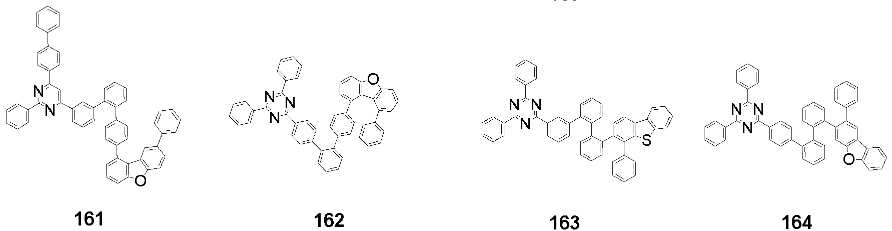 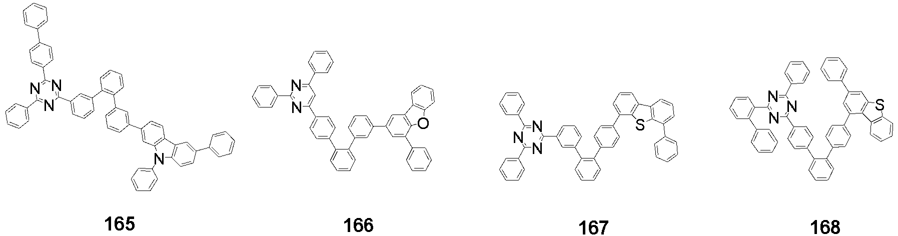 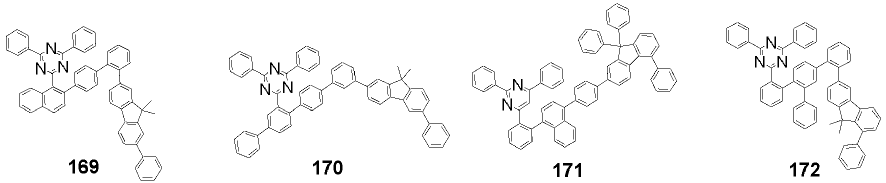 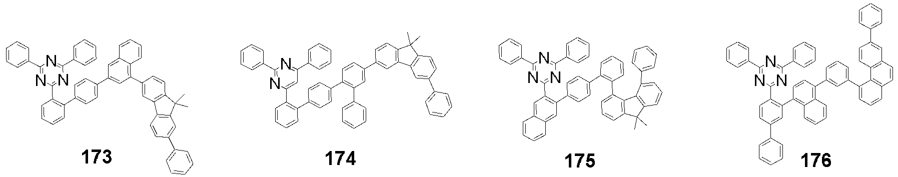 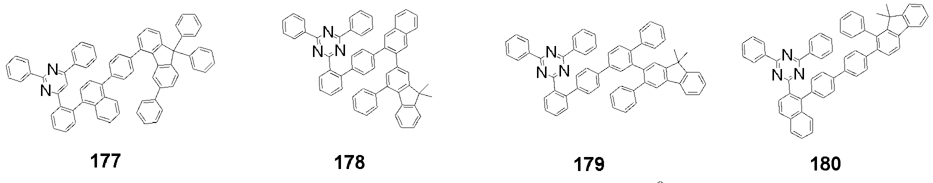 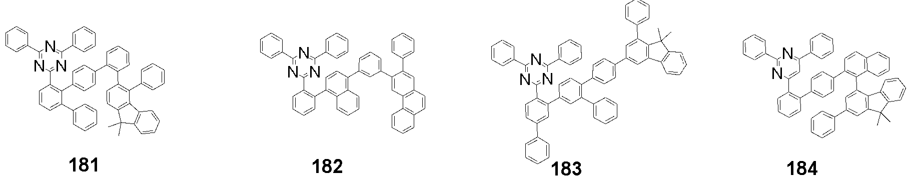 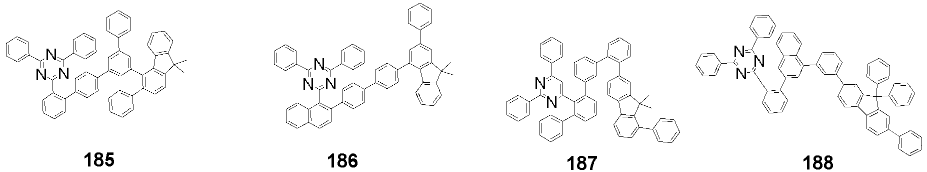 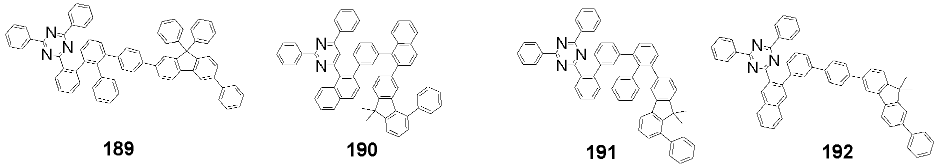 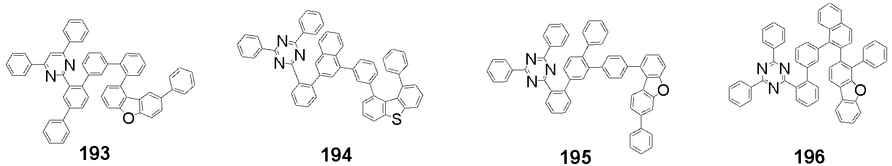 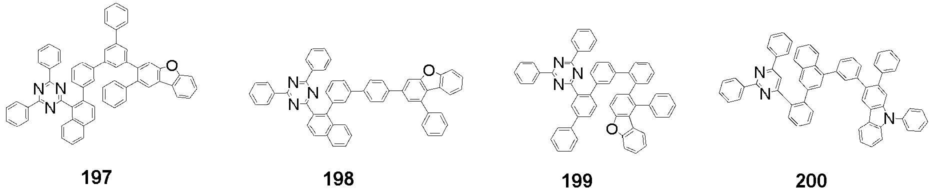 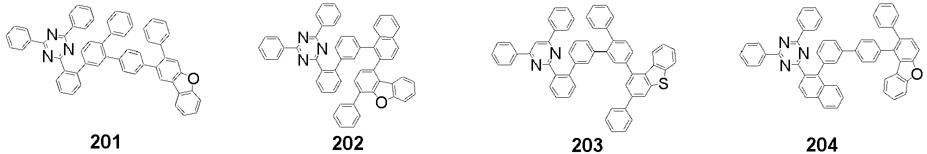 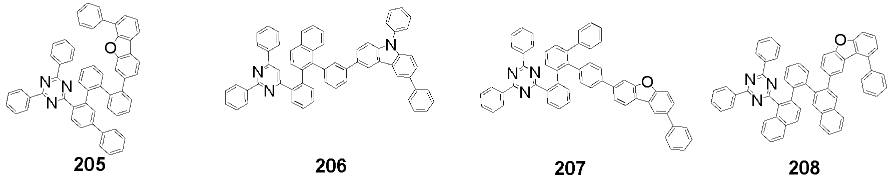 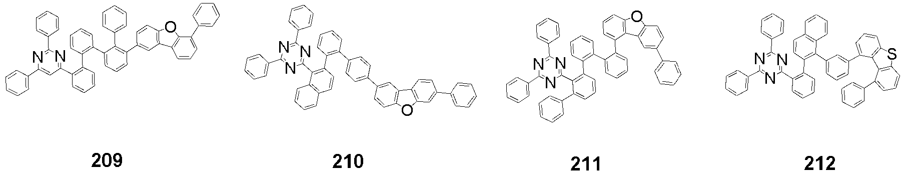 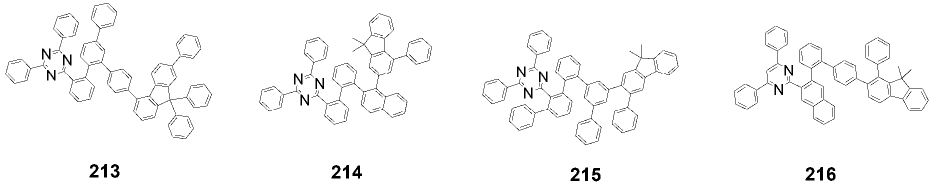 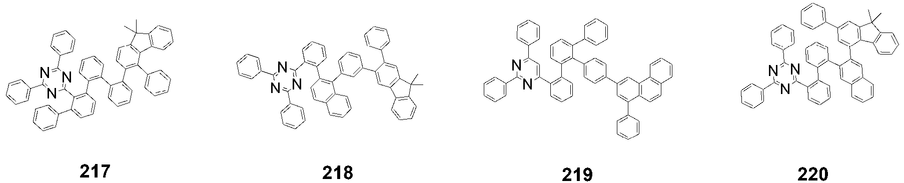 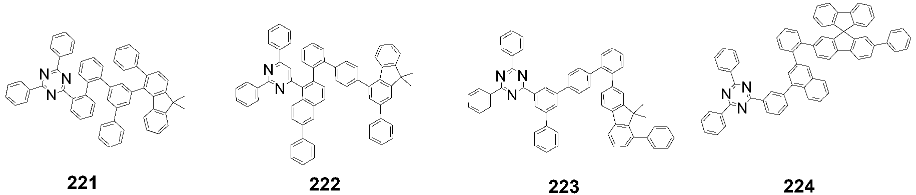 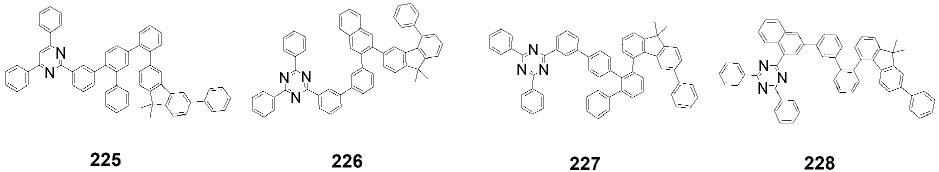 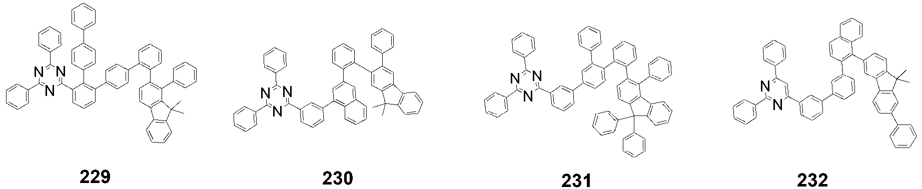 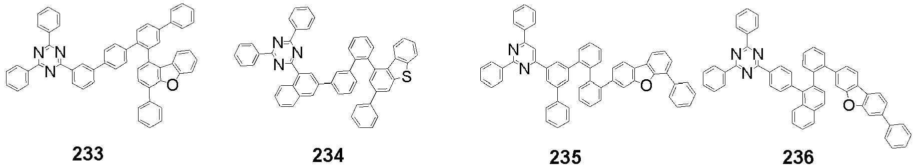 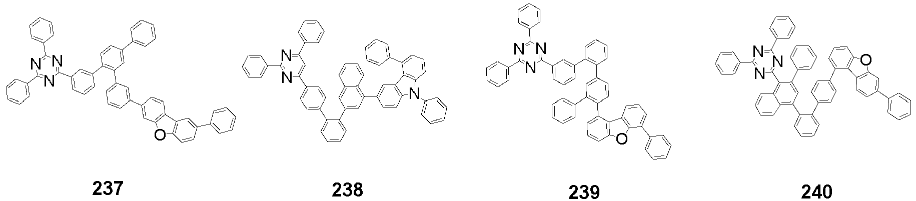 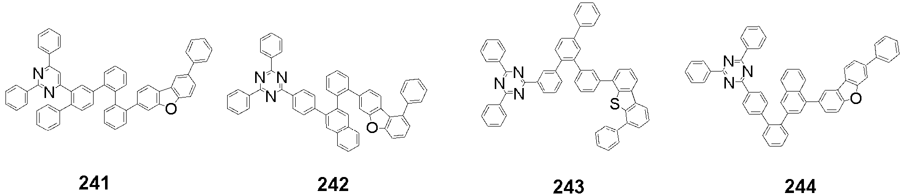 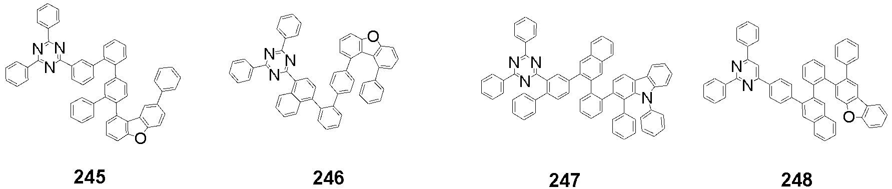 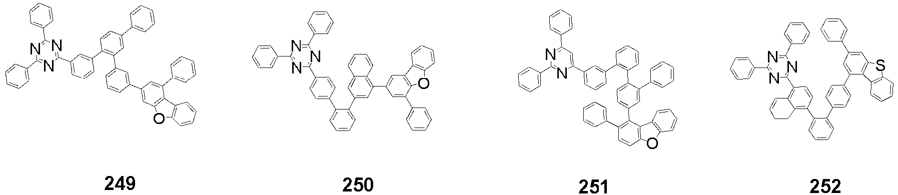 .

7. An organic electroluminescent device comprising:
an anode;
a cathode; and
one or more organic layers disposed between the anode and the cathode,
wherein at least one of the one or more organic layers comprises the compound according to any one of claims 1 to 6.

8. The organic electroluminescent device of claim 7, wherein the organic layer comprising the compound is an electron transport layer or an electron transport auxiliary layer.
